# EUROPEAN PATENT APPLICATION

(11) **EP 2 022 799 A2**
(43) Date of publication of application: **11.02.2009**
(21) Application number: 08020112.2
(22) Date of filing: 18.11.2002
(51) Int. Cl.: C07K 16/00, C12N 5/10

(54) **Polycistronic expression of antibodies**

(30) Priority: 16.11.2001 US 331481 P; 05.08.2002 US 400687 P
(62) Divisional of application: 02802572.4
(71) Applicant: Biogen Idec Inc., Cambridge, MA 02142 (US)
(72) Inventor: Reff, Mitchell, San Diego, CA 92122 (US); Barnett, Richard, San Marcos, CA 92069 (US)
(74) Representative: Vossius & Partner

(57) **Abstract**

Described herein is a eukaryotic cell that is stably transfected with a polycistronic vector for expressing functional antibodies, the vector comprises a polycistronic transcription system comprising the following elements operably linked in the 5' to 3' orientation: (i) a promoter operable in a eukaryotic cell; (ii) a first cistron comprising a first DNA sequence comprising a 5' start codon, a nucleotide sequence encoding an antibody light chain, and a 3' terminal stop codon, wherein said first DNA sequence does not comprise a poly A sequence at its 3' end; (iii) an internal ribosome entry site (IRES) obtained from a member selected from the group consisting of a cardiovirus, a herpes virus and a poliovirus; (iv) at least an additional cistron comprising a second DNA sequence comprising a 5' start codon, a nucleotide sequence encoding an antibody heavy chain, and a 3' terminal stop codon, wherein said second DNA sequence comprises a poly A sequence at its 3' terminus; wherein the IRES is operably linked to the 5' end of the additional cistron with no intervening regulatory elements; and wherein the functional antibodies expressed by the polycistronic vector specifically bind to a tumor associated antigen. Further described is a method of producing functional antibodies comprising culturing the eukaryotic cell of the invention in a cell culture to produce functional antibodies.

## Description

### RELATED APPLICATION

This application relates to and claims priority to provisional patent application 60/331,481 filed on November 16, 2001, and provisional application 60/400,687, filed on August 5, 2002, both incorporated by reference in their entirety herein. Additionally, this application claims priority to PCT/US02/02373 and PCT/US02/02374 both filed January 29, 2002.

### FIELD OF THE INVENTION

The present invention relates to a novel polycistronic expression system and methods for producing antibodies in eukaryotic cells using this expression system. More specifically, the invention relates to a eukaryotic polycistronic expression system in which antibody heavy and light chain genes are transcribed from the same promoter, and, preferably, the antibody heavy and light chain genes are separated by one or more internal ribosome entry sites (IRES's).

### BACKGROUND OF THE INVENTION

Methods for expressing genes of choice in recombinant host cells utilizing polycistronic expression vectors are well known. Historically, polycistronic expression vectors incorporating a desired product gene sequence at the 5' end of a transcribed region and a 3' selectable marker gene sequence have been employed. Such vectors display inefficient translation of the 3' selectable gene, while preferentially translating the desired gene sequence at the 5' end of the polycistronic mRNA. Recombinant host cells expressing high levels of the desired gene product are obtained via a single step method comprising culturing initial transfectants in a selectable medium. A drawback of such vectors is the unpredictable influence an upstream reading frame may exert on translation of the selectable marker sequence. See Kaufman, Meth. Enzymol., 185:487 (1990).

For example, U.S. Patent No. 4,713,339, issued to Levinson et al. (assigned on its face to Genentech, Inc.) discloses a polycistronic expression system capable of producing a gene product of interest in eukaryotic host cells. In the system patented by Levinson et al., the gene sequence of the second cistron encodes a protein that provides for the detection of transfectants or transformants that express a desired gene of interest encoded by the first cistron. Certain growth conditions induce amplified expression of the detector gene in a host cell, which thereby enhances the expression of associated sequences encoding the desired gene contained on the polycistronic transcription unit. In particular, Levinson constructed vectors containing a polycistron in which a sequence encoding hepatitis B-surface antigen was positioned upstream of a sequence encoding the screening marker dihydrofolate reductase. Due to the polycistronic arrangement of the Levinson vectors, unequal expression of the downstream marker gene sequence of the second cistron occurs in comparison to the expression of the first cistron sequence.

Additionally, polycistronic expression systems have been used to express multichain polypeptides. For example, polycistronic expression of multichain polypeptides is reported in US Patent No. 6,060,273 to Dirks et al.; US Patent No. 6,033670 to Bublot; US Patent No. 6,096,505 to Selby et al.; US Patent No. 6,143,520 to Marasco et al.; US Patent No. 6,153,199 to Audonnet et al.; and US Patent No. 6,156,558 to Johnston et al.

Investigators have determined that levels of the second gene in the polycistron are improved by the incorporation of an internal ribosome entry site (IRES) between the genes in the polycistron. IRES elements, first identified in picomaviruses, mediate the initiation of translation by directly recruiting and binding ribosomes to a message, bypassing the 7-methyl guanosine-cap involved in typical ribosome scanning. The presence of an IRES sequence can increase the level of cap-independent translation of a desired protein. Early publications descriptively refer to IRES sequences as "translation enhancers". For example, cardioviral RNA "translation enhancers" are described in U.S. Patent No. 4,937,190 to Palmenberg, et al. and U.S. Patent No. 5,770,428 to Boris-Lawrie.

Some IRES containing reporter cistrons have been patented, such as the XIAP IRES (US Patent Nos. 6,171,821 and 6,159,709 to Korneluk). One IRES sequence having known use in polycistronic expression vectors is that of herpes virus; other viruses may be used as well (See US Patent 6,193,980). Korneluk discloses bicistronic vectors constructed by inserting β-galactosidase and chloramphenicol acetyltransferase reporter sequences into a plasmid having a CMV promoter, such that the two cistrons are separated by a 100 bp intercistronic linker region containing an IRES sequence. The first cistron, encoding β-galactosidase, was translated via a conventional cap-dependent mechanism. The second cistron, encoding chloramphenicol acetyltransferase, was translated only when the preceding linker region contained the IRES site. Thus, Komeluk showed that IRES sequences can mediate the translation of a second open reading frame in bicistronic mRNA constructs designed to measure cellular responses to stress. However, only marker sequences were utilized in the second cistron, as the state of the art recognized the inefficiency associated with expression of second sequences in bicistronic arrangements.

Additionally, the co-expression of amplifiable markers using polycistronic expression systems have been described. For example, WO 92/17566 discloses a method of co-transfecting host cells with an intron-modified selectable gene and a gene encoding a protein of interest. The intron-modified gene is generated via insertion of an intron into the transcribed region of a selectable gene such that the intron is correctly spliced from the mRNA with reduced efficiency. Inefficient splicing of this nature results in low amounts of selectable marker protein being produced from the intron-modified selectable gene in comparison with unmodified selectable gene sequences. While reduced amounts of selectable gene are produced concurrently with the protein of interest, this model does not employ a transcriptional linkage between the selectable gene sequences and the desired gene sequence of interest, as the two sequences are driven by separate promoters.

Antibody production using a dicistronic expression vector has been disclosed, employing vectors exhibiting transcriptional linkage between dihydrofolate reductase (DHFR) and a nucleotide sequence encoding a desired antibody product. See U.S. Patent No. 5,561,053 to Crowley. The method of Crowley utilizes DNA constructs having a single promoter to drive expression of a selectable marker sequence and a single sequence encoding a protein of interest. The heavy chain of the desired antibody was inserted downstream of a selectable gene, DHFR, which was placed within an intron at the 5' end of the DNA construct. The intron was positioned between the cytomegalovirus immediate early promoter (CMV) and the sequence encoding the antibody heavy chain. The light chain of the antibody was placed into a second vector constructed to place the light chain sequence under control of the SV40 promoter/enhancer and the selectable hygromycin B resistance gene sequence driven by the CMV promoter/enhancer and SV40 poly-A. Vectors containing the light and heavy chain sequences were linearized and co-transfected into host cells for expression and subsequent disulfide linkage between light and heavy chains. Thus, in order to obtain a complete antibody structure according to Crowley, two vectors must be constructed for separate expression of the heavy and light chain components of the antibody. This method is inefficient due to the use of multiple vectors.

The method of Crowley exemplifies the limitations on multiple subunit protein expression imposed by the positional effect associated with traditional polycistronic expression systems. The imbalance of expression between the 5' and 3' genes in a polycistron reduces the efficiency with which commercial scale production of multichain proteins or similar products of interest can be generated.

U.S. Patent No. 6,060,273 to Dirks discloses multicistronic expression units that allow equimolar expression of genes located in corresponding cistrons. According to Dirks, bicistronic expression vectors may be configured for expressing two genes of interest, such as PDGF-A and PDGF-B, with the assistance of an IRES. IRES-dependent translation in the bicistronic expression vectors is aided by a *Xenopus laevis* 5'UTR β-globin sequence that enhances expression of the second cistron such that equimolar expression of cistrons 1 and 2 is achieved. The bicistronic vectors may be arranged to as follows: promoter-first cistron-IRES-β**-**globin sequence-second cistron.

While the teachings of Dirks suggest a method of overcoming the inefficiency associated with polycistronic expression of some multichain protein subunits, the teachings are not considered by those skilled in the art to be a satisfactory solution to the unpredictability of downstream cistron expression. Mizuguchi et al. (IRES-dependent second gene expression is significantly lower than cap-dependent first gene expression in a bicistronic vector. Mol. Therapy, 1 (4): 376-382 (April, 2000)) investigated the efficiency of IRES-dependent second gene expression in comparison with cap-dependent first gene expression *in vitro* in several cultured cell lines as well as *in vivo* in mouse liver. IRES-dependent second gene expression ranged from 6 to 100% of first gene expression, depending upon which cell types and reporter genes were used in vector constructs. In addition, the selection of which gene was positioned first in the bicistronic vectors affected the expression of gene positioned downstream.

Moreover, Borman *et al.* noted that the efficiency of IRESes to drive cap-independent translation of a second cistronic sequence was greatly affected by the type of cell chosen as the expression host. (Comparison of picomaviral IRES-driven internal initiation of translation in cultured cell of different origins. Nucleic Acids Res, 25(5): 925-932 (1997)). Dramatic variations in activity were noted for individual IRES elements of vectors transfected into different cell lines.

These concerns with the unpredictability of downstream translation of sequences in polycistronic expression systems are particularly pertinent to the efficient production of antibodies, as the ratio of light chain to heavy chain expression is key to ensuring proper antibody folding and secretion. In this regard, Horwitz and coworkers concluded that because some antibody light chains are naturally poorly secreted, the co-expression and proper association of light and heavy chains is important to the efficient secretion of at least some whole antibodies. (Chimeric immunoglobulin light chains are secreted at different levels: influence of framework-1 amino acids. Mol. Immun. 31 (9): 683-699 (1994)).

Interestingly, Kolb et al. reported inefficient expression of the second cistronic gene in the following genomic DNA dicistronic expression vector: CMV promoter-antibody light chain gene sequence-IRES-antibody heavy chain sequence-polyadenation signal (Expression of a recombinant monoclonal antibody from a bicistronic mRNA. Hybridoma, 16(5): 421-426 (1997)). Western blotting indicated a substantial amount of light chain was produced by this construct following transfection into murine myeloma cells, but heavy chain protein was barely detectable. The investigators specifically designed this dicistronic expression construct to inefficiently express the heavy chain due to concerns about host cell toxicity induced by unpaired heavy chains. Complete, functional antibodies were detected in the supernatant of these cells only after column purification, suggesting this IRES vector/cell combination is a poor model for producing antibodies of choice on a commercially useful scale.

To the best of the inventors' knowledge, an effective means for producing functional multichain proteins, such as antibodies, using a single polycistronic expression system suitable for commercial production schemes, has heretofore been unreported in the literature. Thus, a need exists for an efficient means of producing commercially acceptable amounts of antibodies in recombinant host cell systems, in which a single construct may be utilized for expression of two or more desired protein products, such as the light and heavy chains of a desired antibody.

### SUMMARY OF THE INVENTION

The invention pertains to the expression of functional (antigen-binding) antibodies at adequate levels of expression via a polycistronic expression system in a eukaryotic host cell.

More specifically, the invention relates to the expression of functional antibody molecules in eukaryotic cells, preferably mammalian cells, fungal or yeast cells and still more preferably (Chinese Hamster Ovary) CHO cells, using a polycistronic expression system.

An aspect of this preferred embodiment of the invention is the expression of functional antibodies in mammalian cells using a polycistronic expression system comprising a eukaryotic promoter operably linked to at least one antibody light chain coding sequence and at least one antibody heavy chain coding sequence, wherein such antibody coding sequences are separated by at least one IRES. In this expression system, the gene that is 3' -most of the promoter has at its 3' terminus a poly A sequence, the other coding sequences in the polycistron lack a poly A sequence, and each gene is preceded by a start codon and ends with a stop codon.

Another embodiment of the invention provides a polycistronic expression unit comprising in the 5' to 3' direction the CMV promoter operably linked to an antibody light chain coding sequence that is flanked by a start and a stop codon, followed by one or more antibody heavy chain coding sequences. Each heavy chain coding sequence is also flanked by a start and a stop codon. Each pair of heavy chain coding sequences is separated by at least one IRES, preferably that of a cardiovirus, such as human encephalomyocarditis virus or poliovirus. The DNA sequence encoding the antibody light chain is preferably expressed at a ratio ranging between 10:1 1 and 1:2 relative to the expression of the DNA sequence encoding the antibody heavy chain. More preferably, the antibody light chain will be expressed at ratios relatively balanced with respect to the heavy chain, i.e., from about 5/1 to 1/1 and still more preferably about 3/1 to 1/1, and still more preferably about 1.5/1 to 1/1.

Another embodiment of the invention is a eukaryotic cell line, such as a CHO cell line, that secretes an antibody, wherein expression of said antibody is via a polycistron as described herein. In one aspect of the invention the polycistron comprises in the 5' to 3' orientation following the CMV promoter: a secreting signal; an antibody light chain coding sequence which comprises a start and a stop codon, but which does not comprise a poly A sequence; an IRES preferably selected from the group consisting of a cardiovirus, poliovirus and a herpes virus; at least one antibody heavy chain coding sequence each operably linked to a secreting signal sequence; and 3' of each heavy chain coding sequence an IRES, preferably selected from the group consisting of a cardiovirus, poliovirus and a herpes virus, IRES, and a poly A sequence contained at the 3' end of the gene located at the 3' end of the polycistron. Preferably, a eukaryotic cell containing the polycistron will secrete about 5-100 picograms of functional antibody per cell, per day. Preferably, at least 1-5 picograms of antibody are secreted per cell, per day over at least a continuous 3-4 day period. In preferred embodiments, the eukaryotic cell is a CHO cell or a yeast cell, e.g., Pichia.

Another embodiment of the invention is a culture of mammalian or yeast cells comprising a polycistronic expression system capable of producing functional antibodies. Vectors containing polycistronic sequences according to the invention may be introduced into the mammalian or yeast cells. During cell culturing, desired exogenous DNA sequences may be introduced to target mammalian or yeast cells, such that exogenous DNA is inserted into the genome of the mammalian or yeast cell via homologous recombination. Depending upon the sequences employed, functional antibodies may be recovered from the biomass of the cell culture or from the cell culture medium. Methods of integrating genes at specific sites in eukaryotic, e.g., mammalian cells via homologous recombination and vectors suitable for such recombinant processes are disclosed in U.S. Patent Nos. 5,998,144, 5,830,698, and 6,413,777 the entire contents of which are incorporated herein by reference.

Yet another embodiment is a method of producing a functional antibody comprising culturing eukaryotic cells, preferably mammalian or yeast cells containing a polycistronic expression system that expresses antibody light and heavy chain sequences, and recovering functional antibodies from the cell culture. The functional antibodies may be produced in batch fed cell cultures at levels suitable for therapeutic use under conditions optimized for maximal commercial output. For example, CHO cells grown in batch fed cultures in which glucose levels are continuously controlled can produce recombinant protein for at least 12 days or more. See, for example, U.S. Patent 6,180,401 for a discussion relating to the output of recombinant protein by cells grown in batch fed cultures.

### BRIEF DESCRIPTION OF THE FIGURES

The invention is further illustrated in the Figures discussed herein, wherein:

Figure 1 depicts a schematic drawing of a polycistronic expression construct encoding HuCC49, a humanized anti-TAG72 antibody;

Figure 2 depicts a schematic of the NEOSPLA vector and the situs of the IRES between light and heavy chain sequences;

Figure 3 depicts a schematic of the NEOSPLA vector in which immunoglobulin light and heavy chain gene sequences are located in independent transcriptional cassettes;

Figure 4 is a Southern blot of polycistronic HuCC49 G418-resistant cell isolates 22E6 and 22B6 probed with HuCC49 heavy chain;

Figure 5 is a Southern blot of polycistronic HuCC49 G418-resistant cell isolates 25A2 and 22H10 probed with HuCC49 heavy chain and

Figure 6 shows the sequence of expression construct HuCC49 which is a humanized antibody that binds TAG 72.

Figure 7 schematically depicts construction of a polycistronic expression construct according to the invention. "L" indicates a leader sequence V_{L} indicates DNA encoding an antibody variable light chain and V_{H} indicates DNA encoding antibody variable heavy chain.

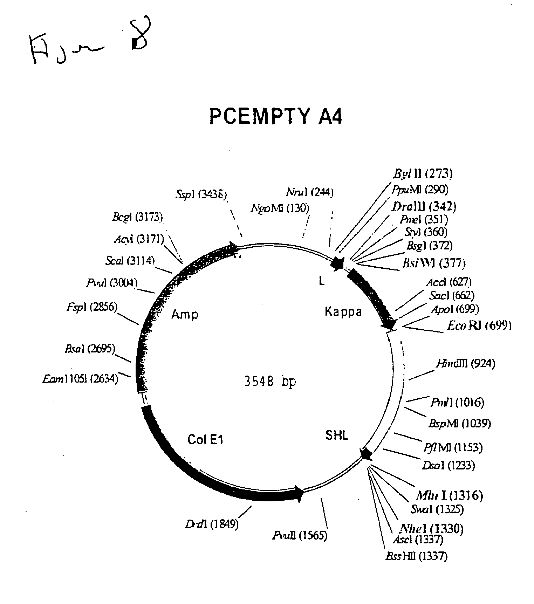

Figure 8 depicts a plasmid, PCEMPTY A4, used to construct a polycistronic vector according to the invention.

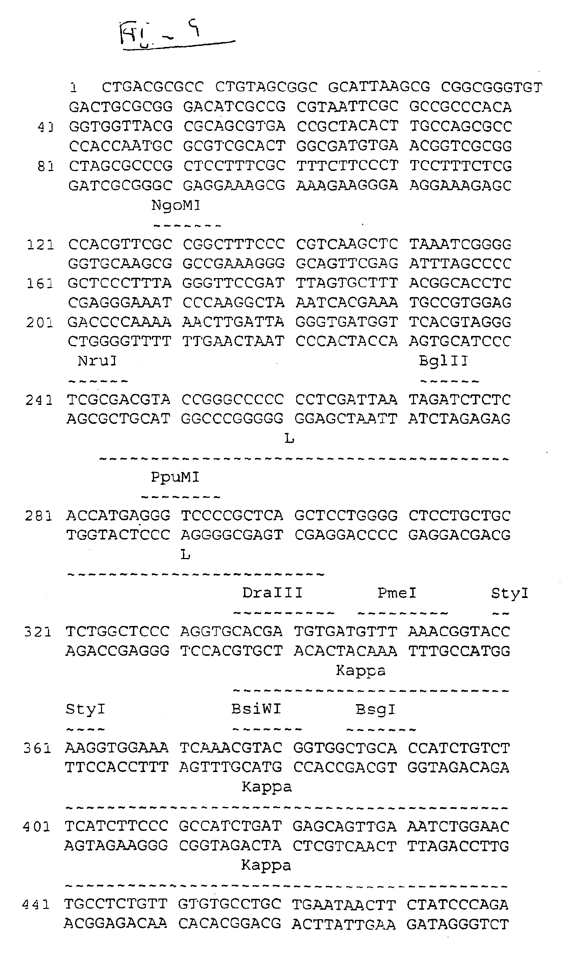

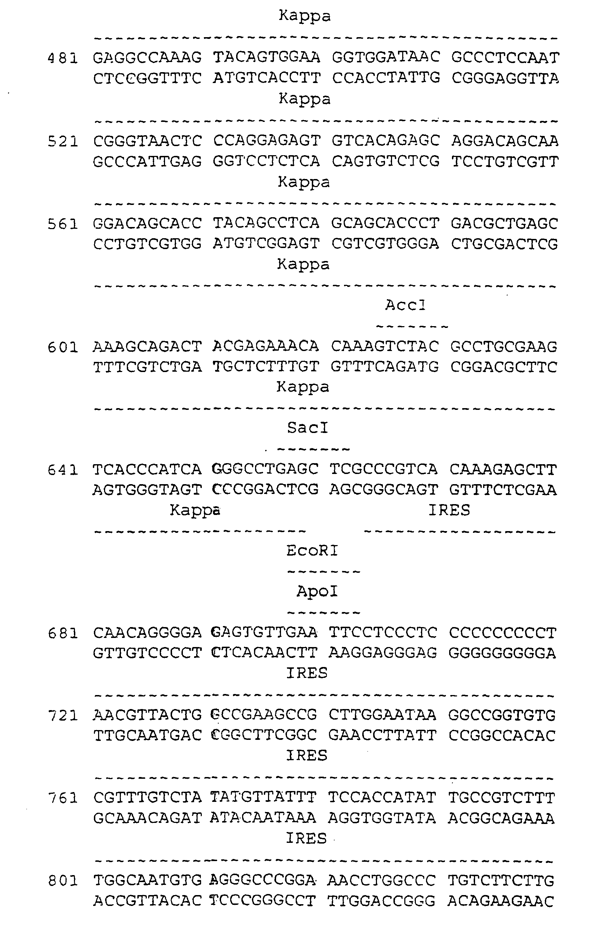

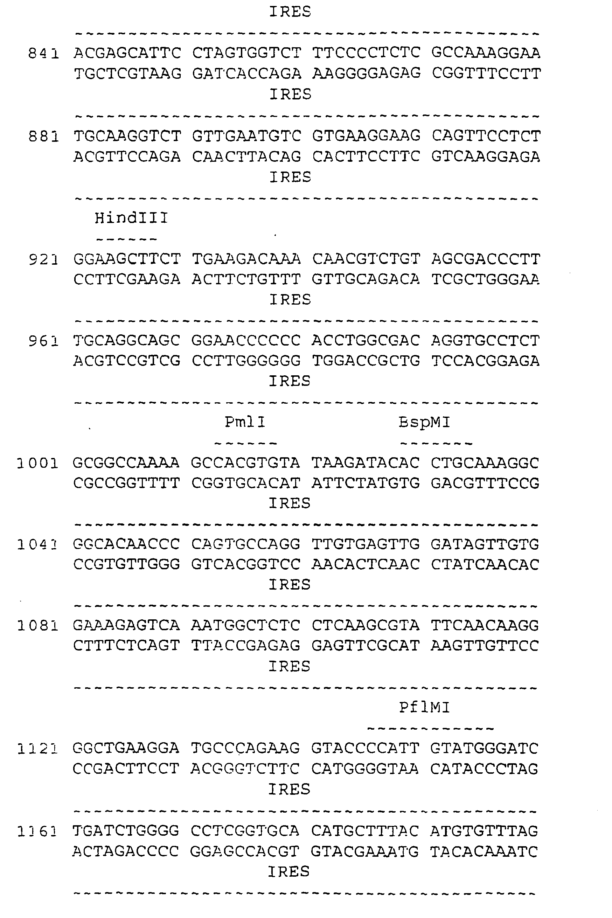

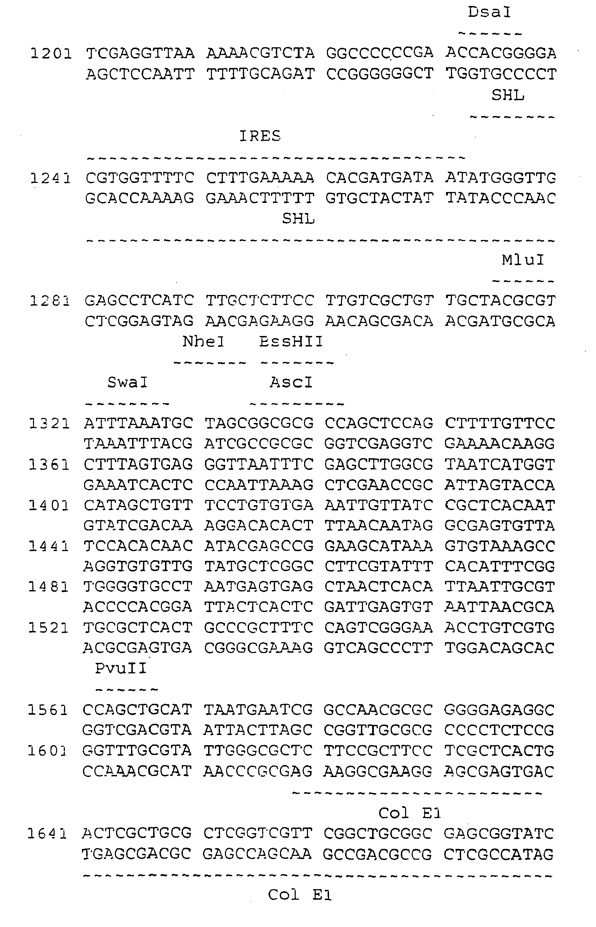

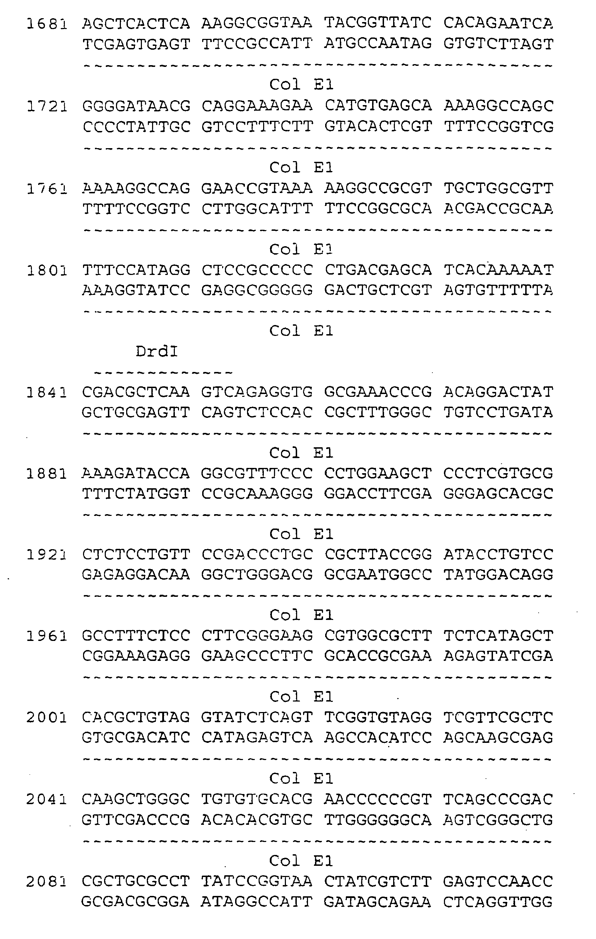

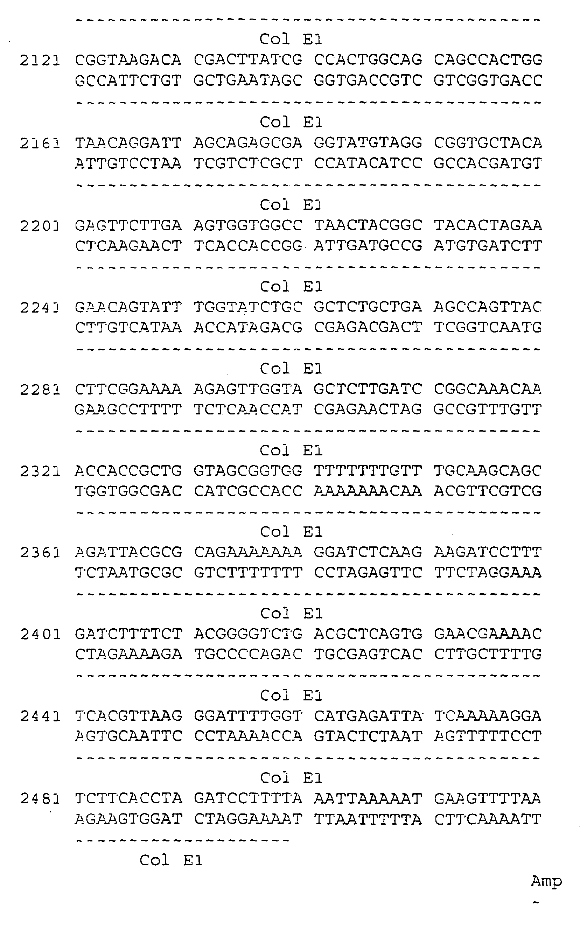

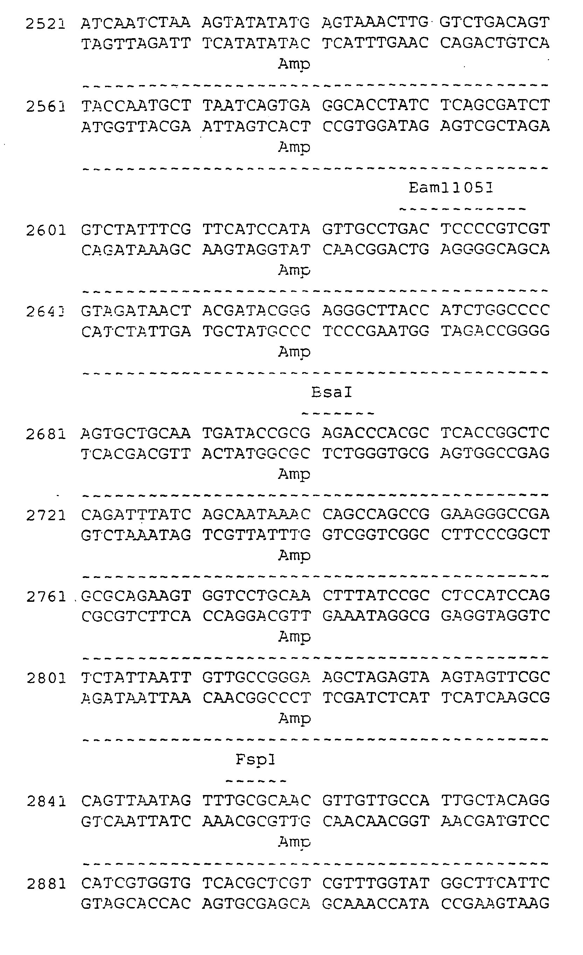

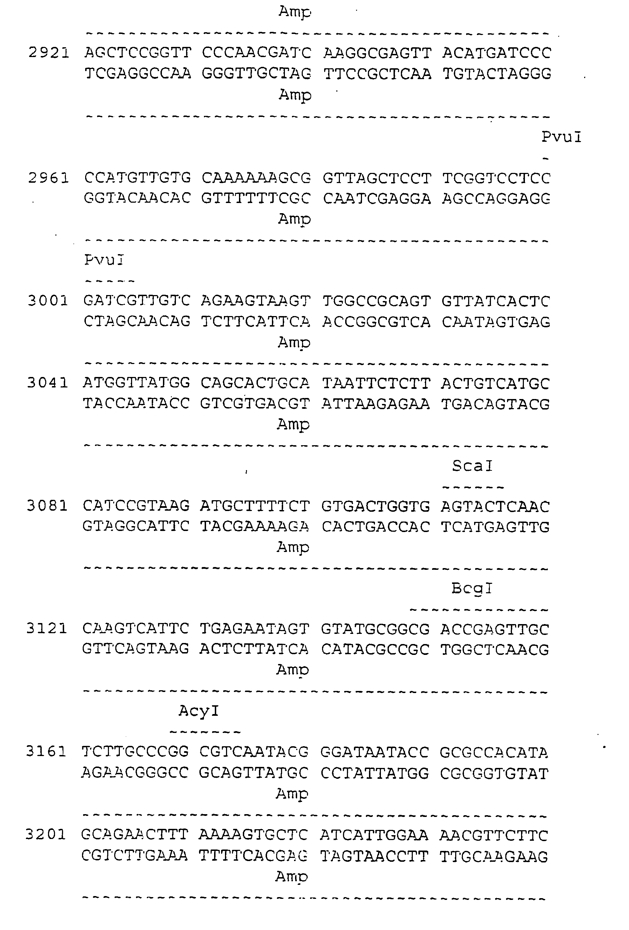

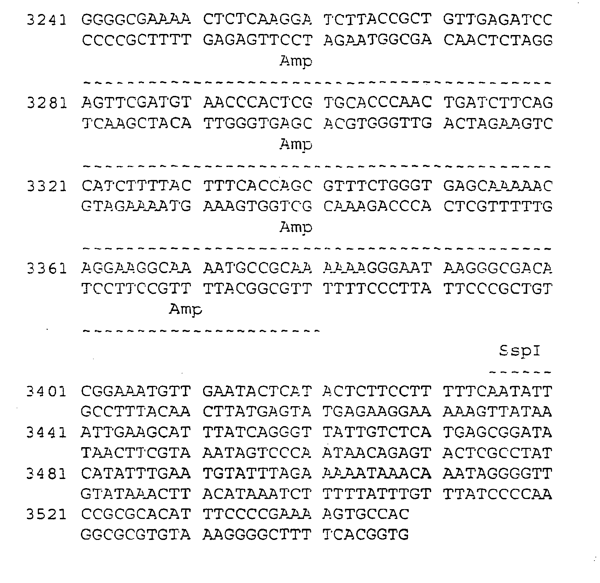

Figure 9 contains the nucleic acid sequence of PCEMPTY A4 plasmid.

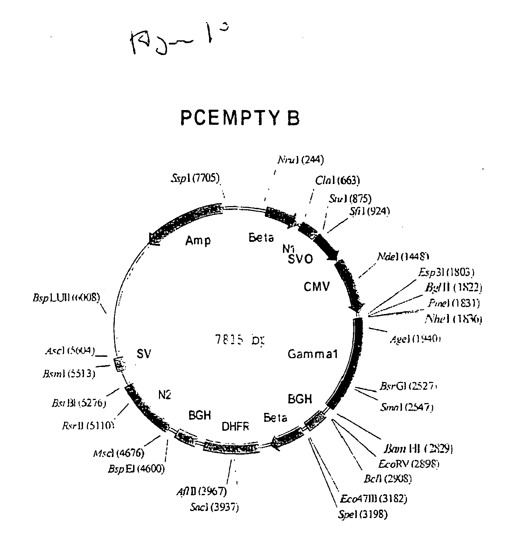

Figure 10 depicts a plasmid, PCEMPTY B, used to construct a polycistronic vector according to invention.

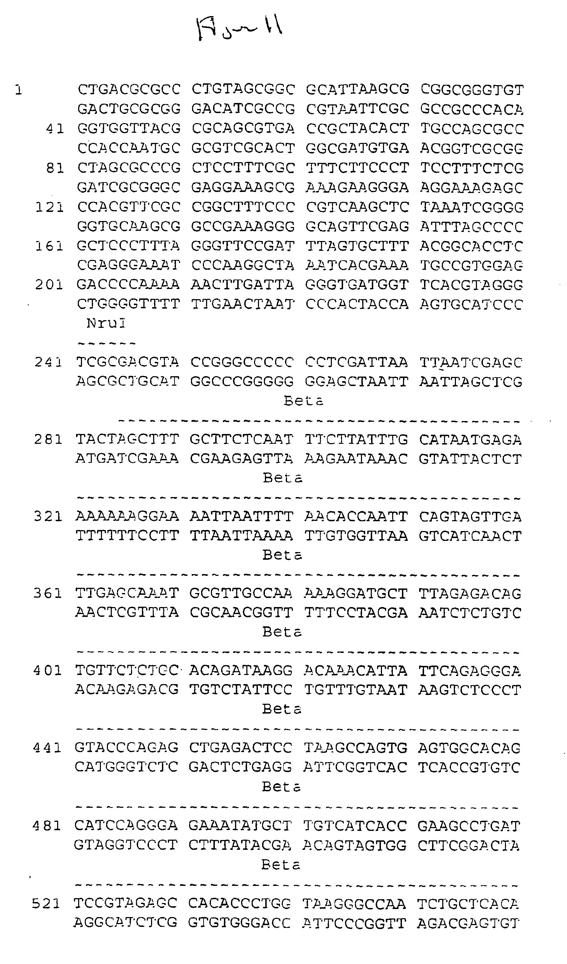

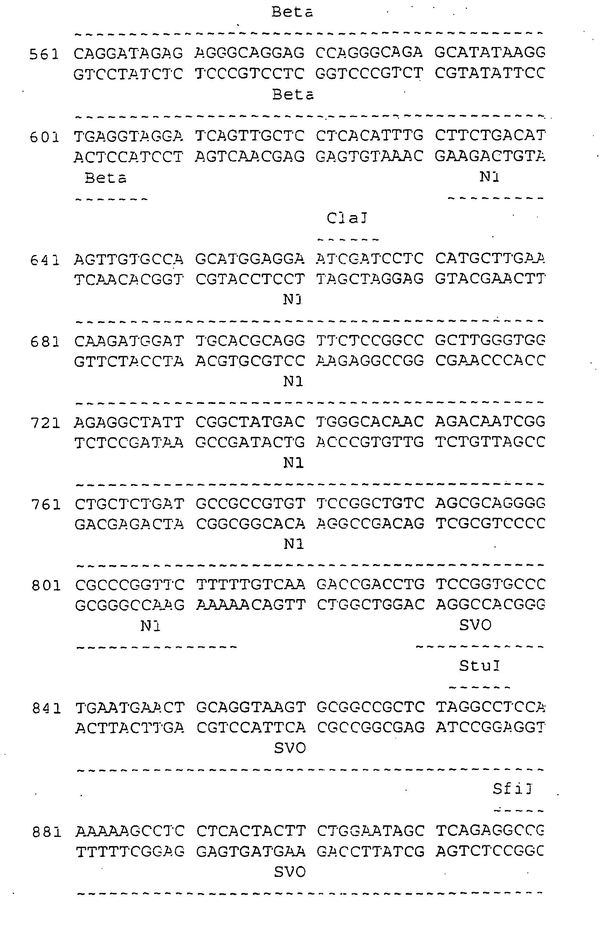

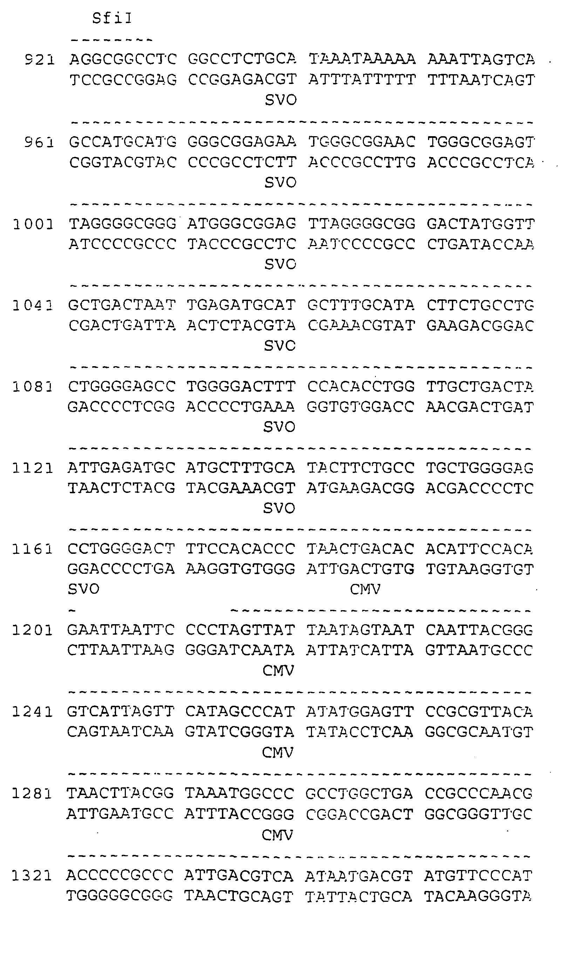

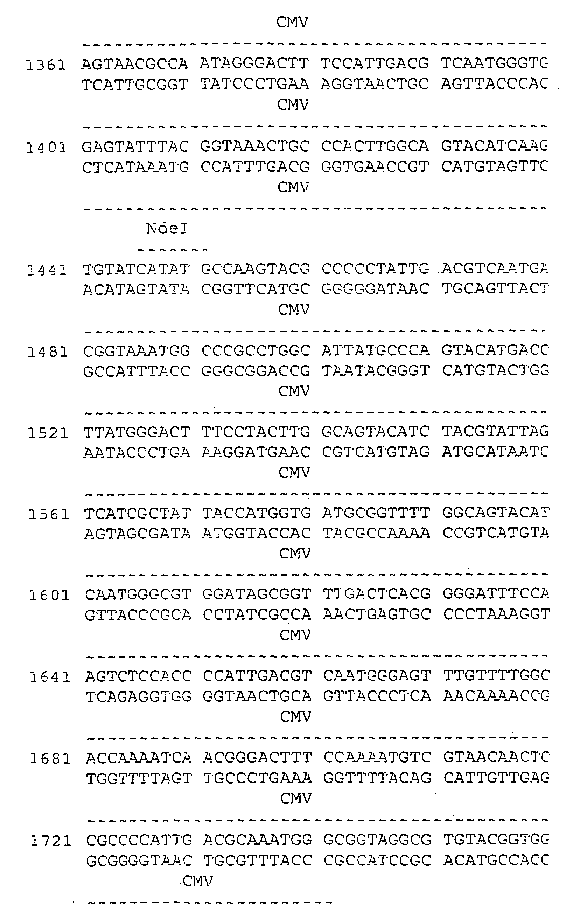

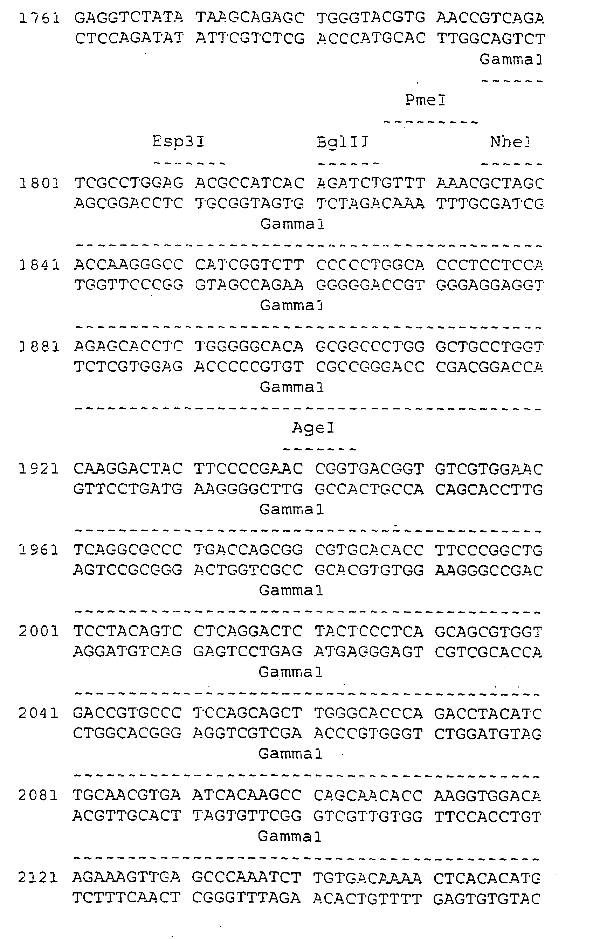

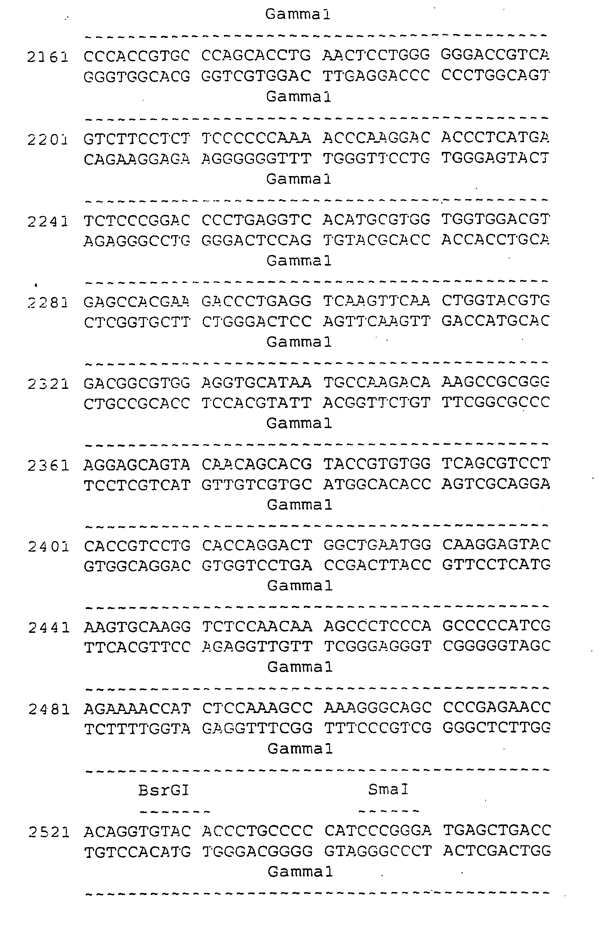

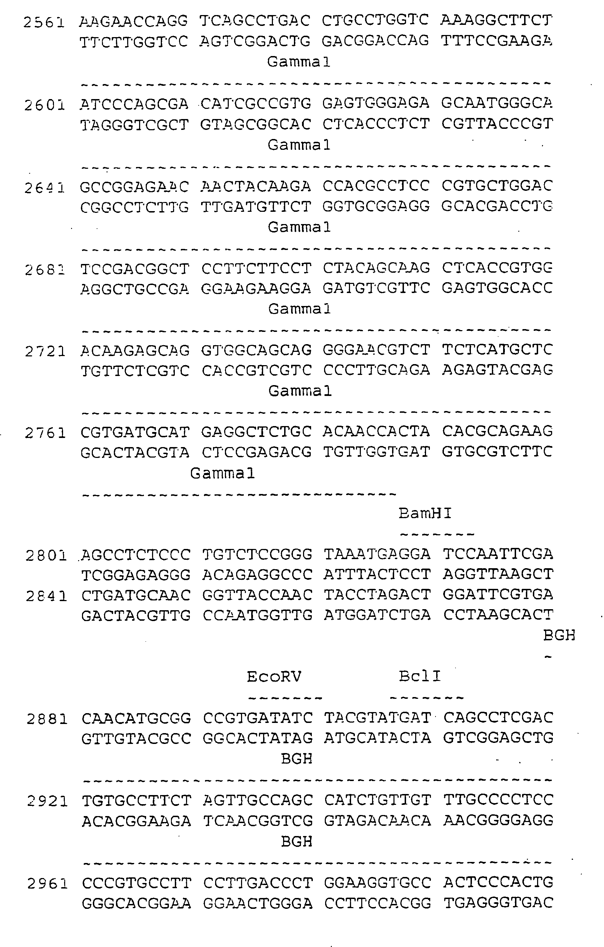

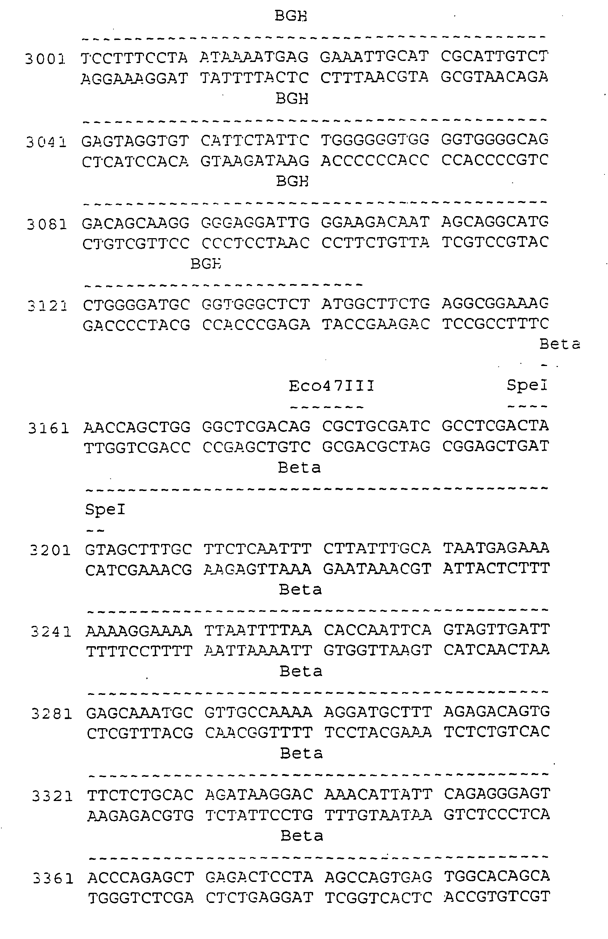

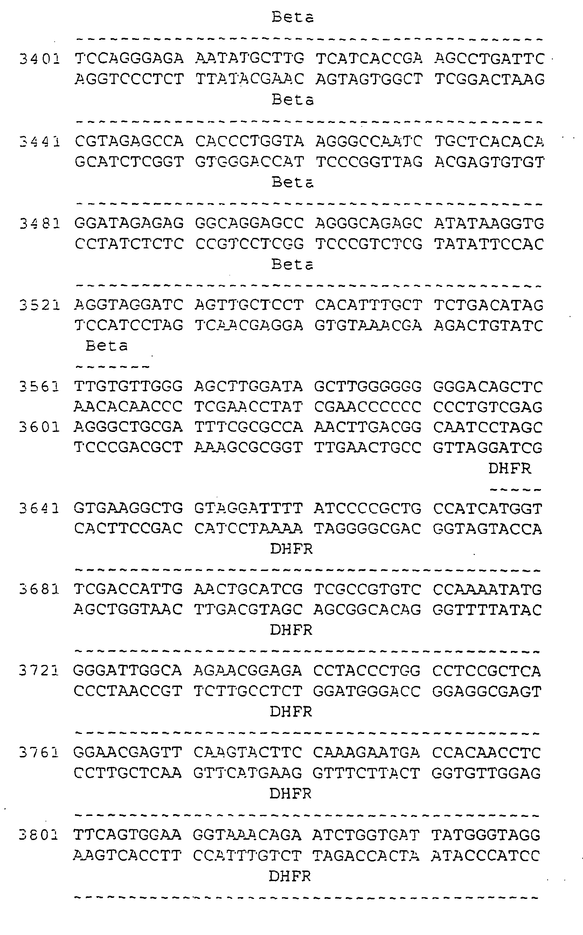

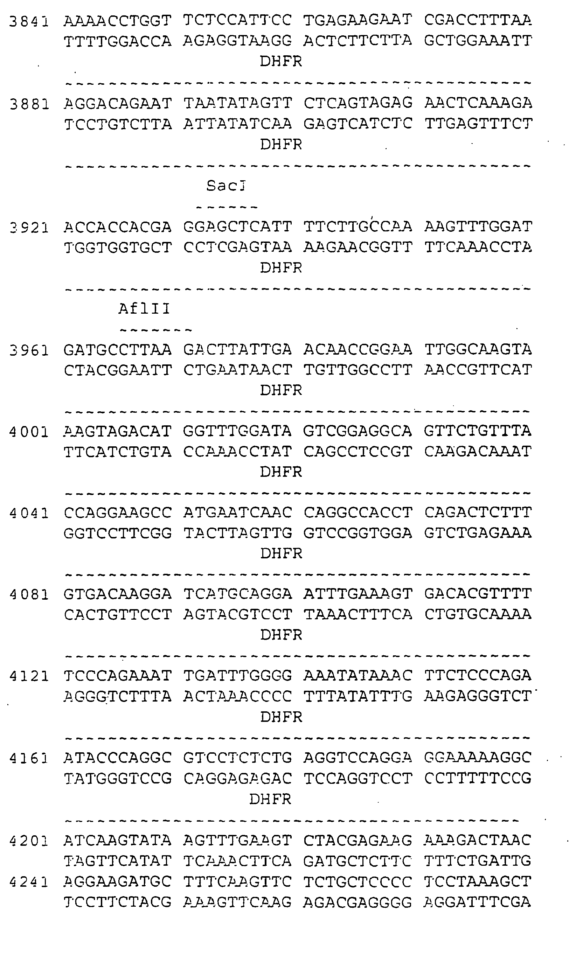

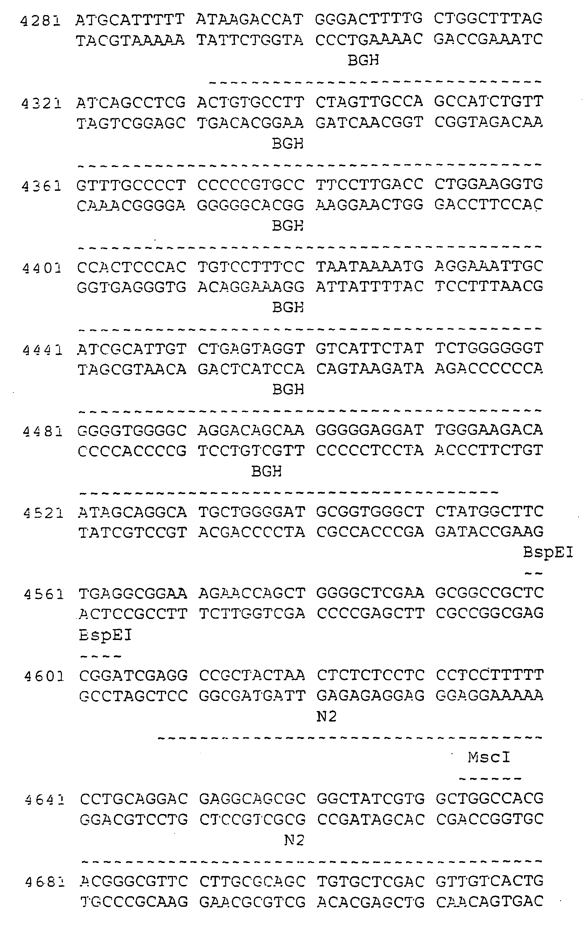

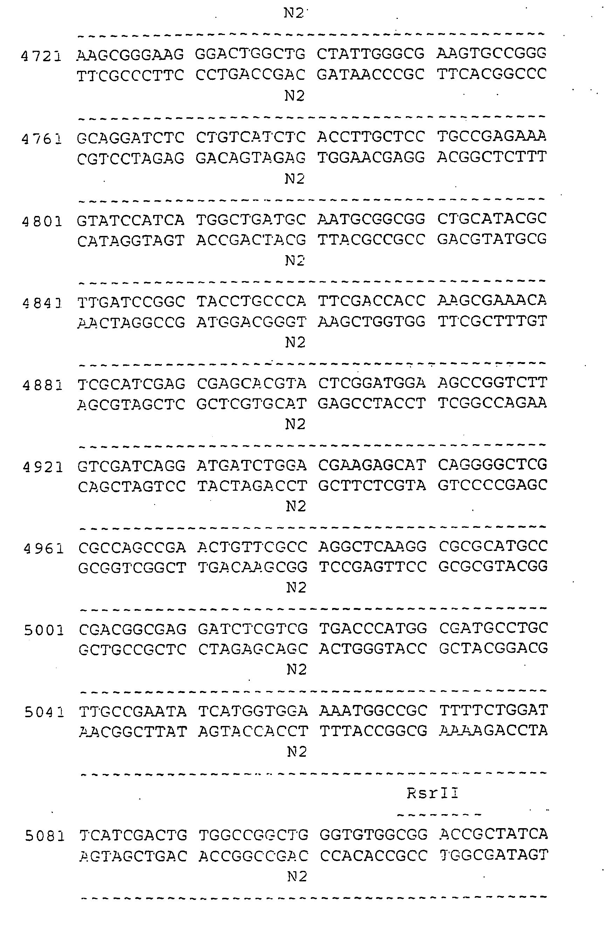

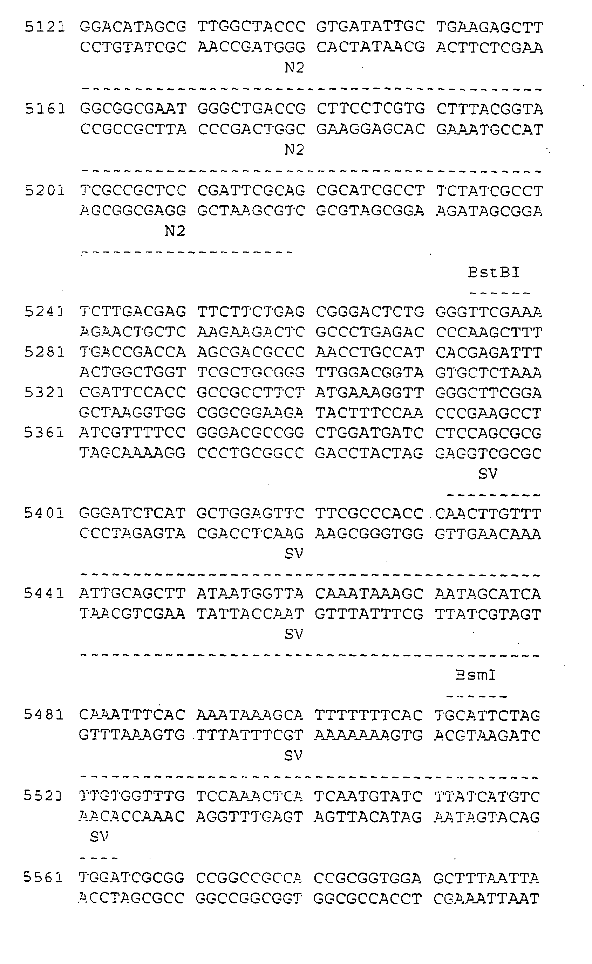

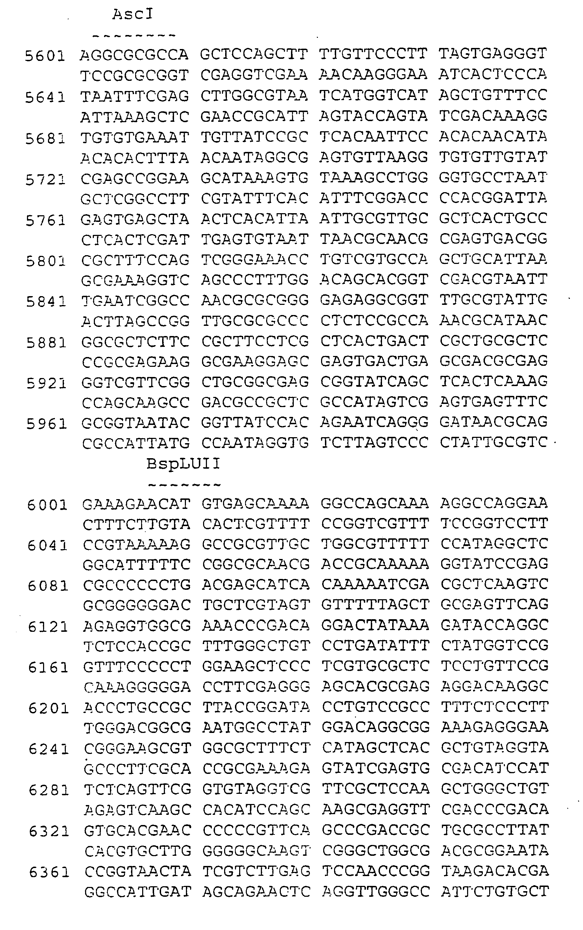

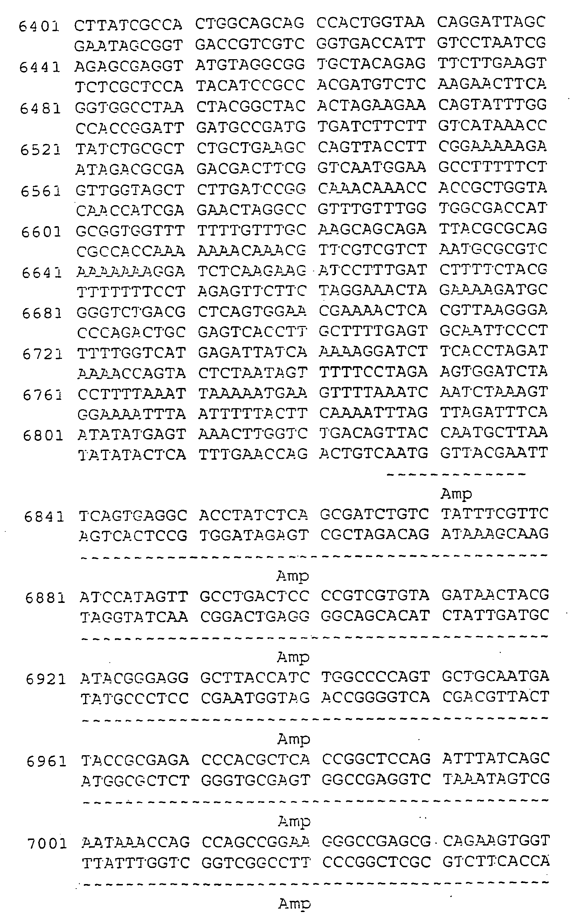

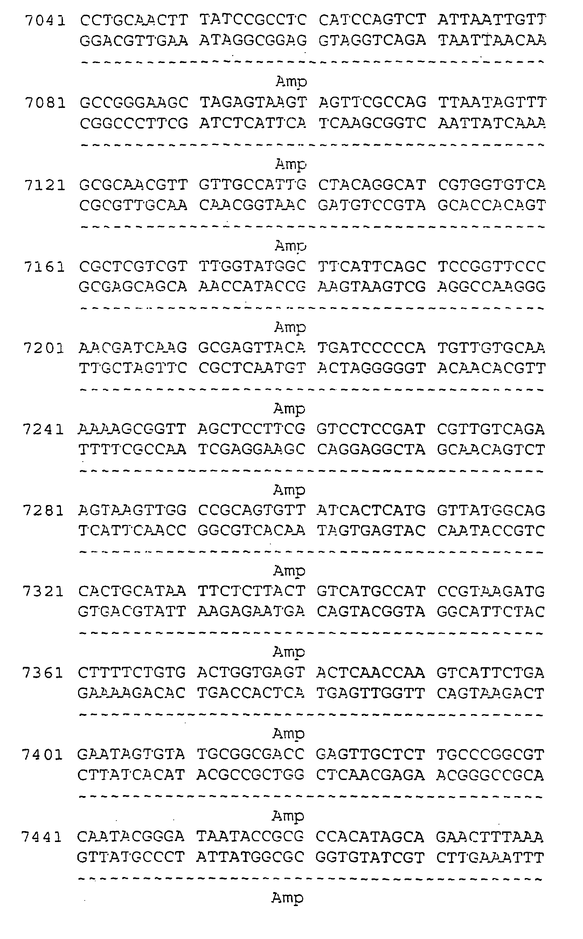

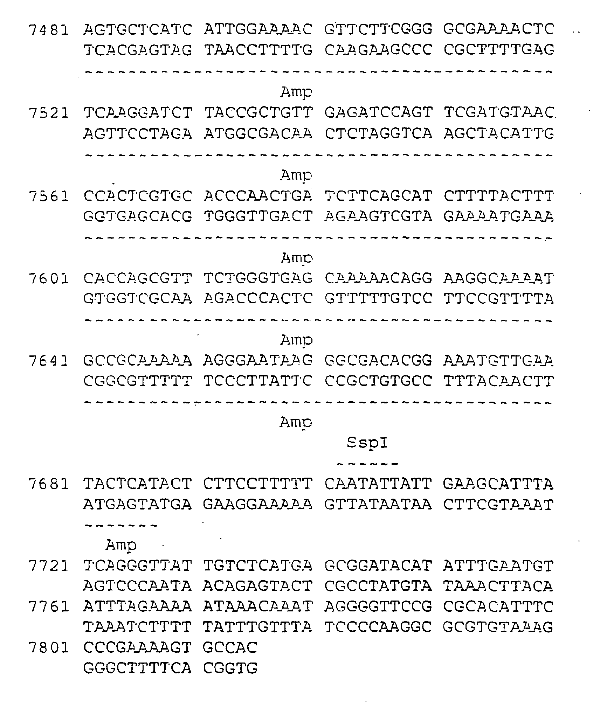

Figure 11 contains the nucleic acid sequence of the plasmid PCEMPTY B.

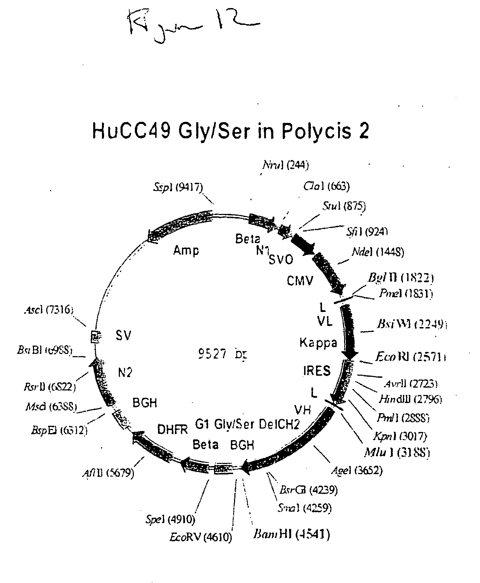

Figure 12 depicts a polycistronic vector according to the invention, Polycis 2 which expresses HuCC49 Gly/Ser.

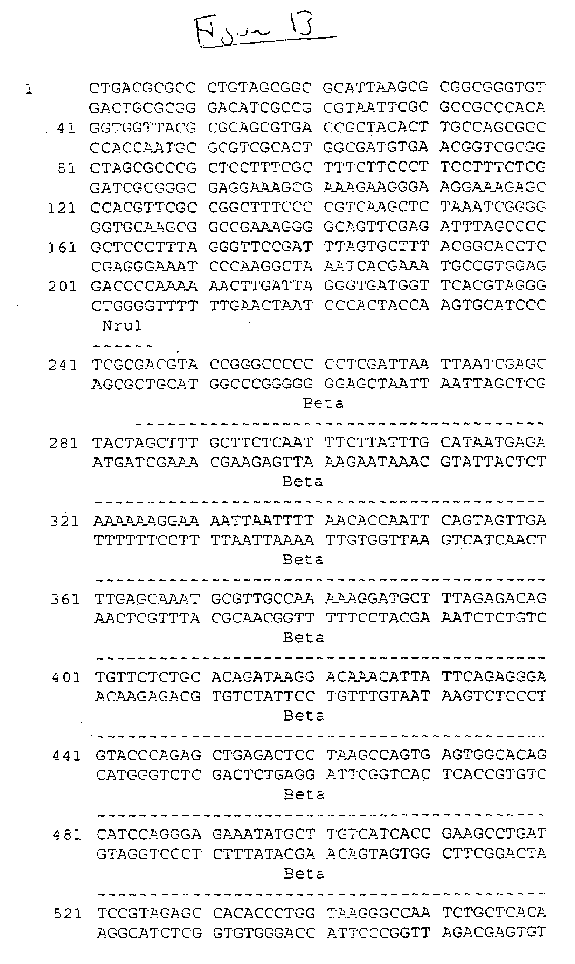

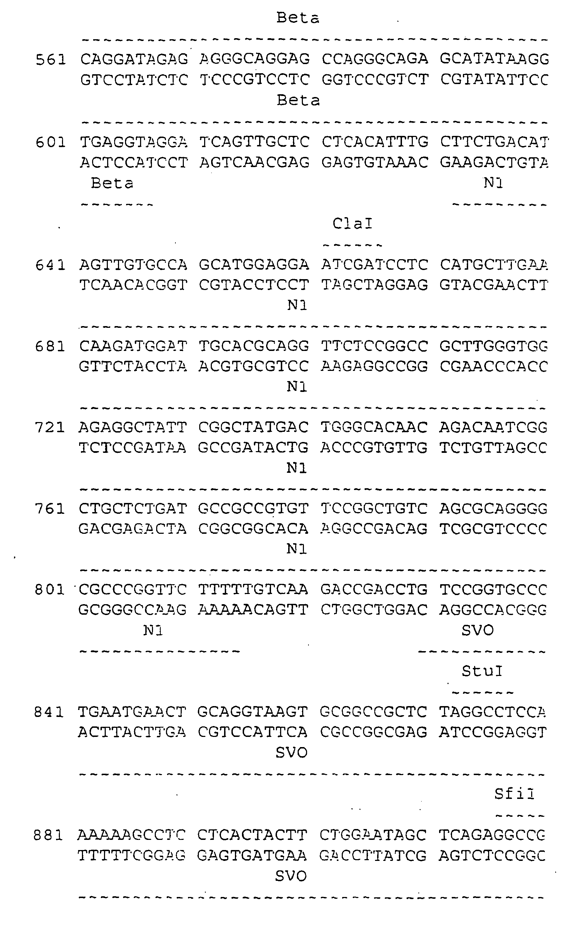

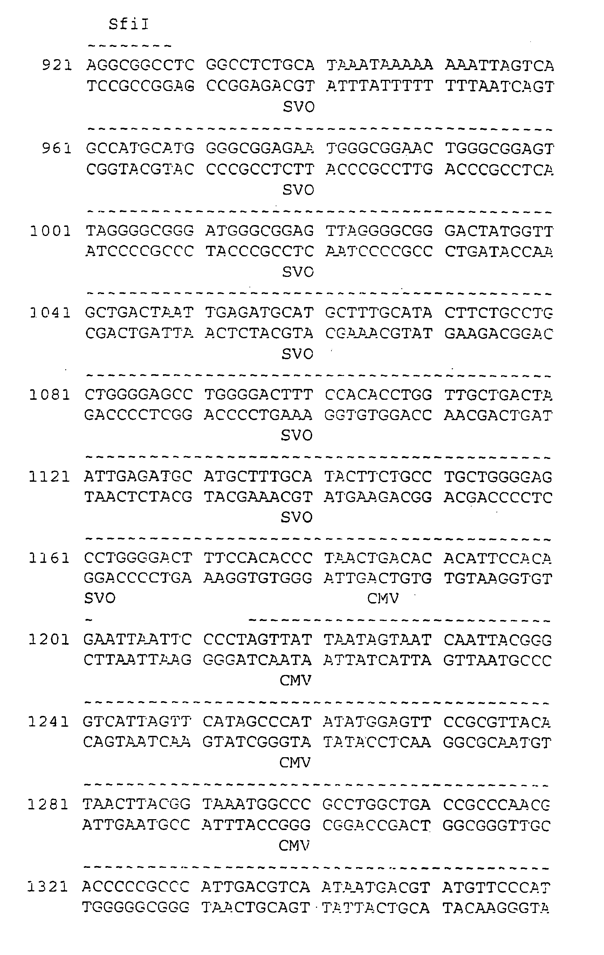

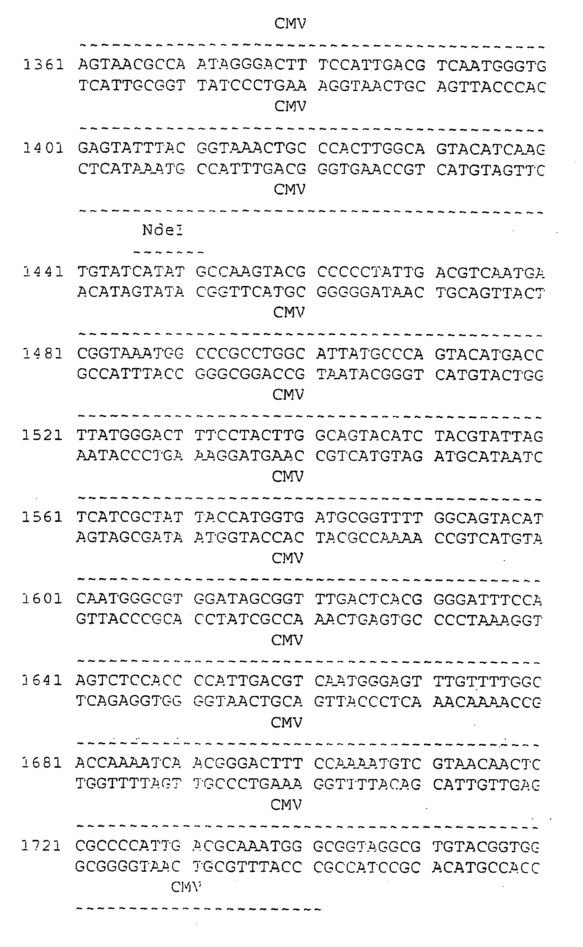

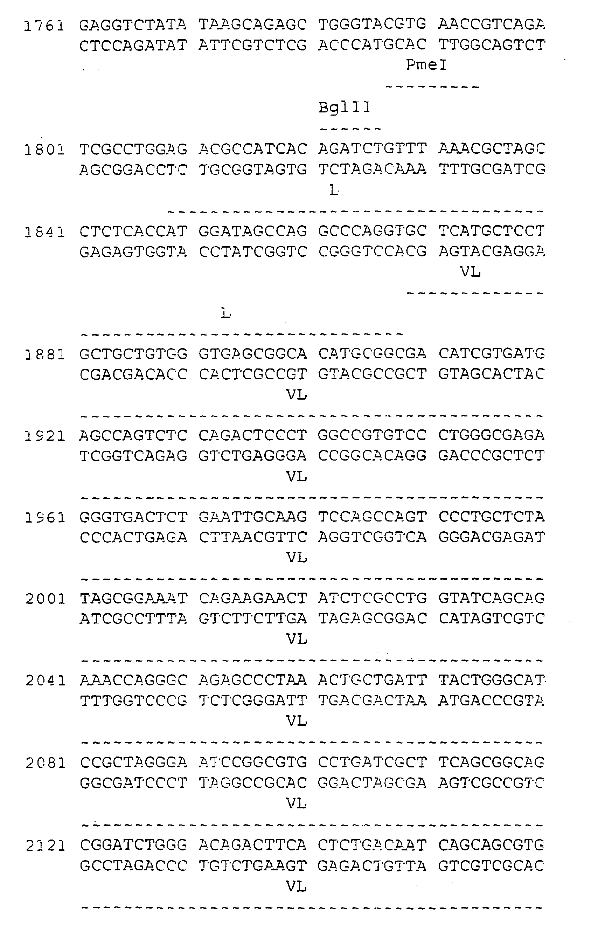

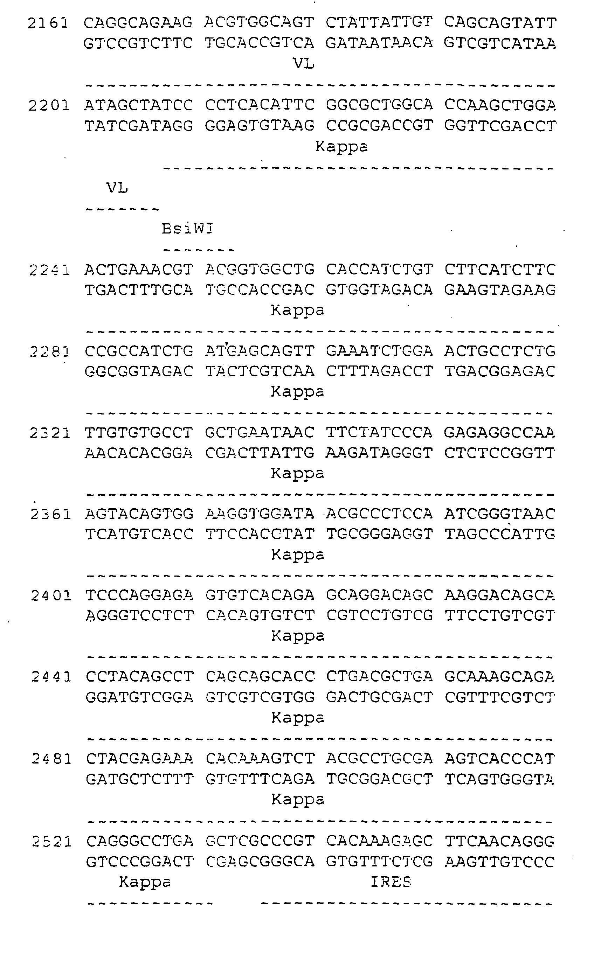

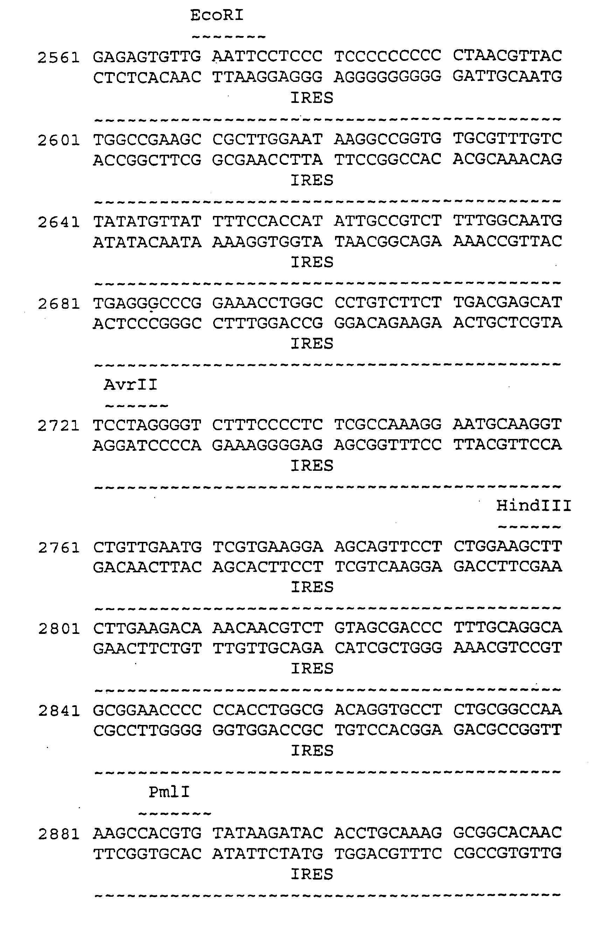

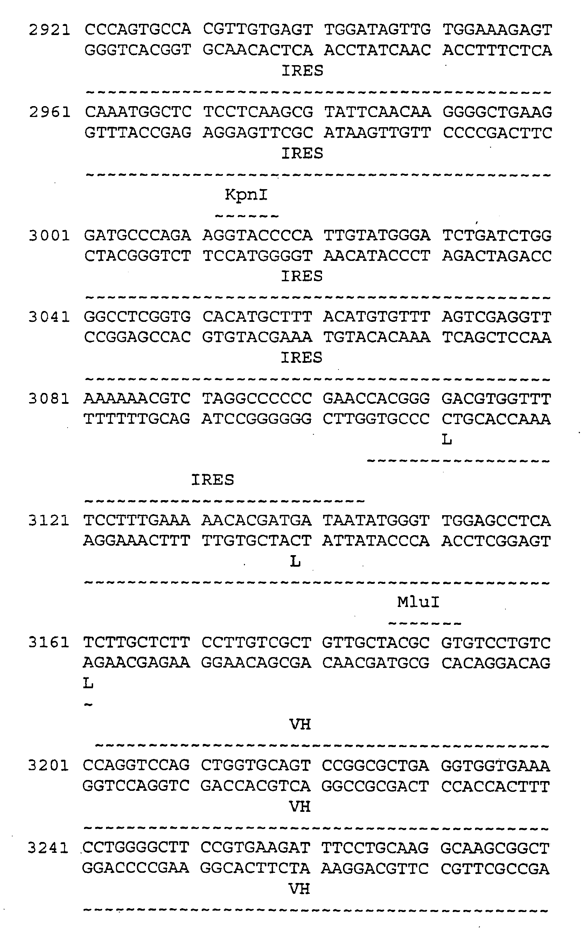

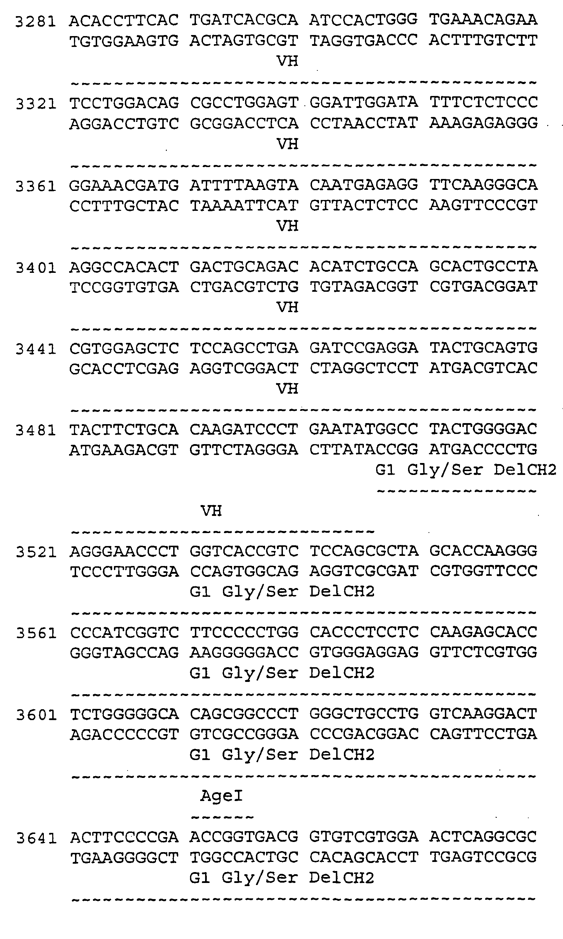

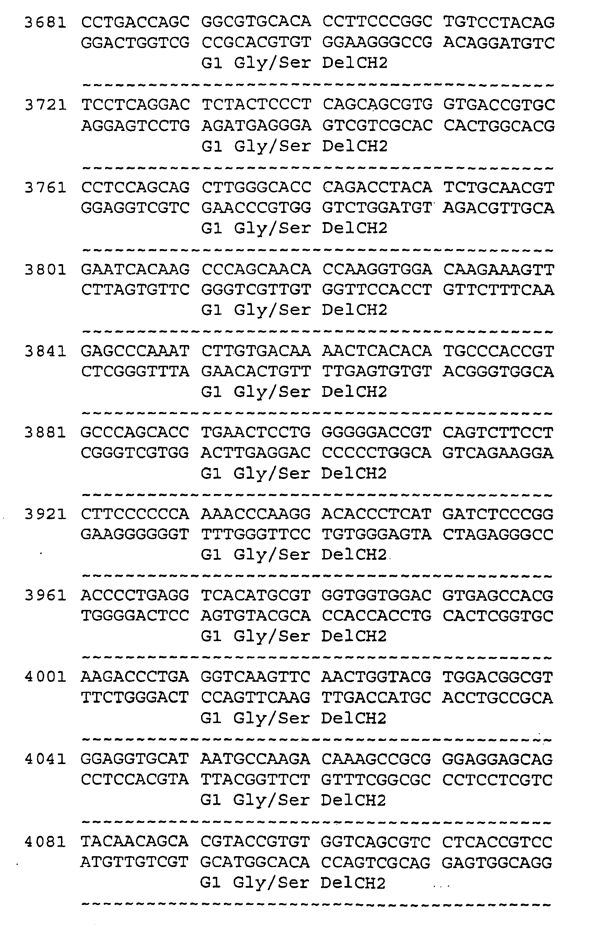

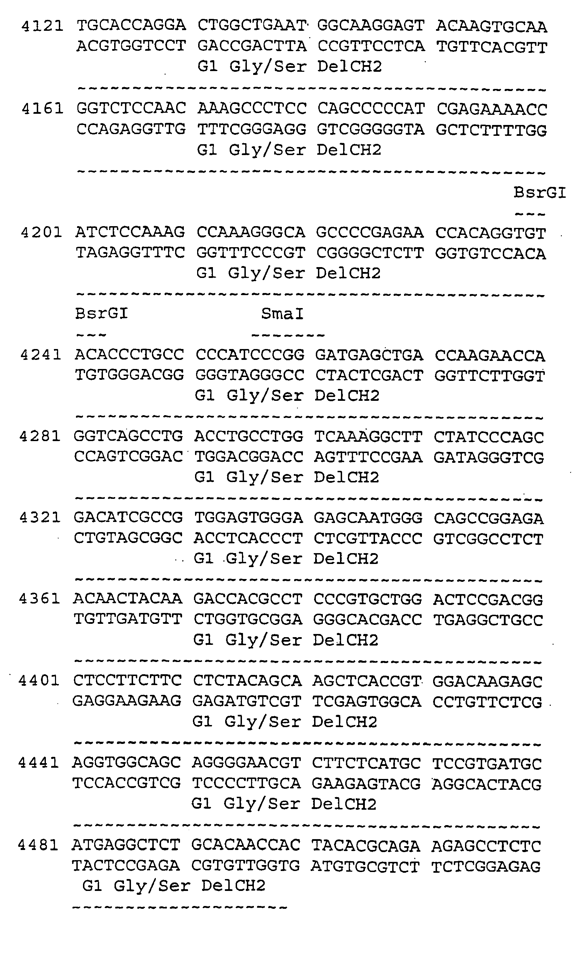

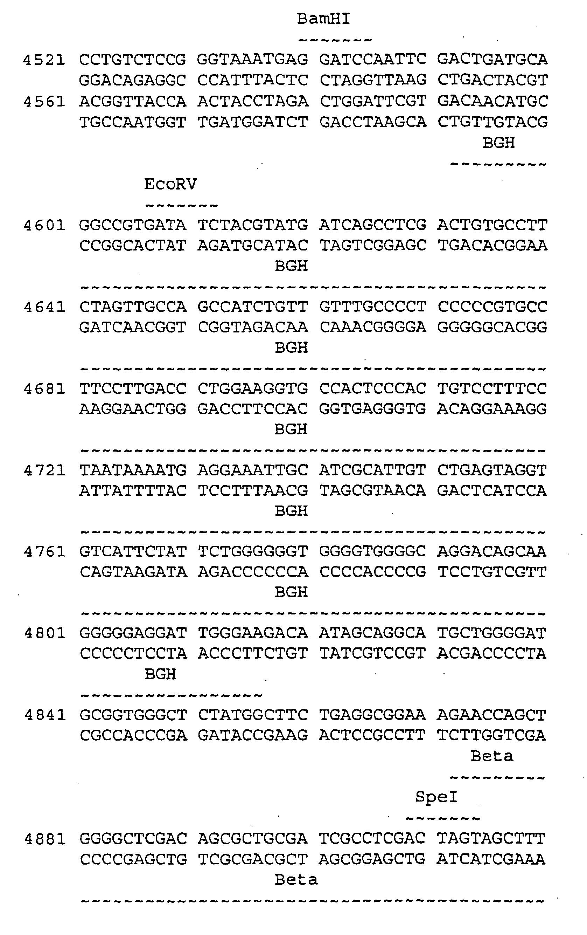

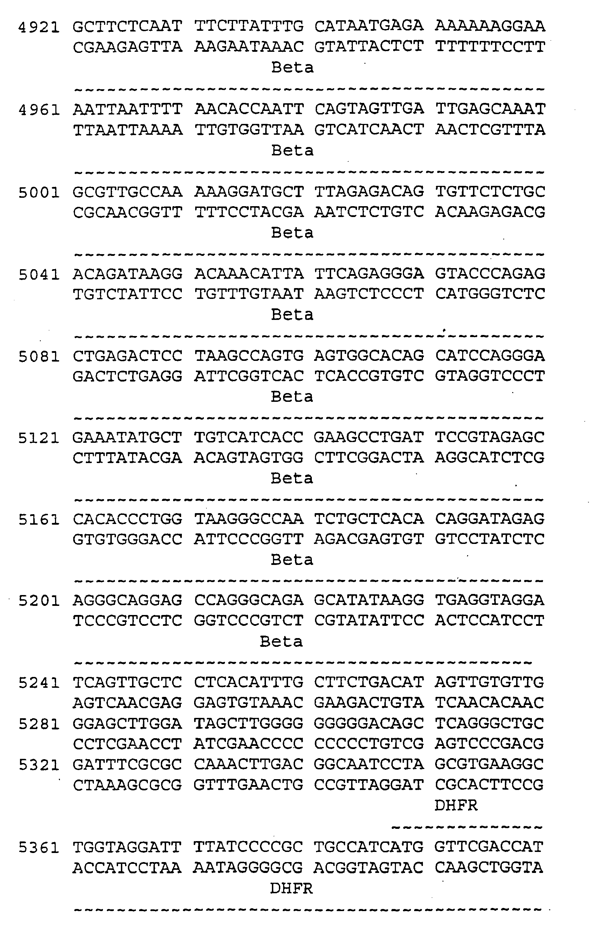

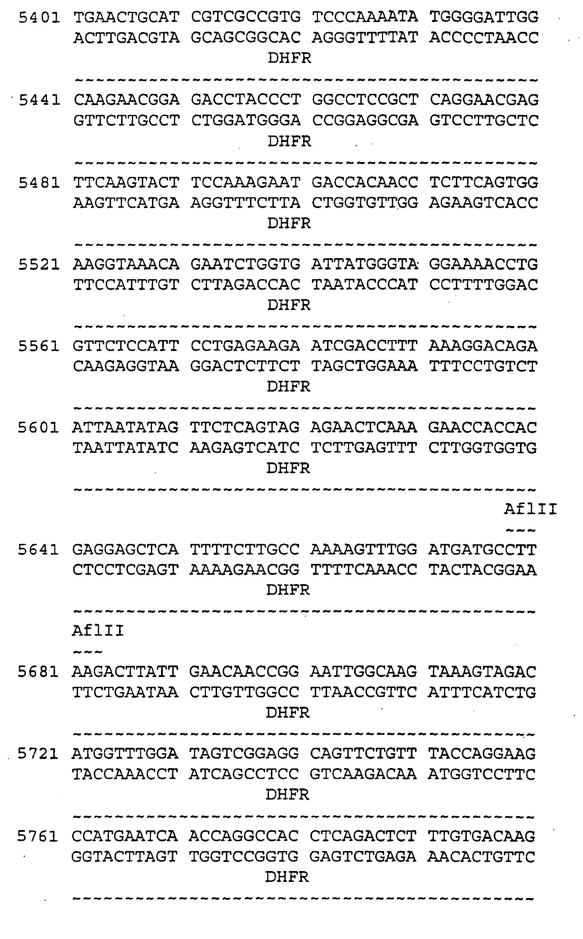

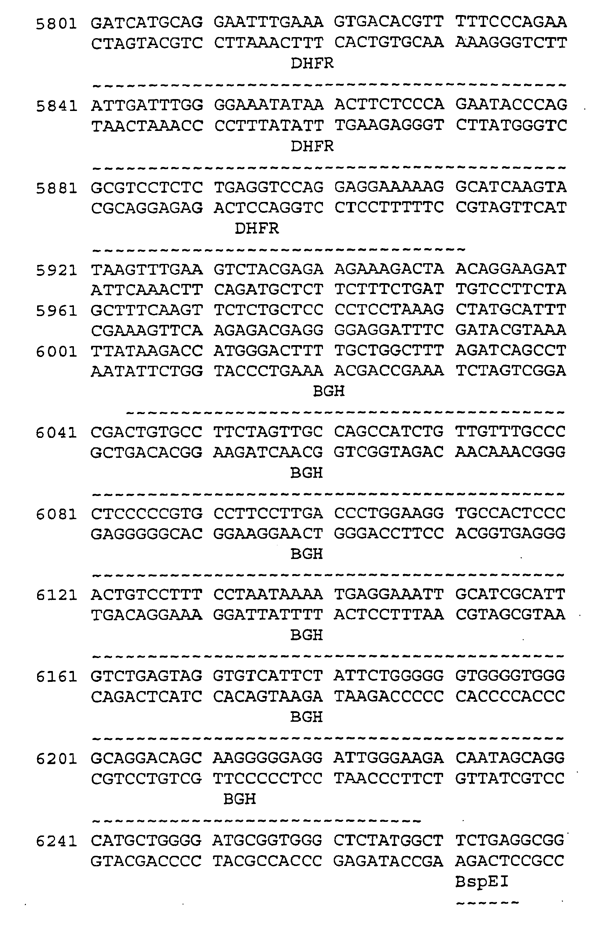

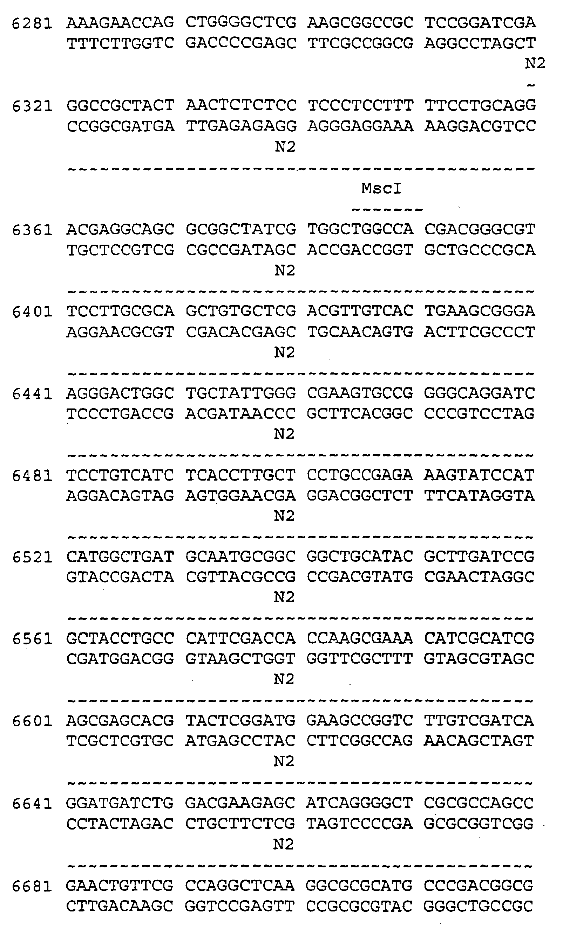

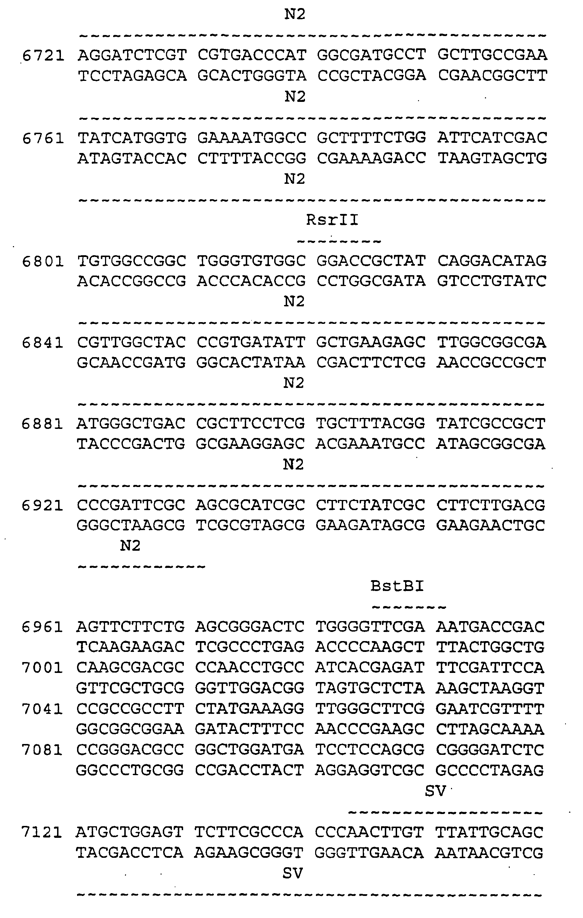

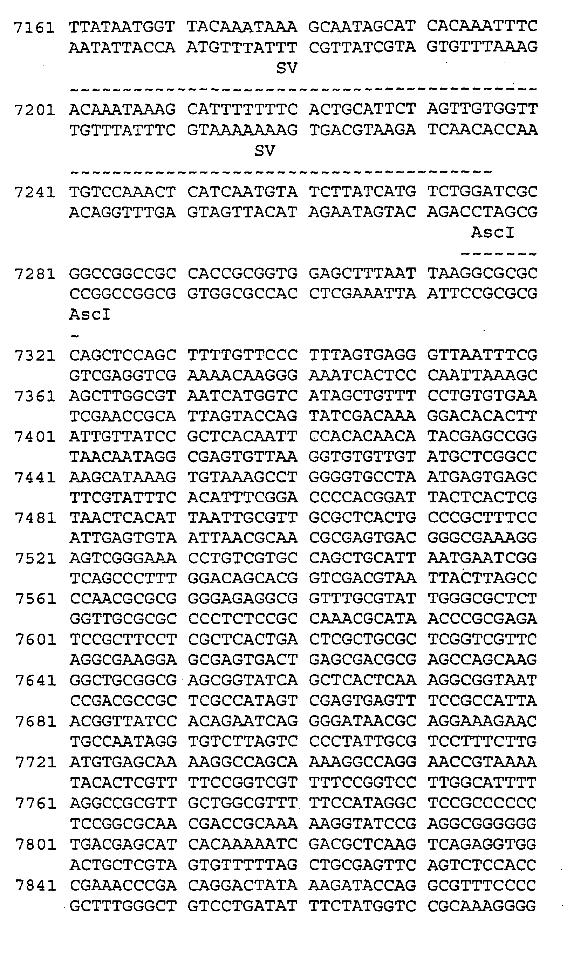

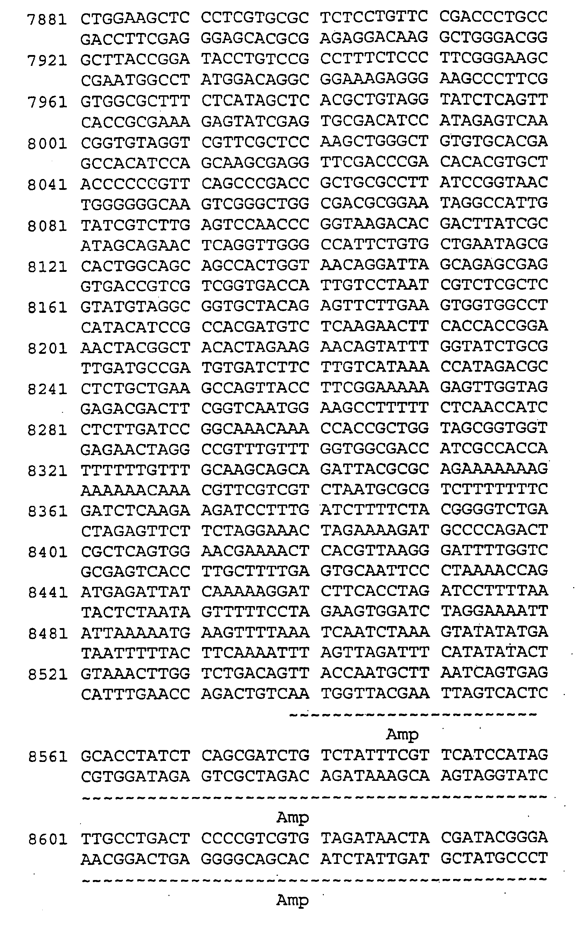

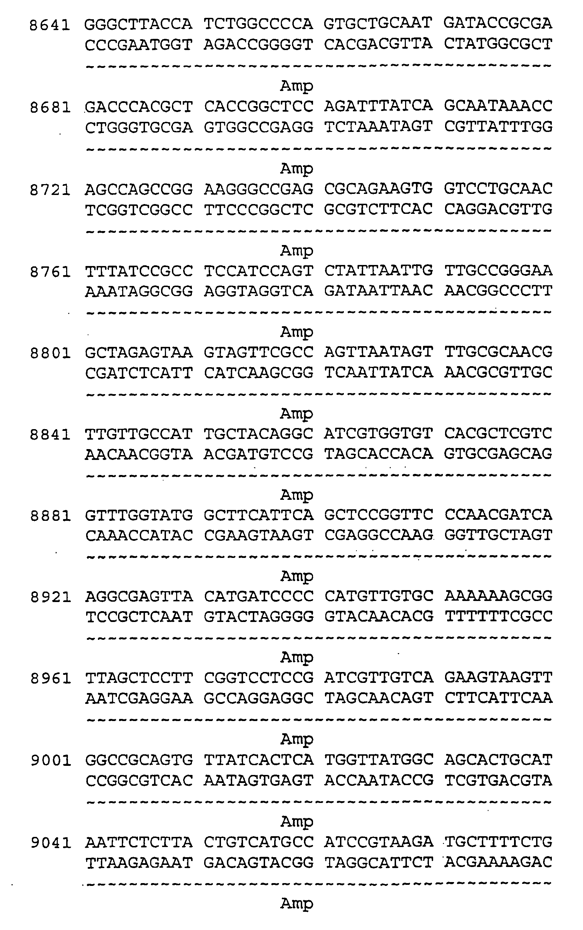

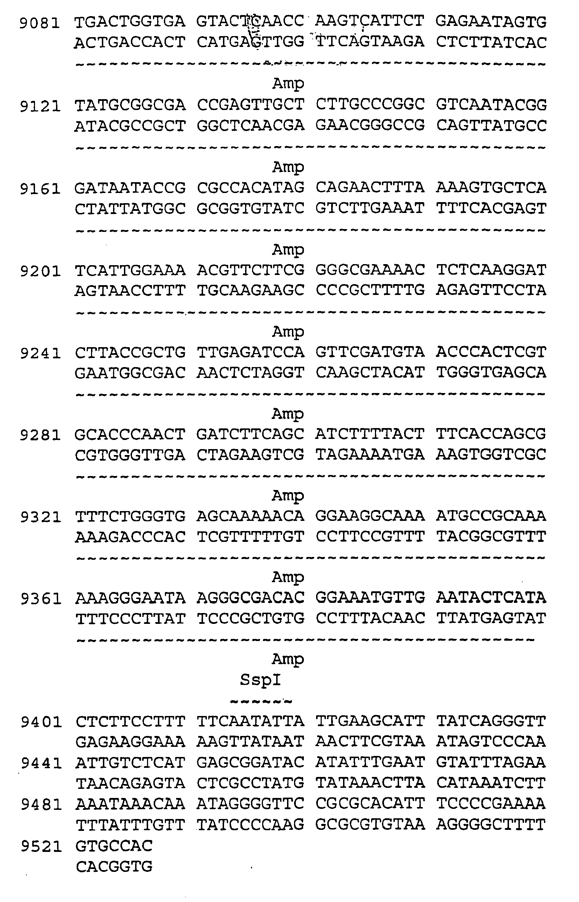

Figure 13 contains the nucleic acid sequence of HuCC49 Gly/Ser contained in Polycis 2 vector.

### DETAILED DESCRIPTION OF THE EMBODIMENTS

Prior to providing a detailed description of preferred embodiments, the following definitions are provided.

"IRES" or an internal ribosome entry site, means a region of a nucleic acid molecule e.g., an mRNA molecule, that allows internal ribosome entry/binding sufficient to initiate translation in an assay for cap-independent translation, such as the bicistronic reporter assay described in U.S. Patent 6,715,821. The presence of an IRES within a mRNA molecule allows cap-independent translation of a linked protein-encoding sequence that otherwise would not be translated. IRES's were first identified in picornaviruses, and are considered the paradigm for cap-independent translation. The 5' UTRS of all picornaviruses are long and mediate translational initiation by directly recruiting and binding ribosomes, thereby circumventing the initial cap-binding step.

IRES elements are frequently found in viral mRNAS, and are rarely found in non-viral mRNAs. To date, the non-viral mRNAS shown to contain functional IRES elements in their respective 5' UTRS include those encoding immunoglobulin heavy chain binding protein (BIP) (Macejak, D.J. et al., Nature 353:90-94 (1991)); Drosophila Antennapedia (Oh, S.K. et al., Genes Dev. 6:1643-53 (1992)); and Ultrabithoran (Ye, X. et al., Mol. Cell. Biol. 17:1714-21 (1997)); fibroblast growth factor 2 (Vagner et al., Mol. Cell. Biol. 15:35-47 (1915); initiation factor (Gan et al., J. Biol. Chem. 273:5006-12 (1992)); protein-oncogene c-myc (Nambru et al., J. Biol. Chem. 272:32061-6 (1995)); Stonely M. Oncogene 16:423-8 (1998)); Vascular endothelial growth factor (VEGF) (Stein J. et al., Mol. Cell. Biol. 18:3112-9 (1998)). Cellular IRES elements have no obvious sequence or structural similarity to IRES sequences or to each other and therefore are identified using translational assays. Another known IRES is the XIAP IRES disclosed in U.S. Patent 6,171,821, incorporated by reference in its entirety herein.

"Cap-dependent translation" means that 7-guano, 7-methylguanosine cap must be present at the 5' end of an mRNA molecule in order to initiate translation of the mRNA into a protein.

"Cap-independent translation" means that a 7-methylguanosine cap is not required for translation of the mRNA molecule. Cap-independent translation mechanisms include ribosome re-initiation, ribosome shunting, and internal ribosome binding.

"Cistron" means a "coding sequence" or sequence of nucleic acid that encodes a single protein or polypeptide.

"Reporter cistron" means a segment of nucleic acid that encodes a detectable gene product; which may be expressed under the translation control of an IRES.

"Reporter gene" means any gene or translatable nucleic acid sequence that encodes a product whose expression is detectable and/or quantifiable by immunological, chemical, biochemical or biological assays. A reporter gene may have e.g., one of the following attributes: fluorescence (e.g., green fluorescent protein), toxicity (e.g., ricin) enzymatic activity (e.g., lacz/beta-galactoidase, luciferase, chloramphenicol transferase), and an ability to be bound by a second molecule (e.g., biotin or a detectable labeled antibody).

"Promoter" means a minimal sequence sufficient to direct transcription, preferably in a eukaryotic cell. Specifically, included are promoter elements that are sufficient to render promoter-dependent gene expression controllable in a cell type-specific, tissue-specific, or temporal-specific manner, or inducible by external signals or agents, such elements may be located in the 5' or 3' or intron sequence regions of a particular gene. Preferred promoters for use in the invention include viral, mammalian and yeast promoters that provide for high levels of expression, e.g., mammalian CMV promoter, yeast alcohol oxidase, phosphoglycerokinase promoter, lactose inducible promoters, galactosidase promoter, adeno-associated viral promoter, baculovirus promoter, poxvirus promoter, retroviral promoters, adenovirus promoters, SV40 promoter HMG *hydroxymethylglutarylcoenzyne A) promoter, TK (thymidine kinase) promoter, 7.5K or H5R poxvirus promoters, adenovirus type 2 MPC late promoter, alpha-antrypsin promoter, factor IX promoter, immunoglobulin promoter, CFTR surfactant promoter, albumin promoter and transferrin promoter.

"Expression vector" means a DNA construct containing at least one promoter operably linked to a downstream gene, cistron or RNA coding region. Herein, the promoter may be operably linked to one or more genes or cistrons each preceded by a start and followed by a stop codon. Transfection of the expression vector into a recipient cells, i.e., eukaryotic cell, e.g., mammalian cell, fungal cell, yeast cell, allows the cell to express RNA encoded by the expression vector. Expression vectors include e.g., genetically engineered plasmids or viruses.

"Transformation" or "transfection" refers to introduction of a polycistronic vector or construct into suitable eukaryotic cells for expression of genes therein.

"Eukaryotic cells" refers to any eukaryotic cell which produces or expresses genes of interest using the polycistronic expression system of the invention. This includes by way of example mammalian cells such as CHO, myeloma, BHK, immune cells, insect cells, avian cells, amphibian cells, e.g., frog oocytes, fungal and yeast cells. Yeast useful for expression include by way of example Saccharomyces, Schizosaccharomyces, Hansenula, Candida, Torulopsis, Yarrowia, Pichia, et al. Particularly preferred yeast for expression include methylotrophic yeast strains, e.g., Pichia pastoris, Hansenula, polymorpha, Pichia guillermordii, Pichia methanolica, Pichia inositovera, et al. (See e.g., U.S. Patent 4,812,405, 4,818,700, 4,929,555, 5,736,383, 5,955,349, 5,888,768, and 6,258,559). These and other patents further describe promoters, terminators, enhancers, signals sequences, and other regulatory sequences useful for facilitating heterologus gene expression in yeast, e.g., antibody genes as in the present invention.

As is apparent from the instant specification, genetic sequences useful for producing the antibodies using the polycistronic expression system of the present invention may be obtained from a number of different sources. For example, a variety of human antibody genes are available in the form of publicly accessible deposits. Many sequences of antibodies and antibody-encoding genes have been published and suitable antibody genes can be synthesized from these sequences much as described herein. Alternatively, antibody-producing cell lines may be selected and cultured using techniques well known to the skilled artisan. Such techniques are described in a variety of laboratory manuals and primary publications. In this respect, techniques suitable for use in the invention as described below are described in Current Protocols in Immunology, Coligan et al., Eds., Green Publishing Associates and Wiley-Interscience, John Wiley and Sons, New York (1991) which is herein incorporated by reference in its entirety, including supplements.

It will further be appreciated that the scope of this invention further encompasses all alleles, variants and mutations of the DNA sequences described herein.

As is well known, antibody-encoding RNA may be isolated from the original antibody-producing hybridoma cells or from other transformed cells by standard techniques, such as guanidinium isothiocyanate extraction and precipitation followed by centrifugation or chromatography. Where desirable, mRNA may be isolated from total RNA by standard techniques such as chromatography on oligodT cellulose. Techniques suitable for these purposes are familiar in the art and are described in the foregoing references.

cDNAs that encode the light and the heavy chains of the antibody may be made, either simultaneously or separately, using reverse transcriptase and DNA polymerase in accordance with well known methods. It may be initiated by consensus constant region primers or by more specific primers based on the published heavy and light chain DNA and amino acid sequences. As discussed above, PCR also may be used to isolate DNA clones encoding the antibody light and heavy chains. In this case libraries may be screened by consensus primers or larger homologous probes, such as mouse constant region probes.

DNA encoding light and heavy chains may be isolated, typically in the form of plasmid DNA, from the cells as described herein, restriction mapped and sequenced in accordance with standard, well known techniques set forth in detail in the foregoing references relating to recombinant DNA techniques. Of course, the DNA may be modified according to the present invention at any point during the isolation process or subsequent analysis.

While the inventive compositions and methods are suitable for any antibody, or indeed any multichain protein, preferred antibody sequences are disclosed herein. Oligonucleotide synthesis techniques compatible with this aspect of the invention are well known to the skilled artisan and may be carried out using any of several commercially available automated synthesizers. In addition, DNA sequences encoding several types of heavy and light chains set forth herein can be obtained through the services of commercial DNA vendors. The genetic material obtained using any of the foregoing methods may then be altered or modified to provide antibodies compatible with the present invention and the desired use of such antibodies.

A variety of different types of antibodies may be expressed according to the instant invention. As used herein "antibody" and "antibodies" refers to assemblies which have significant known specific immunoreactive activity to an antigen (e.g. a tumor associated antigen), comprising light and heavy chains, with or without covalent linkage between them and thus include single chain antibodies, and the like. "Modified antibodies" according to the present invention are held to mean immunoglobulins, antibodies, or immunoreactive fragments or recombinants thereof, in which at least a fraction of one or more of the constant region domains has been deleted or otherwise altered so as to provide desired biochemical characteristics such as the ability to non-covalently dimerize, increased tumor localization or modified serum half-life when compared with a whole, unaltered antibody of approximately the same (scAb) immunogenicity. For the purposes of the instant application, immunoreactive single chain antibody constructs having altered or omitted constant region domains may be considered to be modified antibodies. As discussed above, preferred modified antibodies or domain deleted antibodies expressed using the polycistronic system of the present invention may have at least a portion of one of the constant domains deleted. More preferably, one entire domain of the constant region of the modified antibody will be deleted and even more preferably the entire C_{H}2 domain will be deleted.

Basic immunoglobulin structures (e.g., antibodies and the like) in vertebrate systems are relatively well understood. As will be discussed in more detail below, the generic term "immunoglobulin" comprises five distinct classes of antibody that can be distinguished biochemically. While all five classes are clearly within the scope of the present invention, the following discussion will generally be directed to the class of IgG molecules. With regard to IgG, immunoglobulins comprise two identical light polypeptide chains of molecular weight approximately 23,000 Daltons, and two identical heavy chains of molecular weight 53,000-70,000. The four chains are joined by disulfide bonds in a "Y" configuration wherein the light chains bracket the heavy chains starting at the mouth of the "Y" and continuing through the variable region. The dimeric assemblies disclosed herein may be likened to two associated Ys (H₄L₄) so there will be four binding sites. Hence the term "tetravalent" antibodies.

More specifically, both the light and heavy chains are divided into regions of structural and functional homology. The terms "constant" and "variable" are used functionally. In this regard, it will be appreciated that the variable domains of both the light (V_{L}) and heavy (V_{H}) chains determine antigen recognition and specificity. Conversely, the constant domains of the light chain (C_{L}) and the heavy chain (C_{H}1, C_{H}2 or C_{H}3) confer important biological properties such as secretion, transplacental mobility, Fc receptor binding, complement binding, and the like. By convention the numbering of the constant region domains increases as they become more distal from the antigen binding site or amino-terminus of the antibody. Thus, the C_{H}3 and C_{L} domains actually comprise the carboxy-terminus of the heavy and light chains respectively.

Light chains are classified as either kappa or lambda (κ, λ). Each heavy chain class may be bound with either a kappa or lambda light chain. In general, the light and heavy chains are covalently bonded to each other, and the "tail" portions of the two heavy chains are bonded to each other by covalent disulfide linkages when the immunoglobulins are generated either by hybridomas, B cells or genetically engineered host cells. In the heavy chain, the amino acid sequences run from an N-terminus at the forked ends of the Y configuration to the C-terminus at the bottom of each chain. At the N-terminus is a variable region and at the C-terminus is a constant region. Those skilled in the art will appreciate that heavy chains are classified as gamma, mu, alpha, delta, or epsilon, (γ, µ, α, δ, ε) with some subclasses among them. It is the nature of this chain that determines the "class" of the antibody as IgA, IgD, IgE IgG, or IgM. The immunoglobulin subclasses (isotypes) e.g. IgG₁, IgG₂, IgG₃, IgG₄, IgA₁, etc. are well characterized and are known to confer functional specialization. Modified versions of each of these classes and isotypes are readily discernable to the skilled artisan in view of the instant disclosure and, accordingly, are within the purview of the instant invention.

As indicated above, the variable region allows the antibody to selectively recognize and specifically bind epitopes on immunoreactive antigens. That is, the V_{L} domain and V_{H} domain of an antibody combine to form the variable region that defines a three dimensional antigen binding site. This quaternary antibody structure provides for an antigen binding site present at the end of each arm of the Y. More specifically, the antigen binding site is defined by three complementary determining regions (CDRs) on each of the V_{H} and V_{L} chains.

The six CDRs present on each monomeric antibody (H₂L₂) are short, non-contiguous sequences of amino acids that are specifically positioned to form the antigen binding site as the antibody assumes its three dimensional configuration in an aqueous environment. The remainder of the heavy and light variable domains show less inter-molecular variability in amino acid sequence and are termed the framework regions. The framework regions largely adopt a β-sheet conformation and the CDRs form loops connecting, and in some cases forming part of, the β-sheet structure. Thus, these framework regions act to form a scaffold that provides for positioning the six CDRs in correct orientation by inter-chain, non-covalent interactions. In any event, the antigen binding site formed by the positioned CDRs defines a surface complementary to the epitope on the immunoreactive antigen. This complementary surface promotes the non-covalent binding of the antibody to the immunoreactive antigen epitope.

For the purposes of the present invention, it should be appreciated that antibodies expressed using the subject polycistronic expression system may comprise any type of variable region that provides for the association of the antibody with the selected antigen. In this regard, the variable region may comprise or be derived from any type of mammal that can be induced to mount a humoral response and generate immunoglobulins against the desired antigen. As such, the variable region of the modified antibodies may be, for example, of human, murine, non-human primate (e.g. cynomolgus monkeys, macaques, etc.) or lupine origin. In particularly preferred embodiments both the variable and constant regions of compatible modified antibodies are human. In other selected embodiments the variable regions of compatible antibodies (usually derived from a non-human source) may be engineered or specifically tailored to improve the binding properties or reduce the immunogenicity of the molecule. In this respect, variable regions useful in the present invention may be humanized or otherwise altered through the inclusion of imported DNA or amino acid sequences.

For the purposes of the instant application the term "humanized antibody" shall mean an antibody derived from a non-human antibody, typically a murine antibody, that retains or substantially retains the antigen-binding properties of the parent antibody, but which is less immunogenic in humans. This may be achieved by various methods, including (a) grafting the entire non-human variable domains onto human constant regions to generate chimeric antibodies; (b) grafting at least a part of one or more of the non-human complementarity determining regions (CDRs) into a human framework and constant regions with or without retention of critical framework residues; or (c) transplanting the entire non-human variable domains, but "cloaking" them with a human-like section by replacement of surface residues. Such methods are disclosed in Morrison et al., Proc. Natl. Acad. Sci. 81: 6851-5 (1984); Morrison et al., Adv. Immunol. 44: 65-92 (1988); Verhoeyen et al., Science 239: 1534-1536 (1988); Padlan, Molec. Immun. 28: 489-498 (1991); Padlan, Molec. Immun. 31: 169-217 (1994), and U.S. Pat. Nos. 5,585,089, 5,693,761 and 5,693,762 all of which are hereby incorporated by reference in their entirety.

Those skilled in the art will appreciate that the technique set forth in option (a) above will produce "classic" chimeric antibodies. In the context of the present application the term "chimeric antibodies" will be held to mean any antibody wherein the immunoreactive region or site is obtained or derived from a first species and the constant region (which may be intact, partial or modified in accordance with the instant invention) is obtained from a second species. In preferred embodiments the antigen binding region or site will be from a non-human source (e.g. mouse) and the constant region is human. While the immunogenic specificity of the variable region is not generally affected by its source, a human constant region is less likely to elicit an immune response from a human subject than would the constant region from a non-human source. Certain chimeric antibodies may be generated by first immunizing monkeys with a desired antigen, isolating antibodies raised to the antigen and substituting the constant region of the heavy and light chains with a constant region (e.g., human) having desired function (e.g., human effector function, or the like). Such "Primatized®" antibodies and methods of making same are further described in U.S. Patent No. 5,658,570, incorporated herein by this reference in its entirety.

Preferably, variable domains in both the heavy and light chains of chimeric antibodies are altered by at least partial replacement of one or more CDRs and, if necessary, by partial framework region replacement and sequence changing. Although the CDRs may be derived from an antibody of the same class or even subclass as the antibody from which the framework regions are derived, it is envisaged that the CDRs will be derived from an antibody of different class and preferably from an antibody from a different species. It must be emphasized that it may not be necessary to replace all of the CDRs with the complete CDRs from the donor variable region to transfer the antigen binding capacity of one variable domain to another. Rather, it may only be necessary to transfer those residues that are necessary to maintain the activity of the antigen binding site. Given the explanations set forth in U. S. Pat. Nos. 5,585,089, 5,693,761 and 5,693,762, it will be well within the competence of those skilled in the art, either by carrying out routine experimentation or by trial and error testing to obtain a functional antibody with reduced immunogenicity.

Alterations to the variable region notwithstanding, those skilled in the art will appreciate that modified antibodies compatible with the instant invention will comprise antibodies, or immunoreactive fragments thereof, in which at least a fraction of one or more of the constant region domains has been deleted or otherwise altered so as to provide desired biochemical characteristics such as increased tumor localization or modified (e.g., reduced) serum half-life when compared with an antibody of approximately the same immunogenicity comprising a native or unaltered constant region. In preferred embodiments, the constant region of the antibodies expressed using the subject polycistronic vectors will comprise a human constant region. Modifications to the constant region compatible with the instant invention comprise additions, deletions or substitutions of one or more amino acids in one or more domains. That is, the modified antibodies disclosed herein may comprise alterations or modfications to one or more of the three heavy chain constant domains (C_{H}1, C_{H}2 or C_{H}3) and/or to the light chain constant domain (C_{L}). As will be discussed in more detail below and shown in the examples, certain embodiments of the invention comprise expression of antibodies having modified constant regions wherein one or more domains are partially or entirely deleted ("domain deleted antibodies"). In especially preferred embodiments compatible modified antibodies will comprise domain deleted constructs or variants wherein the entire C_{H}2 domain has been removed (ΔC_{H}2 constructs). For other preferred embodiments a short amino acid spacer may be substituted for the deleted domain to provide flexibility and freedom of movement for the variable region.

As previously indicated, the subunit structures and three dimensional configuration of the constant regions of the various immunoglobulin classes are well known. For example, the C_{H}2 domain of a human IgG Fc region usually extends from about residue 231 to residue 340 using conventional numbering schemes. The C_{H}2 domain is unique in that it is not closely paired with another domain. Rather, two N-linked branched carbohydrate chains are interposed between the two C_{H}2 domains of an intact native IgG molecule. It is also well documented that the C_{H}3 domain extends from the C_{H}2 domain to the C-terminal of the IgG molecule and comprises approximately 108 residues while the hinge region of an IgG molecule joins the C_{H}2 domain with the C_{H}1 domain. This hinge region encompasses on the order of 25 residues and is flexible, thereby allowing the two N-terminal antigen binding regions to move independently.

Besides their configuration, it is known in the art that the constant region mediates several effector functions. For example, binding of the C1 component of complement to antibodies activates the complement system. Activation of complement is important in the opsonisation and lysis of cell pathogens. The activation of complement also stimulates the inflammatory response and may also be involved in autoimmune hypersensitivity. Further, antibodies bind to cells via the Fc region, with a Fc receptor site on the antibody Fc region binding to a Fc receptor (FcR) on a cell. There are a number of Fc receptors which are specific for different classes of antibody, including IgG (gamma receptors), IgE (eta receptors), IgA (alpha receptors) and IgM (mu receptors). Binding of antibody to Fc receptors on cell surfaces triggers a number of important and diverse biological responses including engulfment and destruction of antibody-coated particles, clearance of immune complexes, lysis of antibody-coated target cells by killer cells (called antibody-dependent cell-mediated cytotoxicity, or ADCC), release of inflammatory mediators, placental transfer and control of immunoglobulin production. Although various Fc receptors and receptor sites have been studied to a certain extent, there is still much which is unknown about their location, structure and functioning.

As discussed above, modification of the constant region by some of the methods described herein allows the disclosed modified antibodies to spontaneously assemble or associate into stable antibodies. Moreover, while not limiting the scope of the present invention, it is believed that antibodies comprising constant regions modified as described herein provide for altered effector functions that, in turn, affect the biological profile of the administered antibody. For example, the deletion or inactivation (through point mutations or other means) of a constant region domain may reduce Fc receptor binding of the circulating modified antibody thereby increasing tumor localization. In other cases it may be that constant region modifications consistent with the instant invention moderate compliment binding and thus reduce the serum half life and nonspecific association of a conjugated cytotoxin. Yet other modifications of the constant region may be used to eliminate disulfide linkages or oligosaccharide moities that allow for enhanced localization due to increased antigen specificity or antibody flexibility. More generally, those skilled in the art will realize that antibodies modified as described herein may exert a number of subtle effects that may or may not be readily appreciated. However the resulting physiological profile, bioavailability and other biochemical effects of the modifications, such as tumor localization and serum half-life, may easily be measured and quantified using well known immunological techniques without undue experimentation.

Similarly, modifications to the constant region in accordance with the instant invention may easily be made using well known biochemical or molecular engineering techniques well within the purview of the skilled artisan. In this respect the examples appended hereto provide various constructs having constant regions modified in accordance with the present invention. More specifically, the exemplified constructs comprise chimeric and humanized antibodies having human constant regions that have been engineered to delete the C_{H}2 domain. Those skilled in the art will appreciate that such constructs are particularly preferred due to the regulatory properties of the C_{H}2 domain on the catabolic rate of the antibody.

ΔC_{H}2 domain deleted antibodies set forth in PCT/US02/02373 and PCT/US02/02374, both filed on January 29, 2002 and incorporated by reference in its entirety herein, are derived from the chimeric C2B8 antibody which is immunospecific for the CD20 pan B cell antigen and a humanized CC49 antibody which is specific for the TAG 72 antigen. As discussed in more detail below, both domain deleted constructs were derived from a proprietary vector (IDEC Pharmaceuticals, San Diego) encoding an IgG₁ human constant domain. Essentially, the vector was engineered to delete the C_{H}2 domain and provide a modified vector expressing a domain deleted IgG₁ constant region. Genes encoding the murine variable region of the C2B8 antibody or the variable region of the humanized CC49 antibody were then inserted in the modified vector and cloned. When expressed in transformed cells, these vectors provided huCC49.ΔC_{H}2 or C2B8.ΔC_{H}2 respectively. As illustrated below, these ' constructs exhibited a number of properties that make them particularly attractive candidates for monomeric subunits.

It will be noted that the foregoing exemplary constructs were engineered to fuse the C_{H}3 domain directly to the hinge region of the respective modified antibodies. In other constructs it may be desirable to provide a peptide spacer between the hinge region and the modified C_{H}2 and/or C_{H}3 domains. For example, compatible constructs could be expressed wherein the C_{H}2 domain has been deleted and the remaining C_{H}3 domain (modified or unmodified) is joined to the hinge region with a 5 - 20 amino acid spacer. Such a spacer may be added, for instance, to ensure that the regulatory elements of the constant domain remain free and accessible or that the hinge region remains flexible. However, it should be noted that amino acid spacers may, in some cases, prove to be immunogenic or inhibit the desired dimerization of the monomeric subunits. For example, a domain deleted CC49 construct having a short amino acid spacer GGSSGGGGSG (Seq. ID No. 1) substituted for the C_{H}2 domain (CC49.ΔC_{H}2 [gly/ser]) is used as a control in the examples because it does not assemble spontaneously into a dimeric form. Accordingly, any spacer compatible with the instant invention will be relatively non-immunogenic and not inhibit the non-covalent association of the modified antibodies.

Besides the deletion of whole constant region domains, it will be appreciated that polycistronic antibody constructs of the present invention may be provided by the partial deletion or substitution of a few or even a single amino acid as long as it permits the desired non-covalent association between the monomeric subunits. For example, the mutation of a single amino acid in selected areas of the C_{H}2 domain may be enough to substantially reduce Fc binding and thereby increase tumor localization. Similarly, it may be desirable to simply delete that part of one or more constant region domains that control the effector function (e.g. complement CLQ binding) to be modulated. Such partial deletions of the constant regions may improve selected characteristics of the antibody (serum half-life) while leaving other desirable functions associated with the subject constant region domain intact. Moreover, as alluded to above, the constant regions of the disclosed antibodies may be modified through the mutation or substitution of one or more amino acids that enhances the profile of the resulting construct. In this respect it may be possible to disrupt the activity provided by a conserved binding site (e.g. Fc binding) while substantially maintaining the configuration and immunogenic profile of the modified antibody. Yet other preferred embodiments may comprise the addition of one or more amino acids to the constant region to enhance desirable characteristics such as effector function or provide for more cytotoxin or carbohydrate attachment. In such embodiments it may be desirable to insert or replicate specific sequences derived from selected constant region domains.

Following manipulation of the isolated genetic material to provide antibodies and modified antibodies genes as set forth above, the genes are then inserted in a polycistronic expression vector according to the invention for introduction into host cells that may be used to produce the desired quantity of antibody. Constructive of such constructs is described in detail infra.

The term "vector" or "expression vector" is used herein for the purposes of the specification and claims, to mean vectors used in accordance with the present invention as a vehicle for introducing into and expressing a desired gene in a cell. As known to those skilled in the art, such vectors may easily be selected from the group consisting of plasmids, phages, viruses and retroviruses. In general, vectors compatible with the instant invention will comprise a selection marker, appropriate restriction sites to facilitate cloning of the desired gene and the ability to enter and/or replicate in eukaryotic or prokaryotic cells.

For the purposes of this invention, numerous polycistronic expression vector systems may be employed. For example, polycistronic vector may contain DNA elements which are derived from animal viruses such as bovine papillomavirus virus, polyoma virus, adenovirus, vaccinia virus, baculovirus, retroviruses (RSV, MMTV or MOMLV) or SV40 virus. Additionally, cells which have integrated the polycistronic construct DNA into their chromosomes may be selected by introducing one or more markers which allow selection of transfected host cells. The marker may provide for prototrophy to an auxotrophic host, biocide resistance (e.g., antibiotics) or resistance to heavy metals such as copper. The selectable marker gene can either be directly linked to the DNA sequences to be expressed, or introduced into the same cell by cotransformation. Additional elements may also be needed for optimal synthesis of mRNA. These elements may include splice signals, as well as transcriptional promoters, enhancers, and termination signals.

In preferred embodiments the instant invention will express antibodies, including modified antibodies, using the subject novel polycistronic expression systems. In these novel expression systems, multiple gene products of interest such as heavy and light chains of antibodies may be produced from a single polycistronic construct. These systems advantageously use an internal ribosome entry site (IRES) to provide relatively high levels of modified antibodies in eukaryotic host cells. Compatible IRES sequences are disclosed in U.S.P.N. 6,193,980 which is also incorporated herein in its entirety. Those skilled in the art will appreciate that such expression systems may be used to effectively produce the full range of modified antibodies disclosed in the instant application.

More generally, once the vector or DNA sequence encoding the monomeric subunit (e.g., modified antibody) has been prepared, the expression vector may be introduced into an appropriate host cell. That is, the host cells may be transformed. Introduction of the plasmid into the host cell can be accomplished by various techniques well known to those of skill in the art. These include, but are not limited to, transfection (including electrophoresis and electroporation), protoplast fusion, calcium phosphate precipitation, cell fusion with enveloped DNA, microinjection, and infection with intact virus. See, Ridgway, A. A. G. "Mammalian Expression Vectors" Chapter 24.2, pp. 470-472 Vectors, Rodriguez and Denhardt, Eds. (Butterworths, Boston, Mass. 1988). Most preferably, plasmid introduction into the host is via electroporation. The transformed cells are grown under conditions appropriate to the production of the light chains and heavy chains, and assayed for heavy and/or light chain protein synthesis. Exemplary assay techniques for identifying and quantifying gene products of interest include enzyme-linked immunosorbent assay (ELISA), radioimmunoassay (RIA), or fluorescence-activated cell sorter analysis (FACS), immunohistochemistry and the like.

As used herein, the term "transformation" shall be used in a broad sense to refer to any introduction of DNA into a recipient host cell that changes the genotype and consequently results in a change in the recipient cell.

As used herein, "host cells" refers to cells that have been transformed with vectors constructed using recombinant DNA techniques and encoding at least one heterologous gene. As defined herein, antibodies or modifications thereof produced by a host cell that is, by virtue of this transformation, recombinant. In descriptions of processes for isolation of antibodies from recombinant hosts, the terms "cell" and "cell culture" are used interchangeably to denote the source of antibody unless it is clearly specified otherwise. In other words, recovery of antibody from the "cells" may mean from spun down whole cells, or from the cell culture containing both the medium and the suspended cells.

The host cell line used for protein expression is most preferably of mammalian origin; those skilled in the art can readily determine particular host cell lines which are best suited for expression of the desired gene product. Exemplary host cell lines include, but are not limited to, DG44 and DUXB11 (Chinese Hamster Ovary lines, DHFR minus), HELA (human cervical carcinoma), CVI (monkey kidney line), COS (a derivative of CVI with SV40 T antigen), R1610 (Chinese hamster fibroblast) BALBC/3T3 (mouse fibroblast), HAK (hamster kidney line), SP2/O (mouse myeloma), P3.times.63-Ag3.653 (mouse myeloma), BFA-1c1BPT (bovine endothelial cells), RAJI (human lymphocyte) and 293 (human kidney). CHO cells are particularly preferred. Host cell lines are typically available from commercial services, the American Tissue Culture Collection or from published literature.

*In vitro* production allows scale-up to give large amounts of the desired polypeptide produced using the polycistronic expression system, preferably an antibody. Techniques for eukaryotic, e.g., mammalian and yeast cell cultivation under tissue culture conditions are known in the art and include homogeneous suspension culture, e.g. in an airlift reactor or in a continuous stirrer reactor, or immobilized or entrapped cell culture, e.g. in hollow fibers, microcapsules, on agarose microbeads or ceramic cartridges. For isolation and recovery of the antibodies, the immunoglobulins in the culture supernatants may first be concentrated, e.g. by precipitation with ammonium sulphate, dialysis against hygroscopic material such as PEG, filtration through selective membranes, or the like. If necessary and/or desired, the concentrated solutions of multivalent antibodies are purified by the customary chromatography methods, for example gel filtration, ion-exchange chromatography, chromatography over DEAE-cellulose or (immuno-)affinity chromatography.

Disclosed herein is a novel expression system for producing multiple gene products of interest from a single polycistronic construct. Unlike previously known polycistronic constructs, the inventive expression system produces sufficient levels of desired genes in both the first and subsequent cistrons to be commercially useful. This is surprising, as heretofore, gene sequences located after the first cistron in a polycistronic expression system have been expressed at very low levels in comparison with gene sequences expressed in the first cistron. Accordingly, previous polycistronic expression systems were generally limited to expressing a marker sequence in a second cistron, not a gene sequence of interest. Alternatively, the second cistronic gene was inefficiently expressed, thereby precluding the production of detectable translation products from genes contained in each cistron. By contrast, the polycistronic construct according to the invention may contain two, three or more cistrons, each encoding a gene of interest, if so desired.

The present invention, in its preferred embodiment, expresses antibodies, which may be modified as described herein, and further includes dimeric antibodies, using a polycistronic expression system, wherein two or more antibody genes are expressed off the same eukaryotic promoter, wherein such antibody genes are separated by one or more IRES's. Particularly, as discussed previously, the invention includes the expression of domain-deleted antibodies and other modified antibodies as described in PCT/US02/02373 and PCT/US02/02374, each filed on January 29, 2002, and incorporated by reference in its entirety herein. The eukaryotic cells used for expression will preferably be mammalian or yeast cells, most preferably CHO cells and other cells that can be efficiently cultured for high level protein production. As noted above, the obtaining or cloning of antibody heavy and light chain genes for incorporation into polycistronic expression systems according to the invention is well within the purview of ordinary skill. As noted, such antibody genes may encode mature heavy or light chain antibody genes, e.g., murine, rabbit, human, hamster, etc., or these heavy and/or light chain genes may be modified, e.g., by chimerization, humanization, domain deletion or site-specific mutagenesis.

The invention further contemplates the expression of any heavy chain and light chain sequence which when expressed using the polycistronic expression system of the invention, associate to produce a functional antibody, i.e., one that specifically binds to a target antigen, e.g., a tumor associated antigen.

As discussed, the copy number of the heavy and light chain genes in the polycistronic construct may be selected such that the preferred ratio of light/heavy chain are obtained, e.g., the light chain is expressed at levels which typically range from 10/1, more preferably 5/1, and still more preferably about 3/1 to 1/1 relative to the heavy chain.

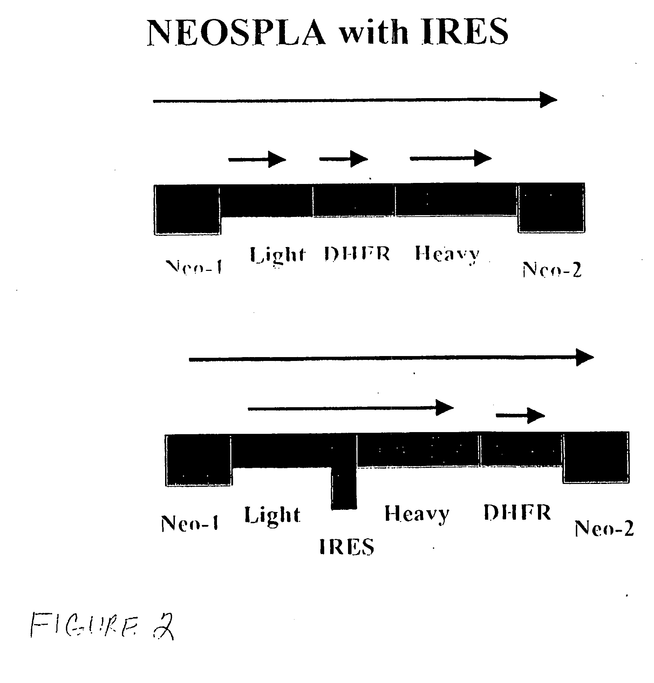

In the preferred embodiment, the expression system produces antibodies in eukaryotic cells, preferably mammalian cells such as Chinese hamster ovary (CHO) cells, baby hamster kidney (BHK) cells, fibroblast cell lines and myeloma cells. For example, CHO cells are employed as hosts for an expression system comprising a polycistron comprising, in the 5' to 3' orientation, at least the following sequences: a eukaryotic promoter sequence that is functional in the particular eukaryotic cell used for expression such as CMV, SV40 early or actin promoter sequences, preferably CMV; a DNA sequence encoding an antibody light chain and, preferably at its 5' end, a eukaryotic secretory leader sequence; an internal ribosome entry site (IRES), preferably that of a cardiovirus, poliovirus or herpes virus, positioned to follow the antibody light chain sequence; at least one DNA sequence encoding an antibody heavy chain, each heavy chain sequence preferably being preceded by a eukaryotic secreting leader sequence, and flanked by a 5' start and a 3' stop codon, wherein some or all DNA's encoding an antibody heavy chain are separated from subsequent heavy chain sequences by an IRES, and wherein the ultimate antibody heavy chain coding sequence comprises a poly A sequence at its 3' terminus. A suitable locale of the IRES site between heavy and light chain sequences is exemplified within the NEOSPLA vector of Figure 2.

As noted herein, the eukaryotic cell preferably comprises a mammalian cell and more preferably a CHO cell. In a preferred embodiment, the promoter is the CMV promoter, and the IRES is derived from a cardiovirus such as Encephalomyocarditis virus, Mengo virus, Mous-Elberfiell virus, MM virus, and Columbia SK virus, most preferably human encephalomyocarditis virus (hEMCV).

The inventive polycistron preferably comprises an antibody light chain encoding sequence and one or two antibody heavy chain coding sequences. However, polycistron constructs according to the invention may include 3 or 4 gene sequences or cistrons, for example, one light chain and two or more heavy chains, are contemplated. Additionally, the subject polycistron will preferably comprise a poly A sequence, preferably that of the bovine growth hormone (bGH) gene. The polycistron system optionally may be used in loci targeting by homologous recombination.

In this embodiment, the polycistronic construct will comprise sequences that facilitate homologus recombination at a targeted site, e.g., gene that is inactivated after recombination.

In a preferred embodiment the inventive polycistron comprises one or two copies of the heavy chain coding sequence dependent upon the stoichiometry of expression of the particular antibody heavy and light chain genes. In this regard, it is well known that in polycistronic expression systems, the second gene is typically expressed at lesser efficiency than the first gene. Accordingly, in one embodiment, the inventive polycistron, in which the first cistron encodes an antibody light chain, may encompass a second cistron encoding two or more heavy chain coding sequences, if deemed necessary, to facilitate sufficient expression of the heavy chain relative to the light chain. In general, it is preferred that the heavy chain be expressed at levels which are at least equivalent to levels observed with non-polycistronic co-expression of the heavy and light chains when expressed in the same eukaryotic cell using a non-polycistronic expression system.

It is permissible, and in fact desirable, that more of the antibody light chain is expressed relative to the antibody heavy chain, as this is analogous to what occurs in endogenous antibody producing cells. Disparate expression levels exist because the light chain is instrumental in directing the appropriate assembly of the antibody heavy and light chains, and excessive unpaired heavy chain is thought to induce cell toxicity. The light chain is also critical in directing folding of the assembled antibody heavy and light chains to produce a functional (antigen-binding) antibody in the endoplasmic reticulum. Preferably, the antibody light chain will be expressed from about 10/1 to 1/1 relative to the antibody heavy chain.

However, levels of the heavy chain must not be *de minimus,* and should be present in sufficient ratios with respect to light chains to enable the generation of functional, secretable antibodies in commercially acceptable levels. Thus, it is undesirable for the heavy chain expression to be too low relative to the light chain, as underexpression results in inadequate yields of functional antibodies. For purposes of industrial utility, inadequate yields of functional antibodies render an expression system commercially non-viable, and makes the recovery of complete antibody molecules from batch cultures difficult to achieve. Generally, functional antibody is recovered from cultured cells at an amount ranging from about 5-100 picograms per cell, per day, however greater levels of expression may be achieved. For example, cultured cells may secrete at least 1-5 picograms of functional antibody per cell each day, or at least 3-10 mg/L for at least 3-4 days.

With respect to the above, it is generally unpredictable whether a given polycistronic expression system will result in adequate levels of antibody production relative to other expression systems. This unpredictability arises because, in some instances, the second desired gene in the polycistronic complex may be expressed at very low levels relative to the first gene. Therefore, preferred embodiments of polycistronic vectors should provide a ratio of antibody light chain expression to antibody heavy chain expression within the range of about 10:1 to about 1:1. More preferably, the ratio of light chain to heavy chain gene expression is from about 3:1 to about 1.5:1.

Initial IRES constructs were created to contain an antibody light chain sequence in the first cistron, followed by two IRES-antibody CH2 domain deleted heavy chain sequence pairings, thereby ensuring sufficient heavy chain protein production to enable suitable levels of antibody to be produced and secreted from host cells.

The inventive polycistronic vectors enable the requisite levels of heavy and light chain expression to be achieved by selection of appropriate heavy chain antibody sequences, by selection of an efficient IRES, such as that of hEMCV, or by the incorporation of multiple copies of the antibody heavy chain genes. Still further, the DNA corresponding to the 5' end of the heavy chain gene may be modified by site specific mutagenesis in a manner whereby the coding structure remains unaltered around the ATG codon, typically the first 10 codons, but which modification results in altered expression of the heavy chain coding sequence relative to an unmodified heavy chain gene.

The heavy chain yield using the subject polycistronic expression system will typically be less than the light chain yield, as is the typical expression relationship in an endogenous antibody producing cell. The light chain yield to heavy chain yield ratio will be sufficient to enable protein secretion and folding. The ratio of the light chain to heavy chain expression may be varied by, for example, increasing the number of IRES-linked downstream gene sequences following the light chain sequence of the first cistron. A particular IRES and expression cell combination may be selected to optimally increase the amount of second cistron expression in a system.

An antibody that is expressed according to the subject expression system may be specific to any desired antigen. Preferably, the antibody will be a functional antibody that elicits a therapeutic effect, such as an antibody useful for treating an autoimmune, inflammatory, infectious, allergic or neoplastic disease. The antibody may be combined with other therapeutic agents for synergistic effects. For example, the antibody may be combined with a radioactive source for use as a cancer chemotherapeutic agent.

In general, antibodies expressed according to the present invention may be used in any one of a number of conjugated (i.e. an immunoconjugate) or unconjugated forms. In particular, the antibodies of the present invention may be conjugated to cytotoxins such as radioisotopes, therapeutic agents, cytostatic agents, biological toxins or prodrugs. Alternatively, the antibodies of the instant invention may be used in a nonconjugated or naked form to harness a subject's natural defense mechanisms to eliminate malignant cells. In particularly preferred embodiments, the antibodies produced according to the expression system of the present invention may be modified, such as by conjugation to radioisotopes. Examples of radioisotopes useful according to the invention include ⁹⁰Y, ¹²⁵I, ¹³¹I, ¹²³I, ¹¹¹In, ¹⁰⁵Rh, ¹⁵³Sm, ⁶⁷Cu, ⁶⁷Ga, ¹⁶⁶Ho, ¹⁷⁷Lu, ¹⁸⁶Re and ¹⁸⁸Re, using anyone of a number of well known chelators or direct labeling. Conjugated and unconjugated antibodies may be used together in the same therapeutic regimen, e.g., as used in the currently approved therapeutic regimen employing Zevalin for the treatment of certain non-Hodgkin's lymphomas.

In other embodiments, the antibodies of the invention may be included in compositions that comprise modified antibodies coupled to drugs, prodrugs or biological response modifiers such as methotrexate, adriamycine, and lymphokines such as interferon. Still other embodiments of the present invention comprise the use of modified antibodies conjugated to specific biotoxins such as ricin or diptheria toxin. In yet other embodiments the modified antibodies may be complexed with other immunologically active ligands (e.g. antibodies or fragments thereof) wherein the resulting molecule binds to both a neoplastic cell and an effector cell such as a T cell. The selection of which conjugated and/or unconjugated modified antibody to use will depend of the type and stage of cancer, use of adjunct treatment (e.g., chemotherapy or external radiation) and patient condition. It will be appreciated that one skilled in the art could readily make such a selection in view of the teachings herein.

As used herein, "a cytotoxin or cytotoxic agent" means any agent that is detrimental to the growth and/or proliferation of cells and which may act to reduce, inhibit or destroy a cell or malignancy when exposed thereto. Exemplary cytotoxins include, but are not limited to, radionuclides, biotoxins, enzymatically active toxins, cytostatic or cytotoxic therapeutic agents, prodrugs, immunologically active ligands and biological response modifiers such as cytokines. As will be discussed in more detail below, radionuclide cytotoxins are particularly preferred for use in the instant invention. However, any cytotoxin that acts to retard or slow the growth of immunoreactive cells or malignant cells or to eliminate these cells and which may be associated with the polycistronic derived functional antibodies disclosed herein is within the scope of the present invention.

It will be appreciated that, in previous studies, anti-tumor antibodies labeled with the above-noted isotopes have been used successfully to destroy cells in solid tumors as well as lymphomas/leukemias in animal models, and in humans. The radionuclides act by producing ionizing radiation which causes multiple strand breaks in nuclear DNA, leading to cell death. The isotopes used to produce therapeutic conjugates typically produce high energy α- or β-particles which have a short path length. Such radionuclides kill cells to which they are in close proximity, for example neoplastic cells to which the conjugate has attached or has entered. They have little or no effect on non-localized cells. Radionuclides are essentially non-immunogenic.

With respect to the use of radiolabeled conjugates in conjunction with the present invention, the modified antibodies may be directly labeled (such as through iodination) or may be labeled indirectly through the use of a chelating agent. As used herein, the phrases "indirect labeling" and "indirect labeling approach" both mean that a chelating agent is covalently attached to an antibody and at least one radionuclide is associated with the chelating agent. Such chelating agents are typically referred to as bifunctional chelating agents as they bind both the polypeptide and the radioisotope. Particularly preferred chelating agents include 1-isothiocyanatobenzyl-3-methyldiothelene triaminepentaacetic acid ("MX-DTPA") and cyclohexyl diethylenetriamine pentaacetic acid ("CHX-DTPA") derivatives. Other chelating agents comprise P-DOTA and EDTA derivatives. Particularly suitable radionuclides for indirect labeling include ¹¹¹In and ⁹⁰Y.

As used herein, the phrases "direct labeling" and "direct labeling approach" both mean that a radionuclide is covalently attached directly to a antibody (typically via an amino acid residue). More specifically, these linking technologies include random labeling and site-directed labeling. In the latter case, the labeling is directed at specific sites on the antibody, such as the N-linked sugar residues present only on the Fc portion of the conjugates. Direct labeling may result in multi-meric antibodies linked by the labels. Various direct labeling techniques and protocols are compatible with the instant invention. For example, Technetium-99m labelled tetravalent antibodies may be prepared by ligand exchange processes, by reducing pertechnate (TcO₄⁻) with stannous ion solution, chelating the reduced technetium onto a Sephadex column and applying the antibodies to this column, or by batch labeling techniques, e.g. by incubating pertechnate, a reducing agent such as SnCl₂, a buffer solution such as a sodium-potassium phthalate-solution, and the antibodies. In any event, preferred radionuclides for directly labeling antibodies are well known in the art and a particularly preferred radionuclide for direct labeling is ¹³¹I which can be covalently attached via tyrosine residues. Modified antibodies according to the invention may be derived, for example, with radioactive sodium or potassium iodide and a chemical oxidizing agent, such as sodium hypochlorite, chloramine T or the like, or an enzymatic oxidizing agent, such as lactoperoxidase, glucose oxidase and glucose. However, for the purposes of the present invention, the indirect labeling approach is generally favored.

Patents relating to chelators and chelator conjugates are known in the art. For instance, U.S. Patent No. 4,831,175 of Gansow is directed to polysubstituted diethylenetriaminepentaacetic acid chelates and protein conjugates containing the same, and methods for their preparation. U.S. Patent Nos. 5,099,069, 5,246,692, 5,286,850, 5,434,287 and 5,124,471 of Gansow also relate to polysubstituted DTPA chelates. These patents are incorporated herein in their entirety. Other examples of compatible metal chelators are ethylenediaminetetraacetic acid (EDTA), diethylenetriaminepentaacetic acid (DPTA), 1,4,8,11-tetraazatetradecane, 1,4,8,11-tetraazatetradecane-1,4,8,11-tetraacetic acid, 1-oxa-4,7,12,15-tetraazaheptadecane-4,7,12,15-tetraacetic acid, or the like. Cyclohexyl-DTPA or CHX-DTPA is particularly preferred and is exemplified extensively below. Still other compatible chelators, including those yet to be discovered, may easily be discerned by a skilled artisan and are clearly within the scope of the present invention.

Compatible chelators, including the specific bifunctional chelator used to facilitate chelation in co-pending application Serial Nos. 08/475,813, 08/475,815 and 08/478,967, are preferably selected to provide high affinity for trivalent metals, exhibit increased tumor-to-non-tumor ratios and decreased bone uptake as well as greater *in vivo* retention of radionuclide at target sites, i.e., B-cell lymphoma tumor sites. However, other bifunctional chelators that may or may not possess all of these characteristics are known in the art and may also be beneficial in tumor therapy.

It will also be appreciated that, in accordance with the teachings herein, modified antibodies may be conjugated to different radiolabels for diagnostic and therapeutic purposes. Radiolabeled therapeutic conjugates for diagnostic "imaging" of tumors before administration of therapeutic antibody may be prepared. "ln2B8" conjugate comprises a murine monoclonal antibody, 2B8, (rituximab) specific to human CD20 antigen, that is attached to ¹¹¹In via a bifunctional chelator, i.e., MX-DTPA, which comprises a 1:1 mixture of 1-isothiocyanatobenzyl-3-methyl-DTPA and 1-methyl-3-isothiocyanatobenzyl-DTPA. ¹¹¹In is particularly preferred as a diagnostic radionuclide because between about 1 to about 10 mCi can be safely administered without detectable toxicity; and the imaging data is generally predictive of subsequent ⁹⁰Y-labeled antibody distribution. Most imaging studies utilize about 5 mCi ¹¹¹In-labeled antibody, because this dose is both safe and has increased imaging efficiency compared with lower doses, with optimal imaging occurring at three to six days after antibody administration. See, for example, Murray, J. Nuc. Med. 26: 3328 (1985) and Carraguillo et al., J. Nuc. Med. 26: 67 (1985).

As indicated above, a variety of radionuclides are applicable to the present invention and those skilled in the art are credited with the ability to readily determine which radionuclide is most appropriate under various circumstances. For example, ¹³¹I is a well known radionuclide used for targeted immunotherapy. However, the clinical usefulness of ¹³¹I can be limited by several factors including: eight-day physical half-life; dehalogenation of iodinated antibody both in the blood and at tumor sites; and emission characteristics (e.g., large gamma component) which can be suboptimal for localized dose deposition in a tumor. With the advent of superior chelating agents, the opportunity for attaching metal chelating groups to proteins has increased the opportunities to utilize other radionuclides such as ¹¹¹In and ⁹⁰Y. ⁹⁰Y provides several benefits for utilization in radioimmunotherapeutic applications: the 64 hour half-life of ⁹⁰Y is long enough to allow antibody accumulation by tumor and, unlike e.g., ¹³¹I, ⁹⁰Y is a pure beta emitter of high energy with no accompanying gamma irradiation in its decay, with a range in tissue of 100 to 1,000 cell diameters. Furthermore, the minimal amount of penetrating radiation allows for outpatient administration of ⁹⁰Y-labeled antibodies. Additionally, internalization of labeled antibody is not required for cell killing, and the local emission of ionizing radiation should be lethal for adjacent tumor cells lacking the target antigen.

Effective single treatment dosages (i.e., therapeutically effective amounts) of ⁹⁰Y-labeled modified antibodies range from between about 5 and about 75 mCi, more preferably between about 10 and about 40 mCi. Effective single treatment non-marrow ablative dosages of ¹³¹I-labeled antibodies range from between about 5 and about 70 mCi, more preferably between about 5 and about 40 mCi. Effective single treatment ablative dosages (i.e., may require autologous bone marrow transplantation) of ¹³¹I-labeled antibodies range from between about 30 and about 600 mCi, more preferably between about 50 and less than about 500 mCi. In conjunction with a chimeric antibody, owing to the longer circulating half life vis-à-vis murine antibodies, an effective single treatment non-marrow ablative dosages of iodine-131 labeled chimeric antibodies range from between about 5 and about 40 mCi, more preferably less than about 30 mCi.

While a great deal of clinical experience has been gained with ¹³¹I and ⁹⁰Y, other radiolabels are known in the art and have been used for similar therapeutic purposes. Still other radioisotopes are used for imaging. For example, additional radioisotopes which are compatible with the scope of the instant invention include, but are not limited to, ¹²³I, ¹²⁵I, ³²P, ⁵⁷Co, ⁶⁴Cu, ⁶⁷Cu, ⁷⁷Br, ⁸¹Rb, ⁸¹Kr, ⁸⁷Sr, ¹¹³In, ¹²⁷Cs, ¹²⁹Cs, ¹³²I, ¹⁹⁷Hg, ²⁰³Pb, ²⁰⁶Bi, ¹⁷⁷Lu, ¹⁸⁶Re, ²¹²Pb, ²¹²Bi, ⁴⁷Sc, ¹⁰⁵Rh, ¹⁰⁹Pd, ¹⁵³Sm, ¹⁸⁸Re, ¹⁹⁹Au, ²²⁵Ac, ²¹¹At, and ²¹³Bi. In this respect alpha, gamma and beta emitters are all compatible with the instant invention. Further, in view of this disclosure it is submitted that one skilled in the art could readily determine which radionuclides are compatible with a selected course of treatment without undue experimentation. To this end, additional radionuclides which have already been used in clinical diagnosis include ¹²⁵I, ¹²³I, ⁹⁹Tc, ⁴³K, ⁵²Fe, ⁶⁷Ga, ⁶⁸Ga, as well as ¹¹¹In. Antibodies have also been labeled with a variety of radionuclides for potential use in targeted immunotherapy. Peirersz et al. Immunol. Cell Biol. 65: 111-125 (1987). These radionuclides include ¹⁸⁸Re and ¹⁸⁶Re as well as ¹⁹⁹Au and ⁶⁷Cu to a lesser extent. U.S. Patent No. 5,460,785 provides additional data regarding such radioisotopes and is incorporated herein by reference.

In addition to radionuclides, the functional antibodies of the present invention may be conjugated to, or associated with, anyone of a number of biological response modifiers, pharmaceutical agents, toxins or immunologically active ligands. Those skilled in the art will appreciate that these non-radioactive conjugates may be assembled using a variety of techniques depending on the selected cytotoxin. For example, conjugates with biotin are prepared e.g. by reacting the dimeric antibodies with an activated ester of biotin such as the biotin N-hydroxysuccinimide ester. Similarly, conjugates with a fluorescent marker may be prepared in the presence of a coupling agent, e.g. those listed above, or by reaction with an isothiocyanate, preferably fluorescein-isothiocyanate. Conjugates of the tetravalent antibodies of the invention with cytostatic/cytotoxic substances and metal chelates are prepared in an analogous manner.

Preferred agents for use in the present invention are cytotoxic drugs, particularly those which are used for cancer therapy. Such drugs include, in general, cytostatic agents, alkylating agents, antimetabolites, anti-proliferative agents, tubulin binding agents, hormones and hormone antagonists, and the like. Exemplary cytostatics that are compatible with the present invention include alkylating substances, such as mechlorethamine, triethylenephosphoramide, cyclophosphamide, ifosfamide, chlorambucil, busulfan, melphalan or triaziquone, also nitrosourea compounds, such as carmustine, lomustine, or semustine. Other preferred classes of cytotoxic agents include, for example, the anthracycline family of drugs, the vinca alkaloid family of drugs, the mitomycins, the bleomycins, the cytotoxic nucleosides, the pteridine family of drugs, diynenes, and the podophyllotoxins. Particularly useful members of those classes include, for example, adriamycine, carminomycin, daunorubicin (daunomycin), doxorubicin, aminopterin, methotrexate, methopterin, mithramycin, streptonigrin, dichloromethotrexate, mitomycin C, actinomycin-D, porfiromycin, 5-fluorouracil, floxuridine, ftorafur, 6-mercaptopurine, cytarabine, cytosine arabinoside, podophyllotoxin, or podophyllotoxin derivatives such as etoposide or etoposide phosphate, melphalan, vinblastine, vincristine, leurosidine, vindesine, leurosine and the like. Still other cytotoxins that are compatible with the teachings herein include taxol, taxane, cytochalasin B, gramicidin D, ethidium bromide, emetine, tenoposide, colchicin, dihydroxy anthracin dione, mitoxantrone, procaine, tetracaine, lidocaine, propranolol, and puromycin and analogs or homologs thereof. Also included as suitable cytotoxins are maytansinoids. Hormones and hormone antagonists, such as corticosteroids, e.g. prednisone, progestins, e.g. hydroxyprogesterone or medroprogesterone, estrogens, e.g. diethylstilbestrol, antiestrogens, e.g. tamoxifen, androgens, e.g. testosterone, and aromatase inhibitors, e.g. aminogluthetimide are also compatible with the teachings herein. As noted previously, one skilled in the art may make chemical modifications to the desired compound in order to make reactions of that compound more convenient for purposes of preparing conjugates of the invention.

One example of particularly preferred cytotoxins comprise members or derivatives of the enediyne family of anti-tumor antibiotics, including calicheamicin, esperamicins or dynemicins. These toxins are extremely potent and act by cleaving nuclear DNA, leading to cell death. Unlike protein toxins which can be cleaved *in vivo* to give many inactive but immunogenic polypeptide fragments, toxins such as calicheamicin, esperamicins and other enediynes are small molecules which are essentially non-immunogenic. These non-peptide toxins are chemically-linked to the dimers or tetramers by techniques which have been previously used to label monoclonal antibodies and other molecules. These linking technologies include site-specific linkage via the N-linked sugar residues present only on the Fc portion of the constructs. Such site-directed linking methods have the advantage of reducing the possible effects of linkage on the binding properties of the constructs.

As previously alluded to, compatible cytotoxins may comprise a prodrug. As used herein, the term "prodrug" refers to a precursor or derivative form of a pharmaceutically active substance that is less cytotoxic to tumor cells compared to the parent drug and is capable of being enzymatically activated or converted into the more active parent form. Prodrugs compatible with the invention include, but are not limited to, phosphate-containing prodrugs, thiophosphate-containing prodrugs, sulfate containing prodrugs, peptide containing prodrugs, β-lactam-containing prodrugs, optionally substituted phenoxyacetamide-containing prodrugs or optionally substituted phenylacetamide-containing prodrugs, 5-fluorocytosine and other 5-fluorouridine prodrugs that can be converted to the more active cytotoxic free drug. Further examples of cytotoxic drugs that can be derivatized into a prodrug form for use in the present invention comprise those chemotherapeutic agents described above.

Among other cytotoxins, it will be appreciated that antibodies can also be associated with a biotoxin such as ricin subunit A, abrin, diptheria toxin, botulinum, cyanginosins, saxitoxin, shigatoxin, tetanus, tetrodotoxin, trichothecene, verrucologen or a toxic enzyme. Preferably, such constructs will be made using genetic engineering techniques that allow for direct expression of the antibody-toxin construct. Other biological response modifiers that may be associated with the modified antibodies of the present invention comprise cytokines such as lymphokines and interferons. In view of the instant disclosure it is submitted that one skilled in the art could readily form such constructs using conventional techniques.

Another class of compatible cytotoxins that may be used in conjunction with the disclosed antibodies are radiosensitizing drugs that may be effectively directed to tumor or immunoreactive cells. Such drugs enhance the sensitivity to ionizing radiation, thereby increasing the efficacy of radiotherapy. An antibody conjugate internalized by the tumor cell would deliver the radiosensitizer nearer the nucleus where radiosensitization would be maximal. The unbound radiosensitizer linked modified antibodies would be cleared quickly from the blood, localizing the remaining radiosensitization agent in the target tumor and providing minimal uptake in normal tissues. After rapid clearance from the blood, adjunct radiotherapy would be administered in one of three ways: 1) external beam radiation directed specifically to the tumor, 2) radioactivity directly implanted in the tumor or 3) systemic radioimmunotherapy with the same targeting antibody. A potentially attractive variation of this approach would be the attachment of a therapeutic radioisotope to the radiosensitized immunoconjugate, thereby providing the convenience of administering to the patient a single drug.

Whether or not the disclosed functional antibodies are used in a conjugated or unconjugated form, it will be appreciated that a major advantage of the present invention is the ability to use these antibody constructs in myelosuppressed patients, especially those who are undergoing, or have undergone, adjunct therapies such as radiotherapy or chemotherapy. That is, the beneficial delivery profile (i.e. relatively short serum dwell time, high binding affinity and enhanced localization) of the dimeric antibodies makes them particularly useful for treating patients that have reduced red marrow reserves and are sensitive to myelotoxicity. In this regard, the unique delivery profile of the functional antibodies make them very effective for the administration of radiolabeled conjugates to myelosuppressed cancer patients. As such, the modified antibodies are useful in a conjugated or unconjugated form in patients that have previously undergone adjunct therapies such as external beam radiation or chemotherapy.

The functional antibodies produced according to the invention may bind to a tumor specific or tumor-associated antigen, an antigen expressed on specific cell types such as T cells or B cells, a viral antigen, a bacterial antigen, or to a parasite. Suitable antigens include TAG-72, CD4, CD11, CD19, CD20, CD22, CD23, CD37, CD40, CD45, CD80, CD86 and CD154. In a preferred embodiment of the invention, the antibody will bind to an antigen expressed by a T cell or B cell, such as CD4, CD19, CD20, CD22, CD23, CD40, CD80, and CD154. In another preferred embodiment, the antibody is directed to a cancer antigen such as CEA, prostate specific antigen, HER-2 (erbB2), a tumor adhesive molecule, etc. The antibody of choice may be a human, humanized or chimeric antibody. In particular, the antibody may be a human, humanized or chimeric antibody specific to CD20 or TAG-72. CD20 is an antigen expressed by B cells that has been targeted for treatment of B cell disorders such as B cell lymphomas and leukemias. Rituxan® is a chimeric anti-CD20 antibody that has been approved by the FDA for treatment of non-Hodgkin's lymphoma.

Tag-72 is an antigen that is known to be overexpressed by numerous human cancers including digestive cancers (gastric, colorectal, pancreatic), and reproductive organ associated cancers (prostate, ovarian, breast) as well as other cancers including e.g., head and neck cancers and lymph node metastases. (See e.g., Galietta et al., Oncol. Rep. 9(1): 135-40 (2002); Meredith et al., Cancer Biother. Radiopharm 16(4): 303-15 (2001); Karan et al., Oncol. Rep. 8(5): 1123-26 (2001); Allende et al., Int. J. Biol. Marker 15(2): 1997-99 (2000) and Altimissi et al., HNO 38*10): 364-66 (1998), all incorporated by reference in their entirety). Anti-TAG-72 antibodies have been reported to possess therapeutic efficacy in treating such cancers.

The DNA sequences encoding the antibody light chain and heavy chain(s) may comprise an intact or modified variable region and constant region. The constant region is preferably human. The variable regions may be of primate origin or of rodent origin, and may be humanized. Primate variable regions may be of human origin. Rodent variable regions may be, for example, of rat or mouse (murine) origin. As noted, domain deleted constant regions are within the scope of the present invention.

Antigens that are characteristically over-expressed by specific cancer types are well known in the art. Preferred embodiments embrace the expression of antibodies that recognize CD22 or CD20. In the most preferred embodiment, the antibody will specifically bind CD22. Even more preferably, the antibody will comprise heavy and light chains of the antibody rituximab (RITUXAN^{®}, IDEC Pharmaceuticals, San Diego, CA, USA), as discussed in, for example, U.S. Patent Nos. 5,736,137; 5,776,456; and 5,843,439. Examples of suitable antibodies for use in the invention are disclosed in U.S. Patent No. 6,136,310 to Hanna et al*.* (CD4); U.S. Patent No. 6,011,138 to Reff (CD23); U.S. Patent No. 6,113,898 to Anderson (CD80); and U.S. Patent No. 6,001,358 to Black (CD54). The entire contents of these patents are hereby incorporated herein by reference.

Although the invention has been illustrated utilizing antibodies, the invention is suitable for expressing any multichain proteins that would not compromise the viability of their mammalian cell hosts. The ratio of the gene products of the first and successive cistrons may be varied by increasing the number of subunits following the first cistron. Thus, for example, two or more cistrons, each encoding the same desired gene product, may be incorporated into a multicistronic expression vector in order to enhance the absolute amount of peptide produced by the desired gene. However, each successive cistron following the first cistron may encode distinct products, if desired.

The invention will be further illustrated by the following non-limiting Examples.

### EXAMPLE 1

A polycistronic DNA expression vector was constructed according to the invention, expressing the heavy and light chains of humanized anti-TAG72 antibody, which is denoted as HuCC49.

### A. Expression Vector Construction

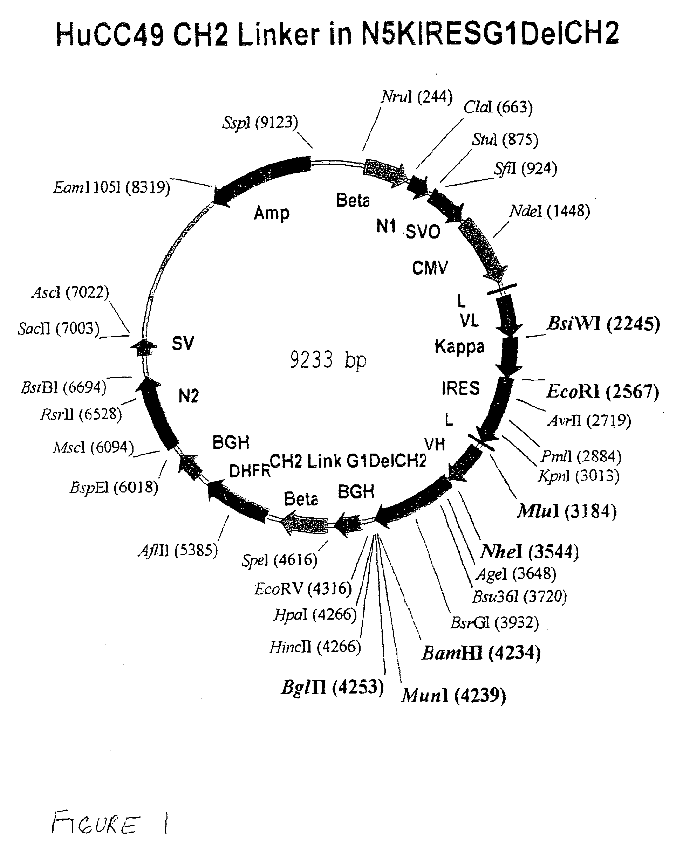

The expression construct, depicted in Figure 1, comprises the mouse beta-globulin major promoter (beta), situated within the construct so as to drive the expression of sequences encoding the neomycin phosphotransferase gene. The neomycin phosphotransferase gene, which is of bacterial origin, is composed of two exons, N1 and N2, and contains an artificial intron. Methods of intronic insertion of selectable markers designed to enhance gene product expression in expression vectors systems are disclosed in, for example, U.S. Patent No. 5,648,267, U.S. Patent No. 5,733,779, U.S. Patent No. 6,017,733, and U.S. Patent No. 6,159,730, all to Reff. The entire contents of each of these patents are hereby incorporated herein by reference. The antibody selected is known in the art for its suitability for treating solid tumors. A high level of tissue penetration is required in order to affect solid tumor cells, and therefore the antibody should have a relatively longer half-life within serum than is seen with Fab fragments. The domain deleted construct used in this example contains the human gamma 1 heavy chain constant domain in which most of the CH2 domain has been deleted, with the exception of the first nine amino acids of CH2 following the hinge region.

As shown in Figure 1, following the first exon of the neomycin phosphotransferase expression cassette (N1) and within the intron of the neomycin phosphotransferase gene is located a Simian virus SV40 origin of replication (SVO), which facilitates replication of the expression construct (vector) in COS cells following transient transfection. Thereafter is located an expression cassette for the gene sequences of interest, which in this case are immunoglobulin coding regions. First in the cassette is the human cytomegalovirus promoter (CMV), which drives expression of the polycistronic immunoglobulin (Ig) message. The Ig message in this construct encodes the humanized anti-TAG-72 antibody (denoted HuCC49), which is sequentially composed of (in the order presented): the light chain leader (L)/HuCC49 light chain variable (VL)/human kappa constant (Kappa)/IRES from the encephalomyocarditis virus (IRES)/synthetic heavy chain leader (L)/HuCC49 heavy chain variable (VH)/human gamma 1 CH2 domain deleted constant region (CH2 link G1 DelCH2). The Ig message is followed by the bovine growth hormone polyadenylation signal (BGH). Thereafter is situated an expression cassette for a dihydrofolate reductase coding region, in which the mouse beta-globin major promoter (Beta) drives expression of the murine dihydrofolate reductase gene (DHFR). The DHFR sequence is followed by a bovine growth hormone polyadenylation signal (BGH).

Following the neomycin intron is the second exon of the neomycin expression cassette (N2), which, in turn, is followed by the SV40 early polyadenylation signal (SV).

The vector also contains sequences required for replication in bacteria, including the colE1 origin of replication and the beta-lactamase gene to confer ampicillin resistance (Amp).

The EMCV is commercially available, and was obtained from ATCC (VR-129B Encephalomyocarditis strain: EMC (TC adapted)). The viral particles were disrupted and subjected to methods known in the art for isolating single stranded viral RNA. A specific cDNA was generated to the IRES region within the viral genome. PCR (polymerase chain reaction) amplification of that cDNA was performed in order to amplify the DNA, as well as to add 5' and 3' ends suitable for insertion between the light and heavy chain Ig coding domains. No polyadenylation signal was placed after the light chain immediately prior to the IRES. The ATG trinucleotide at position 834-836 (Genbank accession number NC-001479) was used as the start codon for the synthetic heavy chain leader sequence.

Because the literature indicates that translation of the open reading frame downstream of the IRES would be less efficient than the upstream open reading frame (translation initiated by 5' CAP), restriction sites were introduced such that the IRES and heavy chain sequences could be duplicated within the vector. Mun I and Bgl II sites were introduced downstream of the BamH I site. Following digestion with EcoR I and BamH I, the fragment containing the IRES and heavy chain gene sequence could be easily inserted into the Mun I and Bgl II sites. The resultant construct would then contain: CMV-light chain-IRES-heavy chain-IRES-heavy chain. The addition of the second heavy chain was made in order to compensate for any inefficiency associated with internal ribosome entry.

### B. CHO Cell Expression

A plasmid containing the expression vector expressing one heavy chain utilizing an SV40 enhancer was transfected into CHO cells using both a transient transfection protocol as well as a protocol designed to yield stable transfected cell lines having high expression capability. These transfections were performed in parallel with conventional expression constructs containing independent light and heavy chain cassettes (CMV-Light-BGH) (CMV-Heavy-BGH). Experimental results indicate that no apparent difference in expression levels arose, meaning no reduction in heavy chain translation as a result of inefficient IRES function was detected. Accordingly, an embodiment of the inventive polycistronic construct is expected to express other gene sequences of interest contained in first and subsequent cistrons in satisfactory amounts as well. Experiments have shown that these constructs express the downstream heavy chain gene in the bicistronic construct at levels equivalent to those obtained using conventional non-polycistronic expression systems, as discussed herein below.

### EXAMPLE 2

### A. Generation of G418 Resistant Cell Lines

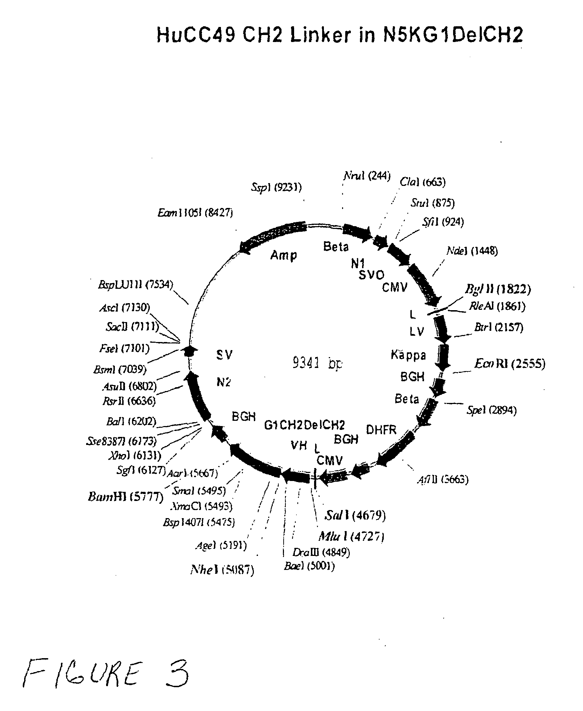

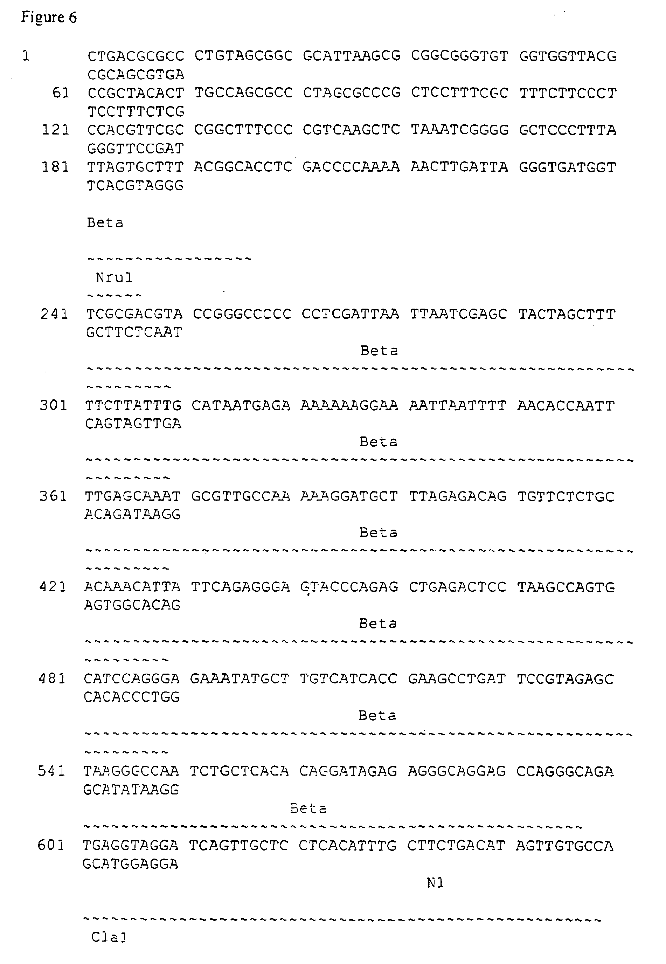

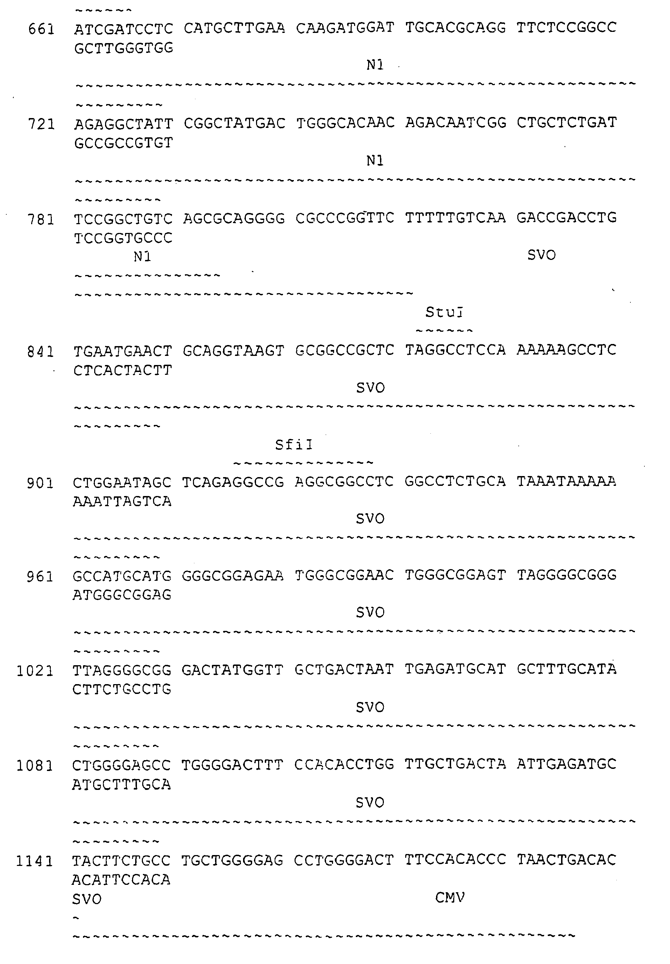

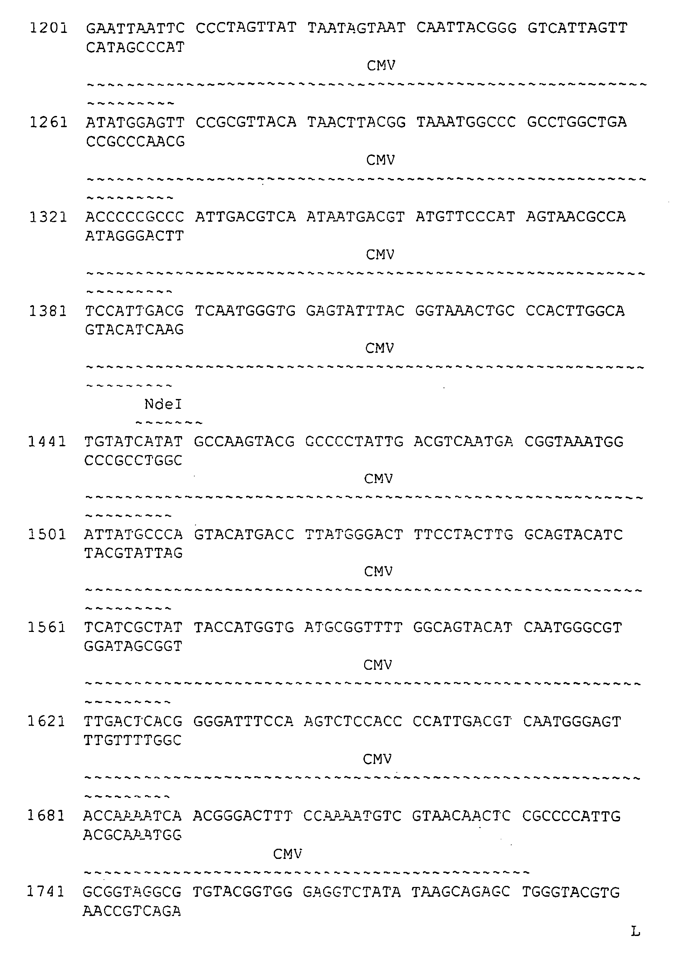

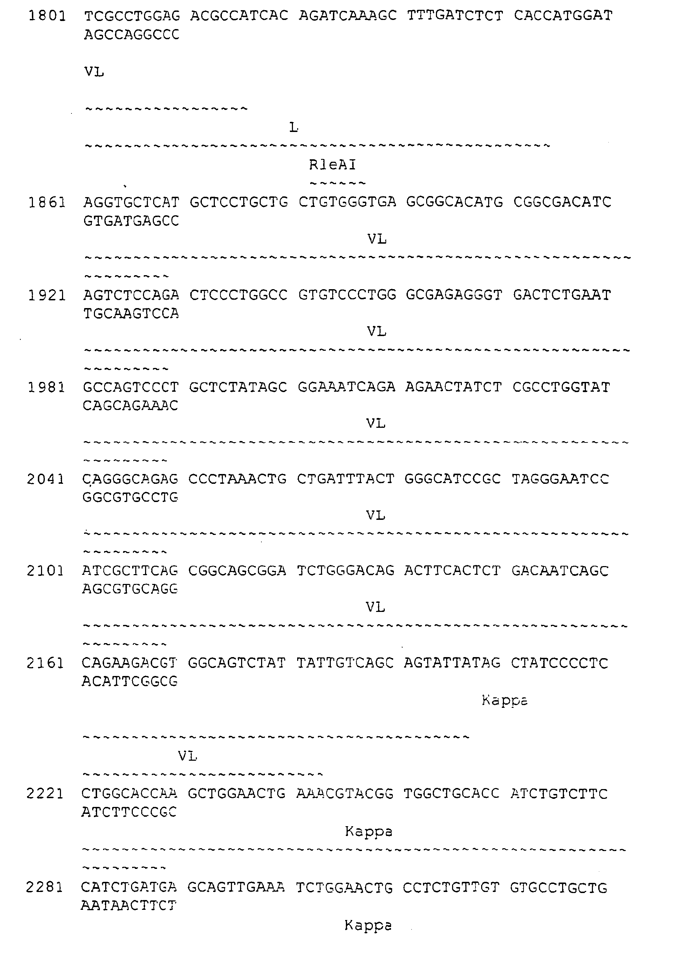

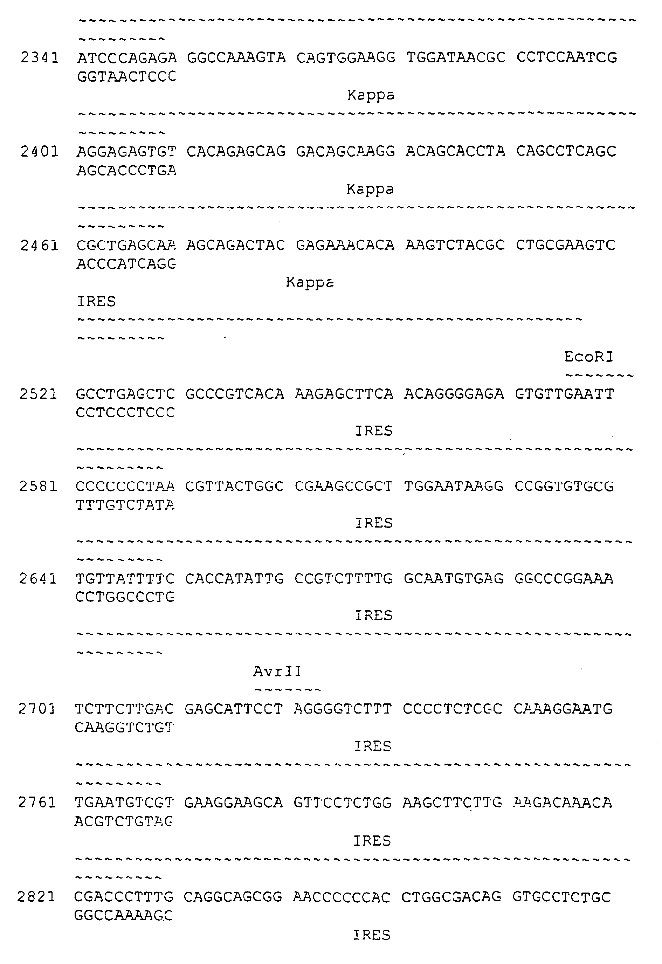

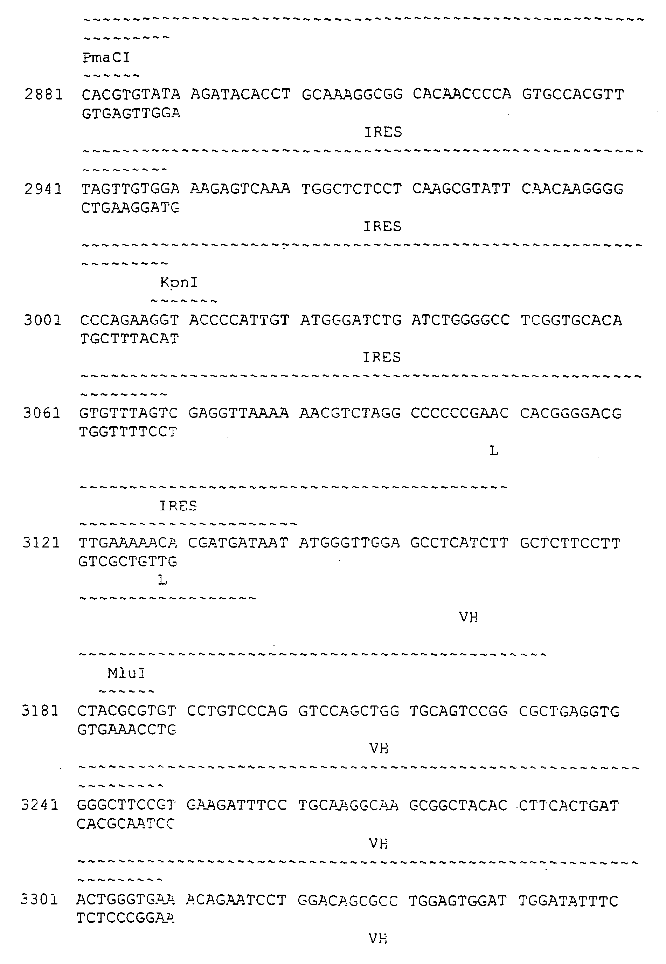

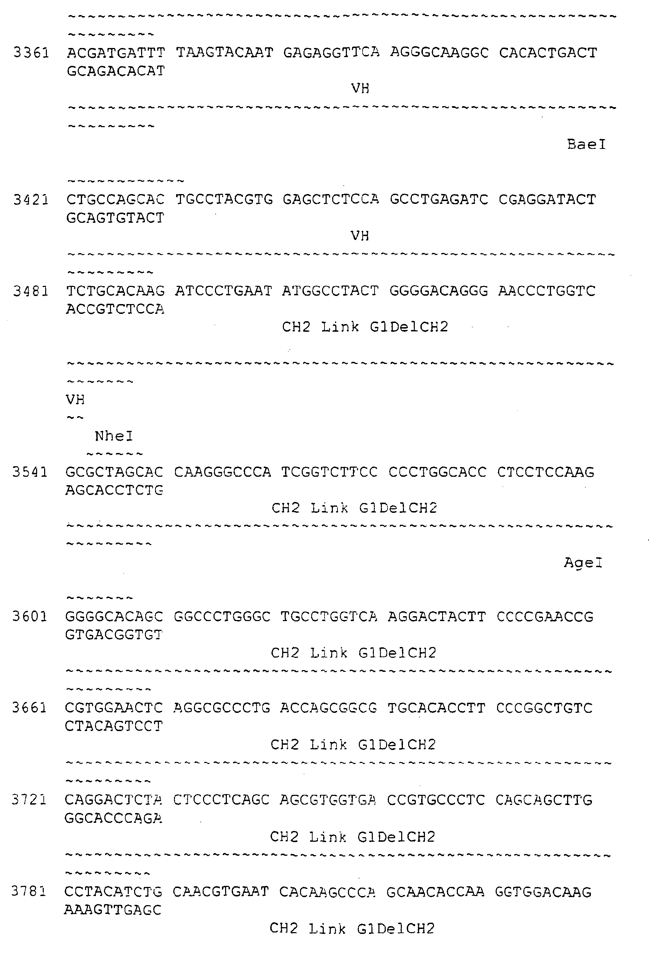

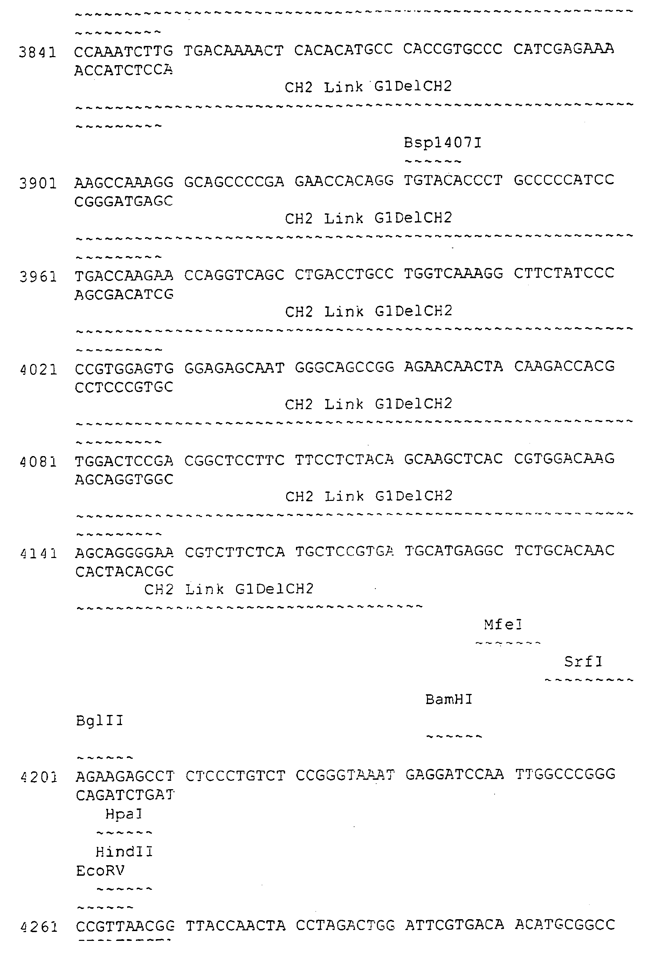

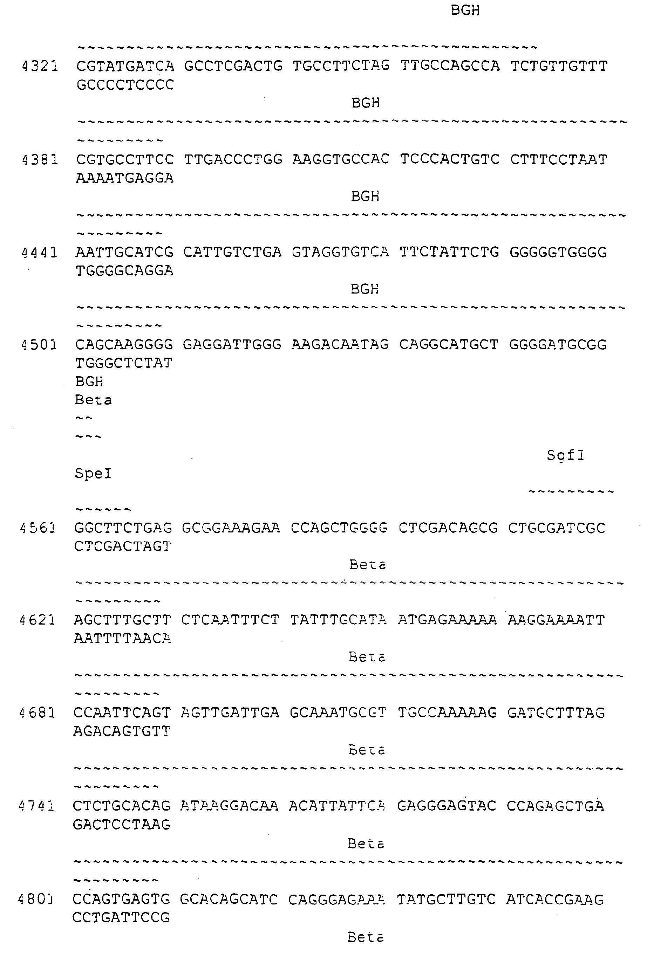

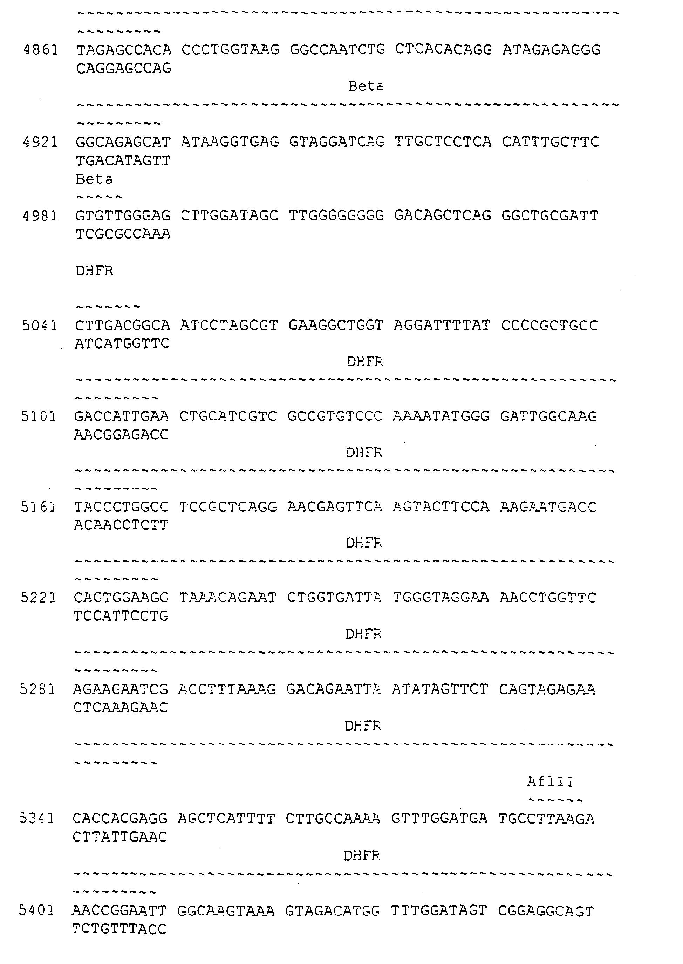

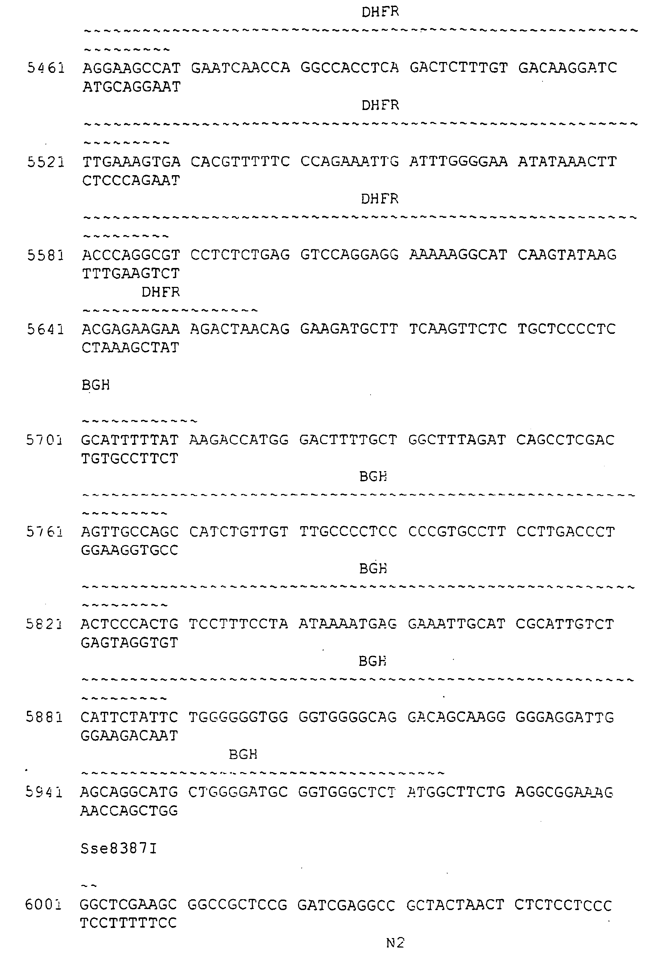

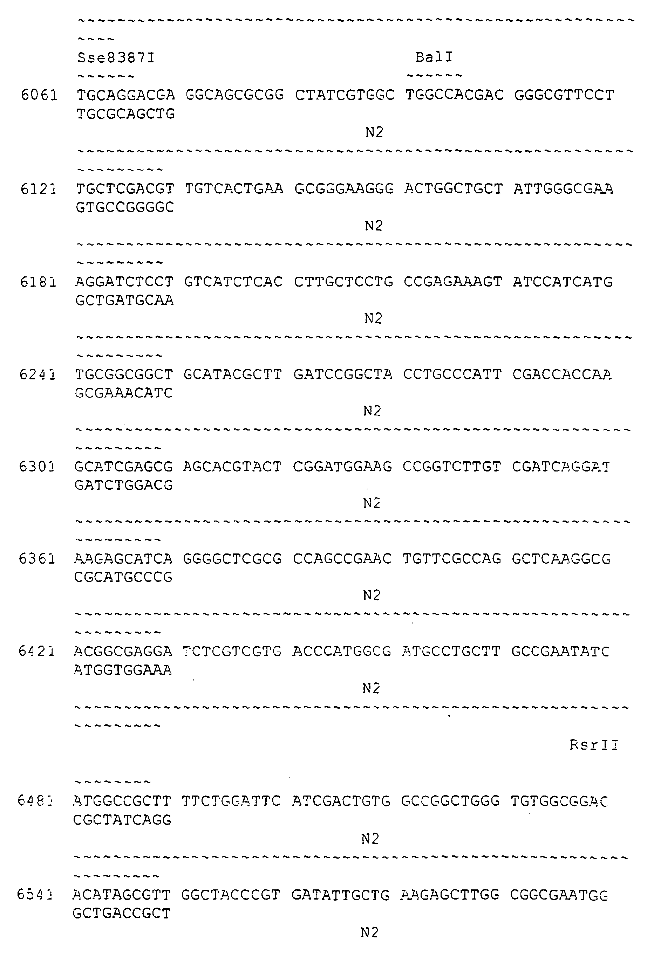

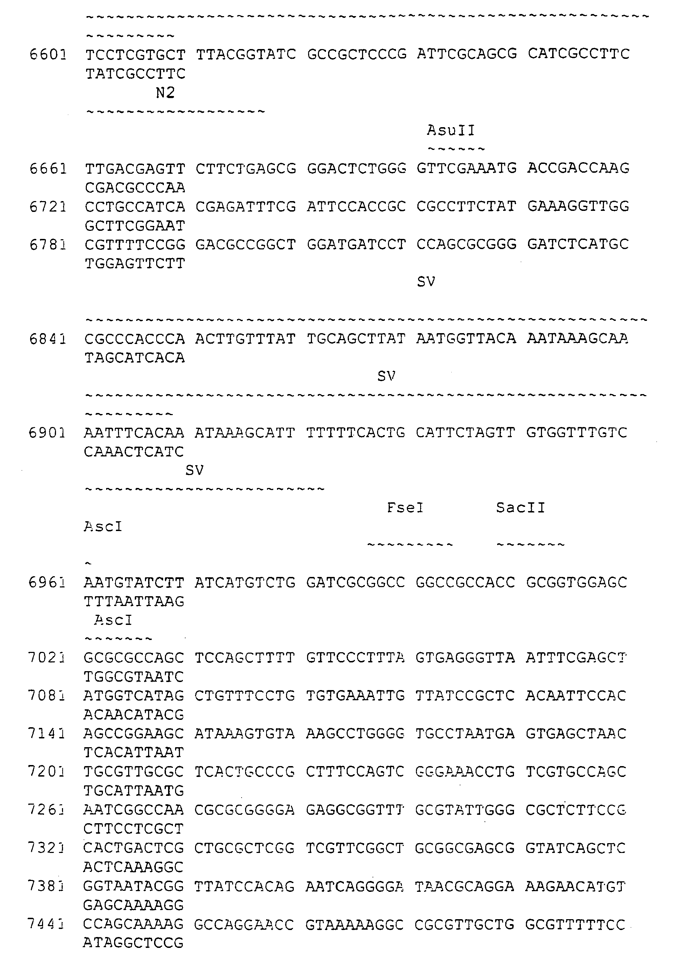

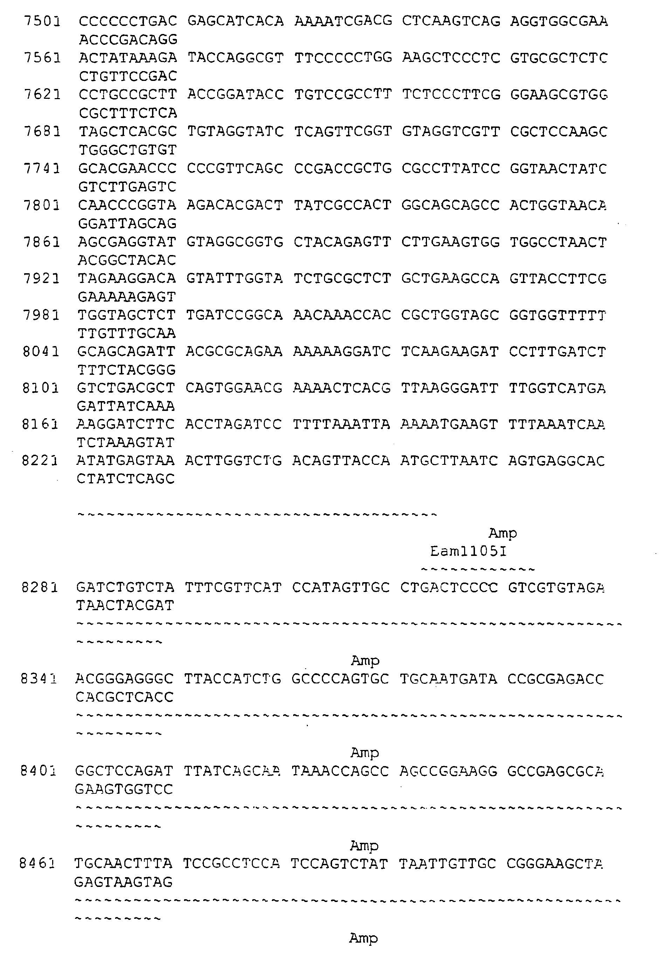

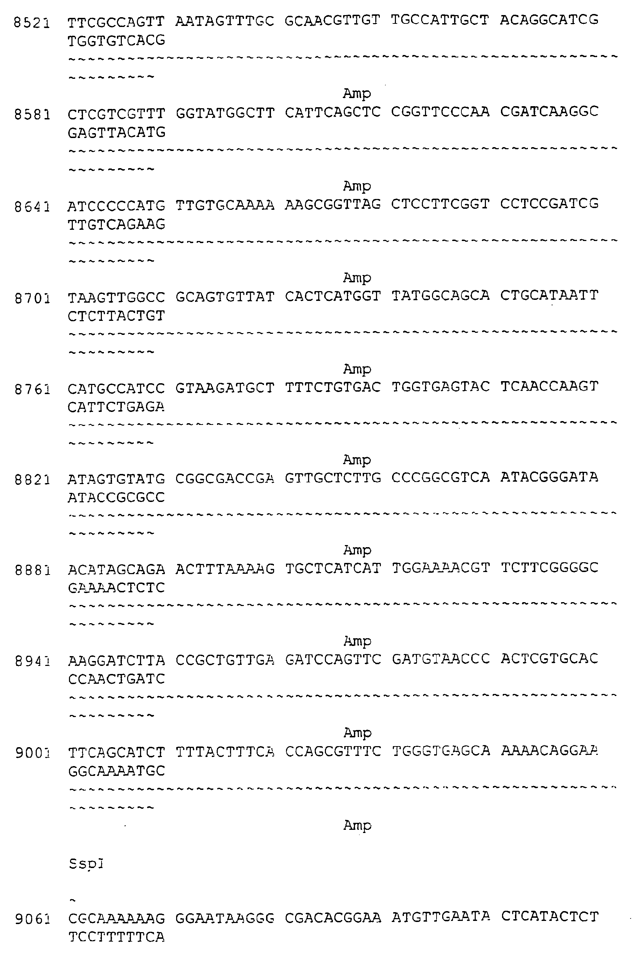

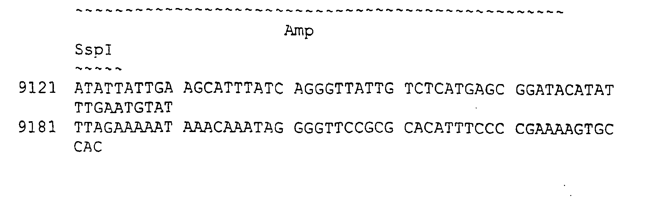

The polycistronic expression construct in this example encodes the modified humanized antibody with specificity to the human TAG 72 protein. This antibody comprised of humanized murine variable domains (both light and heavy), the human kappa light chain constant domain and the human gamma1 heavy chain constant region with a specific deletion of the CH2 domain. This expression construct is referred to as "HuCC49 CH2 Linker in N5KIRESG1 DeICH2", a map of which is depicted in Figure 3, and the sequence of which is set forth in Figure 6. HuCC49 CH2 Linker in N5KIRESG1 DeICH2 was transfected into the CHO-DG44 parent cell line. Transfections were performed using 0.5, 1.0, 2.0 or 4.0 ug linearized DNA per 4 x 10⁶ CHO cells per 96 well plate. Dominant selection of stably transfected cells was accomplished via the expression construct encoded neomycin resistance marker and media (CHO-S-SFM II + HT) containing G418.

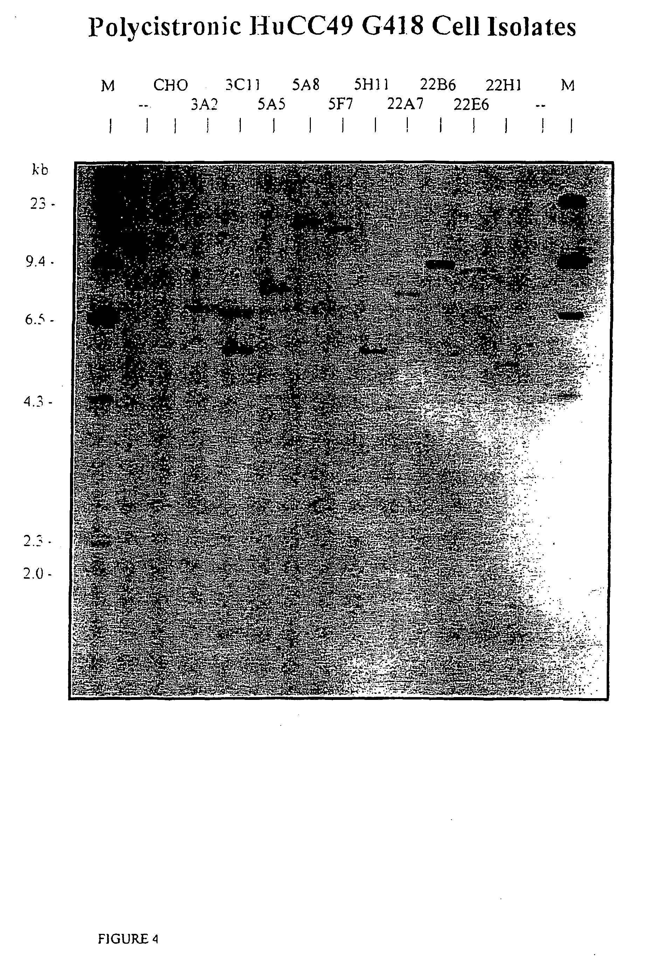

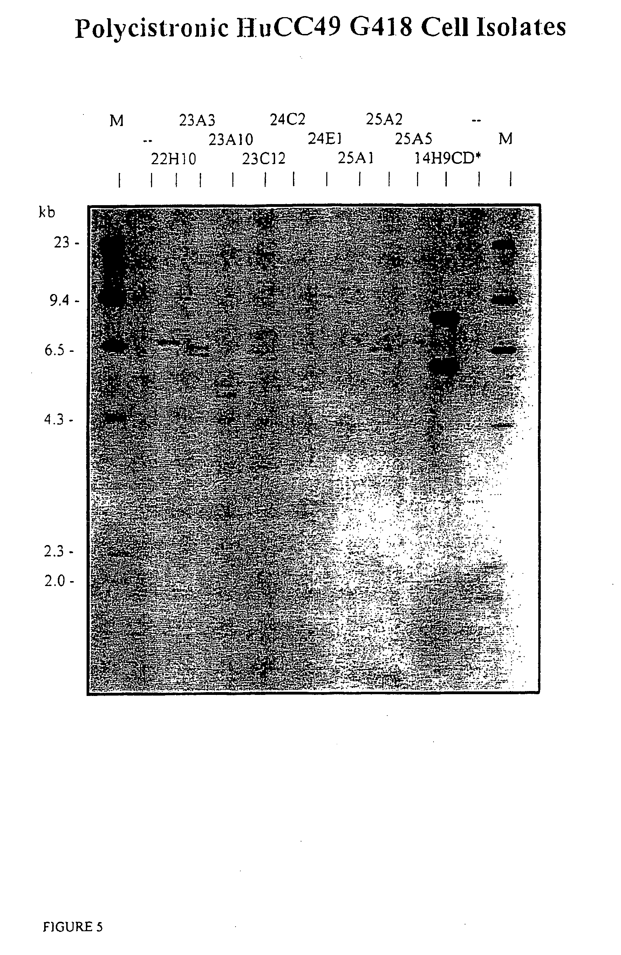

Viable wells were identified, expanded to small scale (120 ml) spinner culture and cellular productivity assessed by ELISA assay. A variety of G418 resistant cell isolates were obtained producing easily detectable secreted antibody. Four in particular, 22B6, 22E6, 22H10 and 25A2 were determined to be producing 3-10 mg/L in 3-4 days, or 1-5 picogram/cell/day (pcd). Southern blot analysis, as shown in Figures 4 and 5, was performed and it was observed that each of these four cell lines possess expression construct integrated at a single site within each cell line. The lanes labeled "M" contained size markers. Chinese hamster ovary cell nucleic acid served as a negative control, and was run in the lanes labeled "CHO". Sample 14H9CD was a NEOSPLA construction.

### B. ELISA Assay Ascertaining Ratio of Light to Heavy Chain

In parallel to generation of these cell lines, a NEOSPLA expression construct of the same antibody was constructed and transfected. HuCC49 CH2 Linker in N5KG1DelCH2, shown in Figure 3, encodes the immunoglobulin light and heavy chain genes on independent transcriptional cassettes, translation of each mediated by 5' CAP translational initiation complexes. A particular G418 resistant cell line isolated by transfection of this expression construct was termed 14H9. Analysis of culture supernatants from 14H9 and the polycistronic isolate 22B6 was performed to examine the relative ratios of secreted light chain and heavy chain. Analysis was performed by ELISA by capturing and detecting with either light or heavy chain specific secondary antibodies. Results of duplicate samples are displayed in Table 1, the data being expressed as mg/L compared to an immunoglobulin standard. Total light chain measured (κκ) is compared to total assembled antibody measured (γκ in the ratio column.

**TABLE 1**

| Construct Type | Cell line | κκ | γγ | γκ | Ratio κκ/γκ |
|---|---|---|---|---|---|
| POLYCISTRONIC | 5F7 | 20.6 | 2.2 | 7.1 | 2.9 |
| | 5F7 | 8.4 | 0.97 | 2.5 | 3.4 |
| | | | | | |
| NEOSPLA | 14H9 | 79.5 | 6.9 | 33.6 | 2.4 |
| | 14H9 | 56.0 | 6.7 | 23.9 | 2.3 |

The cell line 14H9 is an unusually high producing G418 cell line as a result of a high gene copy number. Of interest here are the roughly equivalent relative ratios of free or assembled immunoglobulin light and heavy chains. This data demonstrates that, surprisingly, expression of immunoglobulin heavy chain within the Polycistronic expression system, placed after the IRES, is not significantly reduced as compared to the NEOSPLA system. The observation that the κκ/γκ ratios are greater than one is not unexpected and are likely a result of permissive cellular secretion of free light chain. Therefore, in this Polycistronic expression system, IRES driven heavy chain protein production appears to be very efficient.

### C. Further ELISA Assays Comparing Expressed Ratio of Light to Heavy Chain

Analysis of culture supernatants from a variety of cell lines generated by either the NEOSPLA or Polycistronic expression systems was later performed to examine the relative ratios of secreted light chain and heavy chain. Analysis was performed by ELISA by capturing and detecting with either light or heavy chain specific secondary antibodies. Results of duplicate samples are displayed here and are expressed as mg/L compared to an immunoglobulin standard.

The ELISA is performed by coating with a 96 well micro-titre plate with a "capture" antibody, followed by binding of cell culture supernatant, followed by "detection" via binding of a secondary antibody conjugated to HRPO (horseradish peroxidase), followed by colorometric quantification. κκ indicates capture by goat anti-human kappa : detection by goat anti-human kappa-HRPO γγ indicates capture by goat anti-human IgG : detection by goat anti-human IgG-HRPO γκ indicates capture by goat anti-human IgG : detection by goat anti-human kappa-HRPO γγ indicates capture by goat anti-human kappa : detection by goat anti-human IgG-HRPO

**TABLE 2**

| | κκ | γγ | γκ | κγ | κκ/γ K | κκ/γγ | |
|---|---|---|---|---|---|---|---|
| **ANTI-CD23** | | | | | | | |
| 8B9 | 4.4 | 4.3 | 3.6 | 3.6 | 1.2 | 1.0 | NEOSPLA |
| 8B9-5A12 | 10.7 | 5.0 | 6.0 | 7.2 | 1.8 | 2.1 | NEOSPLA |
| 8B9-5A12-50F1 | 55.9 | 35.9 | 43.3 | 50.4 | 1.3 | 1.6 | NEOSPLA |
| 8B9-5A12-50F1- | 97.4 | 69.5 | 77.8 | 93.5 | 1.3 | 1.4 | NEOSPLA |
| 500G8 | | | | | | | |
| | | | | | | | |
| **HUCC49 NO** | | | | | | | |
| **LINKER** | | | | | | | |
| 12E9 | 4.6 | 4.1 | 3.5 | 3.3 | 1.3 | 1.1 | NEOSPLA |
| 12E9-5E8 | 21.6 | 11.5 | 11.5 | 10.1 | 1.9 | 1.9 | NEOSPLA |
| 12E9-5E8-50C9 | 68.3 | 25.9 | 27.7 | 22.9 | 2.5 | 2.6 | NEOSPLA |
| | | | | | | | |
| **ANTI-CD23** | | | | | | | |
| 5F8 | 4.2 | 3.3 | 3.1 | 3.0 | 1.4 | 1.3 | POLYCISTRONIC |
| | | | | | | | |
| **HUCC49 GLY/SER LINKER** | | | | | | | |
| 3C11 | 4.3 | 3.9 | 3.6 | 2.5 | 1.2 | 1.1 | POLYCISTRONIC |
| 3C11-5B12 | 21.0 | 10.6 | 11.1 | 9.2 | 1.9 | 2.0 | POLYCISTRONIC |
| 3C11-5B12-50B5 | 98.3 | 49.1 | 55.7 | 46.4 | 1.8 | 2.0 | POLYCISTRONIC |
| | | | | | | | |
| **HUCC49 CH2 LINKER** | | | | | | | |
| 25A2-50A5 | 21.8 | 12.4 | 12.3 | 10.6 | 1.8 | 1.8 | POLYCISTRONIC |
| 25A2-5B3-50A5 | 96.9 | 45.0 | 46.5 | 45.5 | 2.1 | 2.2 | POLYCISTRONIC |
| 25A2-5B3-50A5- | 180. | 111. | 105. | 102. | 1.7 | 1.6 | POLYCISTRONIC |
| 500F6 | 0 | 0 | 0 | 0 | | | |

This data further demonstrates that, surprisingly, expression of immunoglobulin heavy chain within the polycistronic expression system, placed after the IRES, is not significantly reduced as compared to the NEOSPLA system. The observation that the κκ/γγ ratios are greater than one is likely a result of permissive cellular secretion of free light chain. Therefore, in this polycistronic expression system IRES driven heavy chain protein production appears to be very efficient.

### EXAMPLE 3

### A. Increases in Expression via Genomic Amplification

Both NEOSPLA and Polycistronic expression systems contain an expression cassette encoding the murine DHFR gene. As the parent CHO-DG44 cell line used for expression is completely deficient in DHFR enzymatic activity (double deletion), amplification of the integrated target gene (murine DHFR) is possible by growth selection in media containing methotrexate (MTX). During this amplification, the directly linked immunoglobulin genes are concomitantly amplified. Thus it is possible to isolate cell lines producing elevated amounts of immunoglobulin. Our first round selection of G418 resistant cell lines is performed in 5 nM MTX. Highest level producers (as determined by ELISA) are identified and then subjected to two subsequent rounds of amplification with increasing concentrations of MTX (50 nM then 500 nM).

Following three successive rounds of amplification in MTX, 500 nM resistant cell lines can be identified. For the Polycistronic cell lineage derived from the G418 resistant cell line 25A2 the expression levels at each step for various isolates are listed below. For comparison purposes, listed below are expression levels for an irrelevant antibody (anti-CD23) expressed using the NEOSPLA expression system. This antibody is Primatized® antibody bearing primate-derived light and heavy chain variable domains, human kappa light chain constant region and the complete human gamma1 heavy chain (including the CH2 domain) region.

A comparison of production levels of the Polycistronic System and the NEOSPLA system grown in controlled bioreactors is also included. Duplicate runs in bioreactors are represented for each system, Polycistronic and NEOSPLA.

**TABLE 3**

| **Polycistronic Expression System** | | | | |
|---|---|---|---|---|
| **HuCC49 CH2 Linker in N5KIRESG1DelCH2** | | | | |
| Media time | Cell | mg/L | pcd | doubling |
| G418 | 25A2 | 4.5 | 2.0 | 30 hrs |
| 5 nM MTX | 25A2-5B3 | 16.6 | 11.5 | 32 |
| 50 nM MTX | 25A2-5B3-50A5 | 45.6 | 20.3 | 35 |
| 500 nM MTX | 25A2-5B3-50A5-500D8 | 78.0 | 26.8 | 42 |
| 500 nM MTX | 25A2-5B3-50A5-500F2 | 84.2 | 27.5 | 40 |
| 500 nM MTX | 25A2-5B3-50A5-500F6 | 89.9 | 25.1 | 35 |
| | | | | |
| In bioreactors | | | | |
| 500 nM MTX | 25A2-5B3-50A5-500F6 | 729.0 | 28.6 | Growth over |
| 15 days | | | | |
| 500 nM MTX | 25A2-5B3-50A5-500F6 | 701.0 | 28.2 | Growth over |
| 15 days | | | | |
| | | | | |
| **NEOSPLA Expression System** | | | | |

| **Anti-CD23 P5E8N-SHL in N5KG1** | | | | |
|---|---|---|---|---|
| Media time | Cell | mg/L | pcd | doubling |
| G418 | 8B9 | 14.9 | 4.5 | 33 |
| 5 nM MTX | 8B9-5A12 | 11.0 | 6.5 | 40 |
| 50 nM MTX | 8B9-5A12-50F1 | 56.1 | 22.0 | 35 |
| 500 nM MTX | 8B9-SA12-50F1-500G8 | 53.5 | 28.6 | 44 |
| | | | | |

| In bioreactors | | | | |
|---|---|---|---|---|
| 500 nM MTX 12 days | 8B9-5A12-50F1-500G8 | 927.0 | 29.1 | Growth over |
| 500 nM MTX 12 days | 8B9-5A12-50F1-500G8 | 680.0 | 29.1 | Growth over |

As can be seen from Table 3 above, expression levels at each stage of selection (G418, 5, 50 or 500 nM MTX) are comparable between the Polycistronic and NEOSPLA expression systems. Also, production levels from controlled bioreactor growth conditions appear comparable. Therefore, in this system, placing the immunoglobulin heavy chain under translational control of the EMCV IRES downstream of the light chain leads to effective immunoglobulin production.

### EXAMPLE 4

### Method For Generation of Expression Construct

A general method for the generation of polycistronic expression construct according to the invention is described herein. In this methodology the final expression construct is generated by insertion of a DNA fragment encoding the genes of interest (i.e. immunolgobulin light and heavy chains) in a single cloning step. The DNA fragment may be generated by use of conventional recombinant DNA technologies including oligo synthesis and ligation, overlapping PCR, complete DNA synthesis, or a combination of any of these methodologies as described previously.

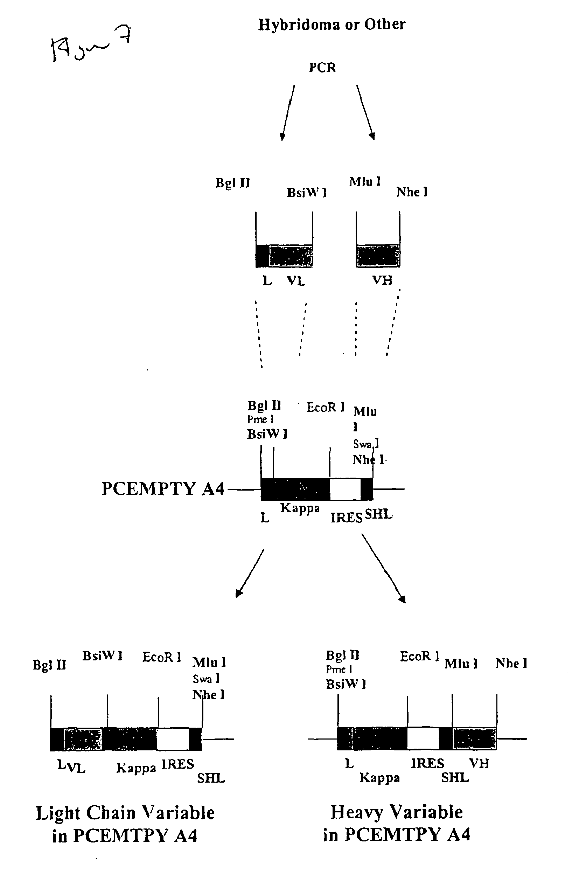

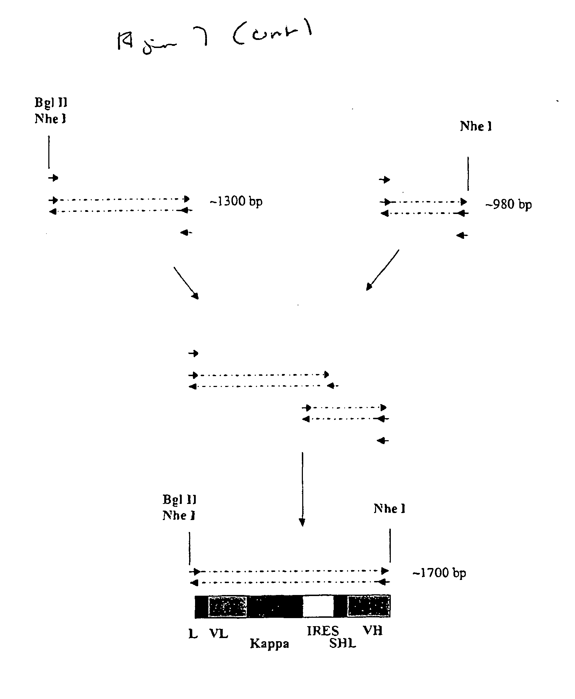

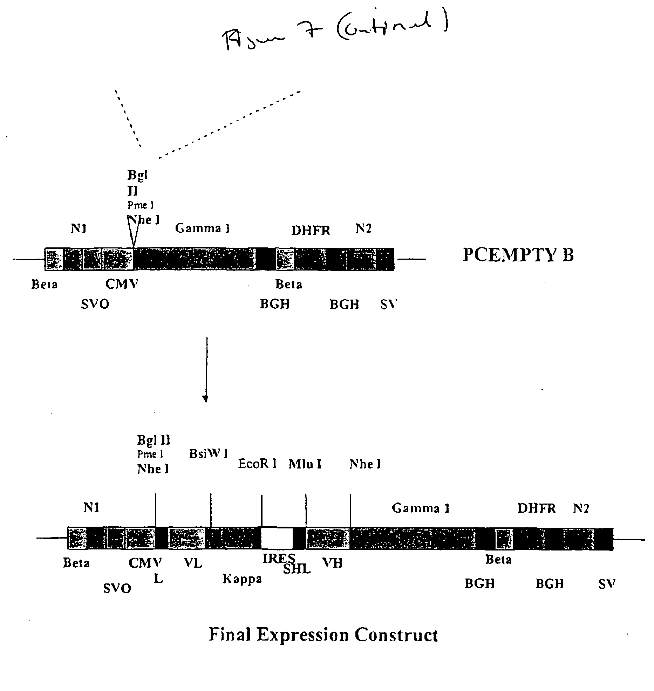

Figure 7 contains a schematic outline summarizing the steps utilized to generate a vector suitable for expression of an immunolgobulin (e.g., HuCC49) in mammalian cells. This methodology demonstrates the use of overlapping PCR to generate a DNA fragment encoding both light and heavy chain variable domains. This fragment is then cloned in a single step into a single insertion site of a desired vector DNA to generate the final immunoglobulin expression construct. However, while this is preferred, other methods are also be used, e.g., the respective heavy and light chain genes alternatively may be inserted at different insertion sites in the vector construct. Also, the number of light and heavy chain genes in the polycistronic construct may be varied.

As disclosed, the immunolgobulin light chain variable domain is isolated or cloned from a suitable source (i.e. hybridoma, B-cell, plasmid clone) and cloned into a suitable vector such as PCEMPTY A4 containing the light chain leader (which may or may not be retained depending on situation), light chain constant domain, IRES sequence, and a heavy chain leader sequence. Also, a heavy chain variable domain is isolated and cloned into the same empty vector (not containing the light chain variable domain). Initially having the light and heavy chain variable domains contained in separate vectors is desirable as it allows subsequent nucleotide modifications to one gene variable region without potentially impacting the other variable region.

The two separate vectors containing the light and heavy chain variable domains serve as DNA templates for independent cognate PCR amplification reactions. Design of the 5' and 3' PCR primers are such that there is a nucleotide sequence in common between the two resulting PCR products. This common sequence then allows an overlapping region for a subsequent PCR amplification step. The product of this ultimate PCR amplification step then encodes the light chain leader/light chain variable/constant/IRES/synthetic heavy leader/heavy variable domains or fragment thereof, e.g., domain deleted fragments. Figure 7 illustrates one example wherein a single DNA sequence comprising light and heavy chains separated by an IRES is inserted into a single restriction site on a vector.

By the judicious design of the above 5' and 3' PCR primers, flanking restriction endonuclease cleavage sites such as Nhe I are included. These Nhe I or other restriction sites then allow cloning of the DNA fragment into a suitable vector such as PCEMPTY B which contains all other elements for efficient expression the immunoglobulin in mammalian cells. The final vector (e.g., HuCC49 Gly/Ser in Polycis2) is then transfected into the desired cells to produce the gene product of interest e.g., (immunoglobulin).

### EXAMPLE 5

### Increases in Expression via Genomic Amplification

HuCC49 in N5KIRESG1DelCH2 also contains an expression cassette encoding the murine DHFR gene. As the parent CHO-DG44 cell line is completely deficient in DHFR enzymatic activity (double deletion), amplification of the integrated target gene (murine DHFR) is possible by growth selection in media containing methotrexate (MTX). During this amplification, the directly linked HuCC49 immunoglobulin genes are concomitantly amplified. Thus it is possible to isolate cell lines producing elevated amounts of immunoglobulin. First round selection of G418 resistant cell lines was performed in 5 nM MTX. Highest level producers (as determined by ELISA) were identified and then subjected to two subsequent rounds of amplification with increasing concentrations of MTX (50 nM then 500 nM).

Following three successive rounds of amplification in MTX, three 500 nM resistant cell lines were identified. For the cell lineage derived from the G418 resistant cell line 25A2, the expression levels at each step are listed in Table 4. For comparison purposes, Table 5 shows expression levels for an irrelevant antibody (anti-CD23) expressed using the NEOSPLA expression system. This antibody is Primatized® antibody bearing primate-derived light and heavy chain variable domains, human kappa light chain constant region and the complete human gamma1 heavy chain (including the CH2 domain) region.

**TABLE 4: Polycistronic Expression System**

| **HuCC49 CH2 Linker in N5KIRESG1DelCH2** | | | | |
|---|---|---|---|---|
| Media | Cell | mg/L | pcd | Doubling Time |
| G418 | 25A2 | 4.5 | 2.0 | 30 hrs |
| 5 nM MTX | 25A2-5B3 | 16.6 | 11.5 | 32 |
| 50 nM MTX | 25A2-5B3-50A5 | 45.6 | 20.3 | 35 |
| 500 nM MTX | 25A2-5B3-50A5-500D8 | 78.0 | 26.8 | 42 |
| 500 nM MTX | 25A2-5B3-50A5-500F2 | 84.2 | 27.5 | 40 |
| 500 nM MTX | 25A2-5B3-50A5-500F6 | 89.9 | 25.1 | 35 |

**TABLE 5: NEOSPLA Expression System**

| **Anti-CD23 P5E8N-SHL in N5KG1** | | | | |
|---|---|---|---|---|
| Media | Cell | mg/L | pcd | Doubling Time |
| G418 | 8B9 | 14.9 | 4.5 | 33 hrs |
| 5 nM MTX | 8B9-5A12 | 11.0 | 6.5 | 40 |
| 50 nM MTX | 8B9-5A12-50F1 | 56.1 | 22.0 | 35 |
| 500 nM MTX | 8B9-5A12-50F1-500G8 | 53.5 | 28.6 | 44 |

As can be seen in Tables 4 and 5 above, expression levels at each stage of selection (G418, 5, 50 or 500 nM MTX) are comparable between the Polycistronic and NEOSPLA expression systems. Therefore, in this system, placing the immunoglobulin heavy chain under translational control of the EMCV IRES downstream of the light chain leads to effective immunoglobulin production.

While the invention has been described in connection with specific embodiments and examples thereof, it will be understood by those of average skill in the art that the invention as disclosed is capable of further obvious modifications. This disclosure is intended to embrace such obvious departures from the preferred embodiments that fall within known or customary practices in the art and may be applied to the essential features set forth hereinabove, as well as to the scope of the appended claims.
The present invention furthermore relates to the following items:
1. A polycistronic vector for expressing functional antibodies in eukaryotic host cells which vector comprises a polycistronic transcription system comprising the following elements operably linked in the 5' to 3' orientation:
   (i) a promoter operable in a eukaryotic cell;
   (ii) a first cistron comprising a first DNA sequence encoding an antibody light chain which optionally comprises at its 5' terminus a signal peptide coding sequence operable in eukaryotic cells wherein said first DNA sequence does not comprise at its 3' end a poly A sequence and wherein said first DNA sequence comprises a 5' start codon and a 3' terminal stop codon;
   (iii) an internal ribosome entry site (IRES) obtained from a member selected from the group consisting of a cardiovirus, a herpes virus and a poliovirus; and
   (iv) at least a second cistron comprising the following elements: (a) a second DNA sequence encoding an antibody heavy chain, wherein said second DNA optionally comprises at its 5' terminus a signal peptide coding sequence operable in eukaryotic cells and wherein said second DNA sequence comprises a poly A sequence at its 3' terminus only if the DNA sequence is the 3' most coding sequence in the polycistron, and further comprises a start and stop codon at the 5' and 3' termini, respectively of said second DNA sequence;
   wherein the first DNA sequence is expressed at a ratio ranging between 10:1 and 1:1 with respect to the second DNA sequence in a eukaryotic host cell containing the polycistronic vector.
2. The polycistronic vector of item 1, wherein the first and second DNA sequences encoding respectively antibody heavy and light chain constant regions which are of primate origin.
3. The polycistronic vector of item 2, wherein said primate is human.
4. The polycistronic vector of item 1, wherein first and second DNA sequences encode respectively antibody heavy and light chain variable regions which are of primate origin.
5. The polycistronic vector of item 4, wherein said primate is human.
6. The polycistronic vector of item 5, wherein the heavy and light chain variable regions are humanized.
7. The polycistronic vector of item 1, wherein the first and second DNA sequences encode respectively antibody heavy and light chain constant regions which are of rodent origin.
8. The polycistronic vector of item 7, wherein said rodent is mouse.
9. The polycistronic vector of item 1, wherein the first and second DNA sequences encode respectively antibody heavy and light chain variable regions which are of rodent origin.
10. The polycistronic vector of item 9, wherein the DNA sequences encoding antibody heavy and light chain variable regions are of mouse origin.
11. The polycistronic vector of item 1, wherein the eukaryotic promoter is a mammalian promoter or viral promoter.
12. The polycistronic vector of item 11, wherein the promoter is a CMV promoter.
13. The polycistronic vector of item 1, wherein the IRES is obtained from a cardiovirus.
14. The polycistronic vector of item 13, wherein the cardiovirus is human encephalomyocarditis virus.
15. The polycistronic vector of item 1, wherein the functional antibodies expressed by the polycistronic vector specifically bind to a tumor associated antigen, an antigen expressed on a B cell or an antigen expressed on a T cell.
16. The polycistronic vector of item 15, wherein the functional antibodies expressed by the polycistronic vector specifically bind to an antigen selected from the group consisting of TAG-72, CD4, CD11, CD19, CD20, CD22, CD23, CD37, CD40, CD45, CD80, CD86 and CD154.
17. The polycistronic vector of item 16, wherein the antigen is TAG-72.
18. The polycistronic vector of item 16, wherein the functional antibody is a human, humanized Primatized or chimeric antibody specific to TAG-72.
19. The polycistronic vector of item 15, wherein the antibody is rituximab or ibritumomab.
20. The polycistronic vector of item 1, wherein DNA sequence encoding the antibody light chain is expressed at a ratio ranging between 3:1 and 1:1 with respect to the antibody heavy chain.
21. A eukaryotic cell comprising a polycistronic vector according to any one of items 1-20, wherein the eukaryotic cell secretes about 5 to about 100 picograms of functional antibody per day.
22. The eukaryotic cell of item 21, wherein the eukaryotic cell is a mammalian cell or yeast cell.
23. The mammalian cell of item 22, wherein the mammalian cell is a member selected from the group consisting of baby hamster kidney cell, fibroblast cell, myeloma cell, and Chinese Hamster Ovary cells (CHO cells).
24. The mammalian cell of item 23 which is a CHO cell.
25. The yeast cell of item 23 wherein said yeast cell is selected from the group consisting of Saccharomyces, Schizosacchoriomyces, Hansenula, Yarrowia, Pichia, and Candida.
26. The yeast cell of item 26 wherein said Pichia strain is Pichia pastoris.
27. A method of producing functional antibodies comprising culturing a eukaryotic cells according to item 21 in a cell culture to produce functional antibodies and recovering the functional antibodies from the eukaryotic cell culture.
28. The method of item 27, wherein the cultured eukaryotic cells produce at least about 1-5 picograms of antibodies/cell per day.
29. The method of item 28 wherein said eukaryotic cell is a mammalian or yeast cell.
30. The method of item 28, wherein the functional antibodies are recovered from cell culture medium.
31. The method of item 28, wherein the functional antibodies specifically bind TAG-72.
32. The method of item 31, wherein said functional antibody comprises a humanized, human or chimeric anti-CH₂ domain deleted HuCC49 antibody.
33. The method of item 28, wherein said chimeric antibody is rituximab or ibritumomab.
34. The method of item 27 wherein the production of functional antibodies further comprises the step of homologous recombination.
35. The method of item 28, wherein the functional antibodies are produced in batch fed cell cultures.

## Claims

1. A eukaryotic cell that is stably transfected with a polycistronic vector for expressing functional antibodies, the vector comprises a polycistronic transcription system comprising the following elements operably linked in the 5' to 3' orientation:
(i) a promoter operable in a eukaryotic cell;
(ii) a first cistron comprising a first DNA sequence comprising a 5' start codon, a nucleotide sequence encoding an antibody light chain, and a 3' terminal stop codon, wherein said first DNA sequence does not comprise a poly A sequence at its 3' end;
(iii) an internal ribosome entry site (IRES) obtained from a member selected from the group consisting of a cardiovirus, a herpes virus and a poliovirus;
(iv) at least an additional cistron comprising a second DNA sequence comprising a 5' start codon, a nucleotide sequence encoding an antibody heavy chain, and a 3' terminal stop codon, wherein said second DNA sequence comprises a poly A sequence at its 3' terminus; wherein the IRES is operably linked to the 5' end of the additional cistron with no intervening regulatory elements; and
wherein the functional antibodies expressed by the polycistronic vector specifically bind to a tumor associated antigen.

2. The eukaryotic cell of claim 1, wherein the nucleotide sequence encoding the antibody light chain is expressed at a ratio on average less than 3:1 with respect to the nucleotide sequence encoding the antibody heavy chain.

3. The eukaryotic cell of claim 2, wherein the nucleotide sequence encoding the antibody light chain is expressed at a ratio ranging on average between about 2:1 and 1:1 with respect to the nucleotide sequence encoding the antibody heavy chain.

4. The eukaryotic cell of claim 1, wherein the nucleotide sequence encoding the antibody light chain and the nucleotide sequence encoding the antibody heavy chain each comprise constant regions which are of primate origin or rodent origin.

5. The eukaryotic cell of claim 1, wherein the nucleotide sequence encoding the antibody light chain and the nucleotide sequence encoding the antibody heavy chain each comprise variable regions which are of primate origin or rodent origin.

6. The eukaryotic cell of claim 4 or 5, wherein said primate is human.

7. The eukaryotic cell of claim 1, wherein the promoter is a mammalian promoter or viral promoter.

8. The eukaryotic cell of claim 1, which secretes about 5 to about 100 picograms of functional antibodies per day.

9. The eukaryotic cell of claim 1, wherein the functional antibody is a human antibody, a humanized antibody, or a chimeric antibody.

10. The eukaryotic cell of claim 1, wherein the nucleotide sequence encoding the antibody light chain or antibody heavy chain comprises at its 5' terminus a sequence encoding a signal peptide that is operable in eukaryotic cells.

11. The eukaryotic cell of claim 1, wherein the functional antibodies specifically bind to the tumor associated antigen which is selected from the group consisting of TAG-72, CD4, CD11, CD19, CD20, CD22, CD23, CD37, CD40, CD45, CD80, CD86, CD154, CEA, prostate specific antigen (PSA), and HER-2 (erbB2).

12. The eukaryotic cell of claim 1, wherein the stably transfected cell is a either a mammalian cell, a fungal cell or a yeast cell.

13. The eukaryotic cell of claim 12, wherein the eukaryotic cell is a mammalian cell selected from the group consisting of myeloma cells, firbroblast cells, Chinese hamster ovary (CHO) cells, baby hamster kidney (BHK) cells, HeLa cells, CV1 cells, COS cells, R1610 cells, BALBC/3T3 cells, HAK cells, SP2/O cells, BFA-1 c1 BPT cells, RAJI cells, and 293 cells.

14. The eukaryotic cell of claim 1, wherein the a polycistronic vector is encoded by the nucleic acid sequence of SEQ ID NO: 2, except that said vector may optionally lack the TAG-72 antibody light chain leader, light chain variable domain, human kappa constant, heavy chain leader, heavy chain variable domain, and gamma constant sequences.

15. A method of producing functional antibodies comprising culturing the eukaryotic cell according to any one of claims 1-14 in a cell culture to produce functional antibodies.


SEQUENCE LISTING

<110> REFF, MITCHELL
      BARNETT, RICHARD

<120> POLYCISTRONIC EXPRESSION OF ANTIBODIES

<130> K1841 EP/1

<150> US 60/331,481
<151> 2001-11-16

<150> US 60/400,687
<151> 2002-08-05

<160> 5

<170> PatentIn version 3.3

<210> 1
<211> 10
<212> PRT
<213> Artificial sequence

<220>
<221> source
<223> /note="Description of artificial sequence: synthetic spacer
      peptide"

<400> 1

Gly Gly Ser Ser Gly Gly Gly Gly Ser Gly
1               5                   10


<210> 2
<211> 9233
<212> DNA
<213> Artificial sequence

<220>
<221> source
<223> /note="Description of artificial sequence: synthetic nucleotide
      sequence of HuCC49"

<400> 2
ctgacgcgcc ctgtagcggc gcattaagcg cggcgggtgt ggtggttacg cgcagcgtga      60

ccgctacact tgccagcgcc ctagcgcccg ctcctttcgc tttcttccct tcctttctcg     120

ccacgttcgc cggctttccc cgtcaagctc taaatcgggg gctcccttta gggttccgat     180

ttagtgcttt acggcacctc gaccccaaaa aacttgatta gggtgatggt tcacgtaggg     240

tcgcgacgta ccgggccccc cctcgattaa ttaatcgagc tactagcttt gcttctcaat     300

ttcttatttg cataatgaga aaaaaaggaa aattaatttt aacaccaatt cagtagttga     360

ttgagcaaat gcgttgccaa aaaggatgct ttagagacag tgttctctgc acagataagg     420

acaaacatta ttcagaggga gtacccagag ctgagactcc taagccagtg agtggcacag     480

catccaggga gaaatatgct tgtcatcacc gaagcctgat tccgtagagc cacaccctgg     540

taagggccaa tctgctcaca caggatagag agggcaggag ccagggcaga gcatataagg 600
tgaggtagga tcagttgctc ctcacatttg cttctgacat agttgtgcca gcatggagga 660
atcgatcctc catgcttgaa caagatggat tgcacgcagg ttctccggcc gcttgggtgg 720
agaggctatt cggctatgac tgggcacaac agacaatcgg ctgctctgat gccgccgtgt 780
tccggctgtc agcgcagggg cgcccggttc tttttgtcaa gaccgacctg tccggtgccc 840
tgaatgaact gcaggtaagt gcggccgctc taggcctcca aaaaagcctc ctcactactt 900
ctggaatagc tcagaggccg aggcggcctc ggcctctgca taaataaaaa aaattagtca 960
gccatgcatg gggcggagaa tgggcggaac tgggcggagt taggggcggg atgggcggag 1020
ttaggggcgg gactatggtt gctgactaat tgagatgcat gctttgcata cttctgcctg 1080
ctggggagcc tggggacttt ccacacctgg ttgctgacta attgagatgc atgctttgca 1140
tacttctgcc tgctggggag cctggggact ttccacaccc taactgacac acattccaca 1200
gaattaattc ccctagttat taatagtaat caattacggg gtcattagtt catagcccat 1260
atatggagtt ccgcgttaca taacttacgg taaatggccc gcctggctga ccgcccaacg 1320
acccccgccc attgacgtca ataatgacgt atgttcccat agtaacgcca atagggactt 1380
tccattgacg tcaatgggtg gagtatttac ggtaaactgc ccacttggca gtacatcaag 1440
tgtatcatat gccaagtacg ccccctattg acgtcaatga cggtaaatgg cccgcctggc 1500
attatgccca gtacatgacc ttatgggact ttcctacttg gcagtacatc tacgtattag 1560
tcatcgctat taccatggtg atgcggtttt ggcagtacat caatgggcgt ggatagcggt 1620
ttgactcacg gggatttcca agtctccacc ccattgacgt caatgggagt ttgttttggc 1680
accaaaatca acgggacttt ccaaaatgtc gtaacaactc cgccccattg acgcaaatgg 1740
gcggtaggcg tgtacggtgg gaggtctata taagcagagc tgggtacgtg aaccgtcaga 1800
tcgcctggag acgccatcac agatcaaagc tttgatctct caccatggat agccaggccc 1860
aggtgctcat gctcctgctg ctgtgggtga gcggcacatg cggcgacatc gtgatgagcc 1920
agtctccaga ctccctggcc gtgtccctgg gcgagagggt gactctgaat tgcaagtcca 1980
gccagtccct gctctatagc ggaaatcaga agaactatct cgcctggtat cagcagaaac 2040
cagggcagag ccctaaactg ctgatttact gggcatccgc tagggaatcc ggcgtgcctg 2100
atcgcttcag cggcagcgga tctgggacag acttcactct gacaatcagc agcgtgcagg 2160
cagaagacgt ggcagtctat tattgtcagc agtattatag ctatcccctc acattcggcg 2220
ctggcaccaa gctggaactg aaacgtacgg tggctgcacc atctgtcttc atcttcccgc 2280
catctgatga gcagttgaaa tctggaactg cctctgttgt gtgcctgctg aataacttct 2340
atcccagaga ggccaaagta cagtggaagg tggataacgc cctccaatcg ggtaactccc 2400
aggagagtgt cacagagcag gacagcaagg acagcaccta cagcctcagc agcaccctga 2460

cgctgagcaa agcagactac gagaaacaca aagtctacgc ctgcgaagtc acccatcagg 2520
gcctgagctc gcccgtcaca aagagcttca acaggggaga gtgttgaatt cctccctccc 2580
ccccccctaa cgttactggc cgaagccgct tggaataagg ccggtgtgcg tttgtctata 2640
tgttattttc caccatattg ccgtcttttg gcaatgtgag ggcccggaaa cctggccctg 2700
tcttcttgac gagcattcct aggggtcttt cccctctcgc caaaggaatg caaggtctgt 2760
tgaatgtcgt gaaggaagca gttcctctgg aagcttcttg aagacaaaca acgtctgtac 2820
cgaccctttg caggcagcgg aaccccccac ctggcgacag gtgcctctgc ggccaaaagc 2880
cacgtgtata agatacacct gcaaaggcgg cacaacccca gtgccacgtt gtgagttgga 2940
tagttgtgga aagagtcaaa tggctctcct caagcgtatt caacaagggg ctgaaggatg 3000
cccagaaggt accccattgt atgggatctg atctggggcc tcggtgcaca tgctttacat 3060
gtgtttagtc gaggttaaaa aacgtctagg ccccccgaac cacggggacg tggttttcct 3120
ttgaaaaaca cgatgataat atgggttgga gcctcatctt gctcttcctt gtcgctgttg 3180
ctacgcgtgt cctgtcccag gtccagctgg tgcagtccgg cgctgaggtg gtgaaacctg 3240
gggcttccgt gaagatttcc tgcaaggcaa gcggctacac cttcactgat cacgcaatcc 3300
actgggtgaa acagaatcct ggacagcgcc tggagtggat tggatatttc tctcccggaa 3360
acgatgattt taagtacaat gagaggttca agggcaaggc cacactgact gcagacacat 3420
ctgccagcac tgcctacgtg gagctctcca gcctgagatc cgaggatact gcagtgtact 3480
tctgcacaag atccctgaat atggcctact ggggacaggg aaccctggtc accgtctcca 3540
gcgctagcac caagggccca tcggtcttcc ccctggcacc ctcctccaag agcacctctg 3600
ggggcacagc ggccctgggc tgcctggtca aggactactt ccccgaaccg gtgacggtgt 3660
cgtggaactc aggcgccctg accagcggcg tgcacacctt cccggctgtc ctacagtcct 3720
caggactcta ctccctcagc agcgtggtga ccgtgccctc cagcagcttg ggcacccaga 3780
cctacatctg caacgtgaat cacaagccca gcaacaccaa ggtggacaag aaagttgagc 3840
ccaaatcttg tgacaaaact cacacatgcc caccgtgccc catcgagaaa accatctcca 3900
aagccaaagg gcagccccga gaaccacagg tgtacaccct gcccccatcc cgggatgagc 3960
tgaccaagaa ccaggtcagc ctgacctgcc tggtcaaagg cttctatccc agcgacatcg 4020
ccgtggagtg ggagagcaat gggcagccgg agaacaacta caagaccacg cctcccgtgc 4080
tggactccga cggctccttc ttcctctaca gcaagctcac cgtggacaag agcaggtggc 4140
agcaggggaa cgtcttctca tgctccgtga tgcatgaggc tctgcacaac cactacacgc 4200
agaagagcct ctccctgtct ccgggtaaat gaggatccaa ttggcccggg cagatctgat 4260
ccgttaacgg ttaccaacta cctagactgg attcgtgaca acatgcggcc gtgatatcta 4320
cgtatgatca gcctcgactg tgccttctag ttgccagcca tctgttgttt gcccctcccc 4380

cgtgccttcc ttgaccctgg aaggtgccac tcccactgtc ctttcctaat aaaatgagga 4440
aattgcatcg cattgtctga gtaggtgtca ttctattctg gggggtgggg tggggcagga 4500
cagcaagggg gaggattggg aagacaatag caggcatgct ggggatgcgg tgggctctat 4560
ggcttctgag gcggaaagaa ccagctgggg ctcgacagcg ctgcgatcgc ctcgactagt 4620
agctttgctt ctcaatttct tatttgcata atgagaaaaa aaggaaaatt aattttaaca 4680
ccaattcagt agttgattga gcaaatgcgt tgccaaaaag gatgctttag agacagtgtt 4740
ctctgcacag ataaggacaa acattattca gagggagtac ccagagctga gactcctaag 4800
ccagtgagtg gcacagcatc cagggagaaa tatgcttgtc atcaccgaag cctgattccg 4860
tagagccaca ccctggtaag ggccaatctg ctcacacagg atagagaggg caggagccag 4920
ggcagagcat ataaggtgag gtaggatcag ttgctcctca catttgcttc tgacatagtt 4980
gtgttgggag cttggatagc ttgggggggg gacagctcag ggctgcgatt tcgcgccaaa 5040
cttgacggca atcctagcgt gaaggctggt aggattttat ccccgctgcc atcatggttc 5100
gaccattgaa ctgcatcgtc gccgtgtccc aaaatatggg gattggcaag aacggagacc 5160
taccctggcc tccgctcagg aacgagttca agtacttcca aagaatgacc acaacctctt 5220
cagtggaagg taaacagaat ctggtgatta tgggtaggaa aacctggttc tccattcctg 5280
agaagaatcg acctttaaag gacagaatta atatagttct cagtagagaa ctcaaagaac 5340
caccacgagg agctcatttt cttgccaaaa gtttggatga tgccttaaga cttattgaac 5400
aaccggaatt ggcaagtaaa gtagacatgg tttggatagt cggaggcagt tctgtttacc 5460
aggaagccat gaatcaacca ggccacctca gactctttgt gacaaggatc atgcaggaat 5520
ttgaaagtga cacgtttttc ccagaaattg atttggggaa atataaactt ctcccagaat 5580
acccaggcgt cctctctgag gtccaggagg aaaaaggcat caagtataag tttgaagtct 5640
acgagaagaa agactaacag gaagatgctt tcaagttctc tgctcccctc ctaaagctat 5700
gcatttttat aagaccatgg gacttttgct ggctttagat cagcctcgac tgtgccttct 5760
agttgccagc catctgttgt ttgcccctcc cccgtgcctt ccttgaccct ggaaggtgcc 5820
actcccactg tcctttccta ataaaatgag gaaattgcat cgcattgtct gagtaggtgt 5880
cattctattc tggggggtgg ggtggggcag gacagcaagg gggaggattg ggaagacaat 5940
agcaggcatg ctggggatgc ggtgggctct atggcttctg aggcggaaag aaccagctgg 6000
ggctcgaagc ggccgctccg gatcgaggcc gctactaact ctctcctccc tcctttttcc 6060
tgcaggacga ggcagcgcgg ctatcgtggc tggccacgac gggcgttcct tgcgcagctg 6120
tgctcgacgt tgtcactgaa gcgggaaggg actggctgct attgggcgaa gtgccggggc 6180
aggatctcct gtcatctcac cttgctcctg ccgagaaagt atccatcatg gctgatgcaa 6240
tgcggcggct gcatacgctt gatccggcta cctgcccatt cgaccaccaa gcgaaacatc 6300

gcatcgagcg agcacgtact cggatggaag ccggtcttgt cgatcaggat gatctggacg 6360
aagagcatca ggggctcgcg ccagccgaac tgttcgccag gctcaaggcg cgcatgcccg 6420
acggcgagga tctcgtcgtg acccatggcg atgcctgctt gccgaatatc atggtggaaa 6480
atggccgctt ttctggattc atcgactgtg gccggctggg tgtggcggac cgctatcagg 6540
acatagcgtt ggctacccgt gatattgctg aagagcttgg cggcgaatgg gctgaccgct 6600
tcctcgtgct ttacggtatc gccgctcccg attcgcagcg catcgccttc tatcgccttc 6660
ttgacgagtt cttctgagcg ggactctggg gttcgaaatg accgaccaag cgacgcccaa 6720
cctgccatca cgagatttcg attccaccgc cgccttctat gaaaggttgg gcttcggaat 6780
cgttttccgg gacgccggct ggatgatcct ccagcgcggg gatctcatgc tggagttctt 6840
cgcccaccca acttgtttat tgcagcttat aatggttaca aataaagcaa tagcatcaca 6900
aatttcacaa ataaagcatt tttttcactg cattctagtt gtggtttgtc caaactcatc 6960
aatgtatctt atcatgtctg gatcgcggcc ggccgccacc gcggtggagc tttaattaag 7020
gcgcgccagc tccagctttt gttcccttta gtgagggtta atttcgagct tggcgtaatc 7080
atggtcatag ctgtttcctg tgtgaaattg ttatccgctc acaattccac acaacatacg 7140
agccggaagc ataaagtgta aagcctgggg tgcctaatga gtgagctaac tcacattaat 7200
tgcgttgcgc tcactgcccg ctttccagtc gggaaacctg tcgtgccagc tgcattaatg 7260
aatcggccaa cgcgcgggga gaggcggttt gcgtattggg cgctcttccg cttcctcgct 7320
cactgactcg ctgcgctcgg tcgttcggct gcggcgagcg gtatcagctc actcaaaggc 7380
ggtaatacgg ttatccacag aatcagggga taacgcagga aagaacatgt gagcaaaagg 7440
ccagcaaaag gccaggaacc gtaaaaaggc cgcgttgctg gcgtttttcc ataggctccg 7500
cccccctgac gagcatcaca aaaatcgacg ctcaagtcag aggtggcgaa acccgacagg 7560
actataaaga taccaggcgt ttccccctgg aagctccctc gtgcgctctc ctgttccgac 7620
cctgccgctt accggatacc tgtccgcctt tctcccttcg ggaagcgtgg cgctttctca 7680
tagctcacgc tgtaggtatc tcagttcggt gtaggtcgtt cgctccaagc tgggctgtgt 7740
gcacgaaccc cccgttcagc ccgaccgctg cgccttatcc ggtaactatc gtcttgagtc 7800
caacccggta agacacgact tatcgccact ggcagcagcc actggtaaca ggattagcag 7860
agcgaggtat gtaggcggtg ctacagagtt cttgaagtgg tggcctaact acggctacac 7920
tagaaggaca gtatttggta tctgcgctct gctgaagcca gttaccttcg gaaaaagagt 7980
tggtagctct tgatccggca aacaaaccac cgctggtagc ggtggttttt ttgtttgcaa 8040
gcagcagatt acgcgcagaa aaaaaggatc tcaagaagat cctttgatct tttctacggg 8100
gtctgacgct cagtggaacg aaaactcacg ttaagggatt ttggtcatga gattatcaaa 8160
aaggatcttc acctagatcc ttttaaatta aaaatgaagt tttaaatcaa tctaaagtat 8220

atatgagtaa acttggtctg acagttacca atgcttaatc agtgaggcac ctatctcagc 8280

gatctgtcta tttcgttcat ccatagttgc ctgactcccc gtcgtgtaga taactacgat 8340

acgggagggc ttaccatctg gccccagtgc tgcaatgata ccgcgagacc cacgctcacc 8400

ggctccagat ttatcagcaa taaaccagcc agccggaagg gccgagcgca gaagtggtcc 8460

tgcaacttta tccgcctcca tccagtctat taattgttgc cgggaagcta gagtaagtag 8520

ttcgccagtt aatagtttgc gcaacgttgt tgccattgct acaggcatcg tggtgtcacg 8580

ctcgtcgttt ggtatggctt cattcagctc cggttcccaa cgatcaaggc gagttacatg 8640

atcccccatg ttgtgcaaaa aagcggttag ctccttcggt cctccgatcg ttgtcagaag 8700

taagttggcc gcagtgttat cactcatggt tatggcagca ctgcataatt ctcttactgt 8760

catgccatcc gtaagatgct tttctgtgac tggtgagtac tcaaccaagt cattctgaga 8820

atagtgtatg cggcgaccga gttgctcttg cccggcgtca atacgggata ataccgcgcc 8880

acatagcaga actttaaaag tgctcatcat tggaaaacgt tcttcggggc gaaaactctc 8940

aaggatctta ccgctgttga gatccagttc gatgtaaccc actcgtgcac ccaactgatc 9000

ttcagcatct tttactttca ccagcgtttc tgggtgagca aaaacaggaa ggcaaaatgc 9060

cgcaaaaaag ggaataaggg cgacacggaa atgttgaata ctcatactct tcctttttca 9120

atattattga agcatttatc agggttattg tctcatgagc ggatacatat ttgaatgtat 9180

ttagaaaaat aaacaaatag gggttccgcg cacatttccc cgaaaagtgc cac 9233

<210> 3
<211> 3548
<212> DNA
<213> Artificial sequence

<220>
<221> source
<223> /note="Description of artificial sequence: synthetic
      polycistronic expression construct nucleotide sequence"

<400> 3
ctgacgcgcc ctgtagcggc gcattaagcg cggcgggtgt ggtggttacg cgcagcgtga 60

ccgctacact tgccagcgcc ctagcgcccg ctcctttcgc tttcttccct tcctttctcg 120

ccacgttcgc cggctttccc cgtcaagctc taaatcgggg gctcccttta gggttccgat 180

ttagtgcttt acggcacctc gaccccaaaa aacttgatta gggtgatggt tcacgtaggg 240

tcgcgacgta ccgggccccc cctcgattaa tagatctctc accatgaggg tccccgctca 300

gctcctgggg ctcctgctgc tctggctccc aggtgcacga tgtgatgttt aaacggtacc 360

aaggtggaaa tcaaacgtac ggtggctgca ccatctgtct tcatcttccc gccatctgat 420

gagcagttga aatctggaac tgcctctgtt gtgtgcctgc tgaataactt ctatcccaga 480

gaggccaaag tacagtggaa ggtggataac gccctccaat cgggtaactc ccaggagagt 540

gtcacagagc aggacagcaa ggacagcacc tacagcctca gcagcaccct gacgctgagc 600

aaagcagact acgagaaaca caaagtctac gcctgcgaag tcacccatca gggcctgagc 660

tcgcccgtca caaagagctt caacagggga gagtgttgaa ttcctccctc ccccccccct 720

aacgttactg gccgaagccg cttggaataa ggccggtgtg cgtttgtcta tatgttattt 780

tccaccatat tgccgtcttt tggcaatgtg agggcccgga aacctggccc tgtcttcttg 840

acgagcattc ctagtggtct ttcccctctc gccaaaggaa tgcaaggtct gttgaatgtc 900

gtgaaggaag cagttcctct ggaagcttct tgaagacaaa caacgtctgt agcgaccctt 960

tgcaggcagc ggaacccccc acctggcgac aggtgcctct gcggccaaaa gccacgtgta 1020

taagatacac ctgcaaaggc ggcacaaccc cagtgccagg ttgtgagttg gatagttgtg 1080

gaaagagtca aatggctctc ctcaagcgta ttcaacaagg ggctgaagga tgcccagaag 1140

gtaccccatt gtatgggatc tgatctgggg cctcggtgca catgctttac atgtgtttag 1200

tcgaggttaa aaaacgtcta ggccccccga accacgggga cgtggttttc ctttgaaaaa 1260

cacgatgata atatgggttg gagcctcatc ttgctcttcc ttgtcgctgt tgctacgcgt 1320

atttaaatgc tagcggcgcg ccagctccag cttttgttcc ctttagtgag ggttaatttc 1380

gagcttggcg taatcatggt catagctgtt tcctgtgtga aattgttatc cgctcacaat 1440

tccacacaac atacgagccg gaagcataaa gtgtaaagcc tggggtgcct aatgagtgag 1500

ctaactcaca ttaattgcgt tgcgctcact gcccgctttc cagtcgggaa acctgtcgtg 1560

ccagctgcat taatgaatcg gccaacgcgc ggggagaggc ggtttgcgta ttgggcgctc 1620

ttccgcttcc tcgctcactg actcgctgcg ctcggtcgtt cggctgcggc gagcggtatc 1680

agctcactca aaggcggtaa tacggttatc cacagaatca ggggataacg caggaaagaa 1740

catgtgagca aaaggccagc aaaaggccag gaaccgtaaa aaggccgcgt tgctggcgtt 1800

tttccatagg ctccgccccc ctgacgagca tcacaaaaat cgacgctcaa gtcagaggtg 1860

gcgaaacccg acaggactat aaagatacca ggcgtttccc cctggaagct ccctcgtgcg 1920

ctctcctgtt ccgaccctgc cgcttaccgg atacctgtcc gcctttctcc cttcgggaag 1980

cgtggcgctt tctcatagct cacgctgtag gtatctcagt tcggtgtagg tcgttcgctc 2040

caagctgggc tgtgtgcacg aaccccccgt tcagcccgac cgctgcgcct tatccggtaa 2100

ctatcgtctt gagtccaacc cggtaagaca cgacttatcg ccactggcag cagccactgg 2160

taacaggatt agcagagcga ggtatgtagg cggtgctaca gagttcttga agtggtggcc 2220

taactacggc tacactagaa gaacagtatt tggtatctgc gctctgctga agccagttac 2280

cttcggaaaa agagttggta gctcttgatc cggcaaacaa accaccgctg gtagcggtgg 2340

tttttttgtt tgcaagcagc agattacgcg cagaaaaaaa ggatctcaag aagatccttt 2400

gatcttttct acggggtctg acgctcagtg gaacgaaaac tcacgttaag ggattttggt 2460

catgagatta tcaaaaagga tcttcaccta gatcctttta aattaaaaat gaagttttaa 2520

atcaatctaa agtatatatg agtaaacttg gtctgacagt taccaatgct taatcagtga 2580

ggcacctatc tcagcgatct gtctatttcg ttcatccata gttgcctgac tccccgtcgt 2640

gtagataact acgatacggg agggcttacc atctggcccc agtgctgcaa tgataccgcg 2700

agacccacgc tcaccggctc cagatttatc agcaataaac cagccagccg gaagggccga 2760

gcgcagaagt ggtcctgcaa ctttatccgc ctccatccag tctattaatt gttgccggga 2820

agctagagta agtagttcgc cagttaatag tttgcgcaac gttgttgcca ttgctacagg 2880

catcgtggtg tcacgctcgt cgtttggtat ggcttcattc agctccggtt cccaacgatc 2940

aaggcgagtt acatgatccc ccatgttgtg caaaaaagcg gttagctcct tcggtcctcc 3000

gatcgttgtc agaagtaagt tggccgcagt gttatcactc atggttatgg cagcactgca 3060

taattctctt actgtcatgc catccgtaag atgcttttct gtgactggtg agtactcaac 3120

caagtcattc tgagaatagt gtatgcggcg accgagttgc tcttgcccgg cgtcaatacg 3180

ggataatacc gcgccacata gcagaacttt aaaagtgctc atcattggaa aacgttcttc 3240

ggggcgaaaa ctctcaagga tcttaccgct gttgagatcc agttcgatgt aacccactcg 3300

tgcacccaac tgatcttcag catcttttac tttcaccagc gtttctgggt gagcaaaaac 3360

aggaaggcaa aatgccgcaa aaaagggaat aagggcgaca cggaaatgtt gaatactcat 3420

actcttcctt tttcaatatt attgaagcat ttatcagggt tattgtctca tgagcggata 3480

catatttgaa tgtatttaga aaaataaaca aataggggtt ccgcgcacat ttccccgaaa 3540

agtgccac 3548

<210> 4
<211> 7815
<212> DNA
<213> Artificial sequence

<220>
<221> source
<223> /note="Description of artificial sequence: plasmid PCEMPTYB
nucleotide sequence"

<400> 4
ctgacgcgcc ctgtagcggc gcattaagcg cggcgggtgt ggtggttacg cgcagcgtga 60

ccgctacact tgccagcgcc ctagcgcccg ctcctttcgc tttcttccct tcctttctcg 120

ccacgttcgc cggctttccc cgtcaagctc taaatcgggg gctcccttta gggttccgat 180

ttagtgcttt acggcacctc gaccccaaaa aacttgatta gggtgatggt tcacgtaggg 240

tcgcgacgta ccgggccccc cctcgattaa ttaatcgagc tactagcttt gcttctcaat 300

ttcttatttg cataatgaga aaaaaaggaa aattaatttt aacaccaatt cagtagttga 360

ttgagcaaat gcgttgccaa aaaggatgct ttagagacag tgttctctgc acagataagg 420

acaaacatta ttcagaggga gtacccagag ctgagactcc taagccagtg agtggcacag 480
catccaggga gaaatatgct tgtcatcacc gaagcctgat tccgtagagc cacaccctgg 540
taagggccaa tctgctcaca caggatagag agggcaggag ccagggcaga gcatataagg 600
tgaggtagga tcagttgctc ctcacatttg cttctgacat agttgtgcca gcatggagga 660
atcgatcctc catgcttgaa caagatggat tgcacgcagg ttctccggcc gcttgggtgg 720
agaggctatt cggctatgac tgggcacaac agacaatcgg ctgctctgat gccgccgtgt 780
tccggctgtc agcgcagggg cgcccggttc tttttgtcaa gaccgacctg tccggtgccc 840
tgaatgaact gcaggtaagt gcggccgctc taggcctcca aaaaagcctc ctcactactt 900
ctggaatagc tcagaggccg aggcggcctc ggcctctgca taaataaaaa aaattagtca 960
gccatgcatg gggcggagaa tgggcggaac tgggcggagt taggggcggg atgggcggag 1020
ttaggggcgg gactatggtt gctgactaat tgagatgcat gctttgcata cttctgcctg 1080
ctggggagcc tggggacttt ccacacctgg ttgctgacta attgagatgc atgctttgca 1140
tacttctgcc tgctggggag cctggggact ttccacaccc taactgacac acattccaca 1200
gaattaattc ccctagttat taatagtaat caattacggg gtcattagtt catagcccat 1260
atatggagtt ccgcgttaca taacttacgg taaatggccc gcctggctga ccgcccaacg 1320
acccccgccc attgacgtca ataatgacgt atgttcccat agtaacgcca atagggactt 1380
tccattgacg tcaatgggtg gagtatttac ggtaaactgc ccacttggca gtacatcaag 1440
tgtatcatat gccaagtacg ccccctattg acgtcaatga cggtaaatgg cccgcctggc 1500
attatgccca gtacatgacc ttatgggact ttcctacttg gcagtacatc tacgtattag 1560
tcatcgctat taccatggtg atgcggtttt ggcagtacat caatgggcgt ggatagcggt 1620
ttgactcacg gggatttcca agtctccacc ccattgacgt caatgggagt ttgttttggc 1680
accaaaatca acgggacttt ccaaaatgtc gtaacaactc cgccccattg acgcaaatgg 1740
gcggtaggcg tgtacggtgg gaggtctata taagcagagc tgggtacgtg aaccgtcaga 1800
tcgcctggag acgccatcac agatctgttt aaacgctagc accaagggcc catcggtctt 1860
ccccctggca ccctcctcca agagcacctc tgggggcaca gcggccctgg gctgcctggt 1920
caaggactac ttccccgaac cggtgacggt gtcgtggaac tcaggcgccc tgaccagcgg 1980
cgtgcacacc ttcccggctg tcctacagtc ctcaggactc tactccctca gcagcgtggt 2040
gaccgtgccc tccagcagct tgggcaccca gacctacatc tgcaacgtga atcacaagcc 2100
cagcaacacc aaggtggaca agaaagttga gcccaaatct tgtgacaaaa ctcacacatg 2160
cccaccgtgc ccagcacctg aactcctggg gggaccgtca gtcttcctct tccccccaaa 2220
acccaaggac accctcatga tctcccggac ccctgaggtc acatgcgtgg tggtggacgt 2280
gagccacgaa gaccctgagg tcaagttcaa ctggtacgtg gacggcgtgg aggtgcataa 2340

tgccaagaca aagccgcggg aggagcagta caacagcacg taccgtgtgg tcagcgtcct 2400
caccgtcctg caccaggact ggctgaatgg caaggagtac aagtgcaagg tctccaacaa 2460
agccctccca gcccccatcg agaaaaccat ctccaaagcc aaagggcagc cccgagaacc 2520
acaggtgtac accctgcccc catcccggga tgagctgacc aagaaccagg tcagcctgac 2580
ctgcctggtc aaaggcttct atcccagcga catcgccgtg gagtgggaga gcaatgggca 2640
gccggagaac aactacaaga ccacgcctcc cgtgctggac tccgacggct ccttcttcct 2700
ctacagcaag ctcaccgtgg acaagagcag gtggcagcag gggaacgtct tctcatgctc 2760
cgtgatgcat gaggctctgc acaaccacta cacgcagaag agcctctccc tgtctccggg 2820
taaatgagga tccaattcga ctgatgcaac ggttaccaac tacctagact ggattcgtga 2880
caacatgcgg ccgtgatatc tacgtatgat cagcctcgac tgtgccttct agttgccagc 2940
catctgttgt ttgcccctcc cccgtgcctt ccttgaccct ggaaggtgcc actcccactg 3000
tcctttccta ataaaatgag gaaattgcat cgcattgtct gagtaggtgt cattctattc 3060
tggggggtgg ggtggggcag gacagcaagg gggaggattg ggaagacaat agcaggcatg 3120
ctggggatgc ggtgggctct atggcttctg aggcggaaag aaccagctgg ggctcgacag 3180
cgctgcgatc gcctcgacta gtagctttgc ttctcaattt cttatttgca taatgagaaa 3240
aaaaggaaaa ttaattttaa caccaattca gtagttgatt gagcaaatgc gttgccaaaa 3300
aggatgcttt agagacagtg ttctctgcac agataaggac aaacattatt cagagggagt 3360
acccagagct gagactccta agccagtgag tggcacagca tccagggaga aatatgcttg 3420
tcatcaccga agcctgattc cgtagagcca caccctggta agggccaatc tgctcacaca 3480
ggatagagag ggcaggagcc agggcagagc atataaggtg aggtaggatc agttgctcct 3540
cacatttgct tctgacatag ttgtgttggg agcttggata gcttgggggg gggacagctc 3600
agggctgcga tttcgcgcca aacttgacgg caatcctagc gtgaaggctg gtaggatttt 3660
atccccgctg ccatcatggt tcgaccattg aactgcatcg tcgccgtgtc ccaaaatatg 3720
gggattggca agaacggaga cctaccctgg cctccgctca ggaacgagtt caagtacttc 3780
caaagaatga ccacaacctc ttcagtggaa ggtaaacaga atctggtgat tatgggtagg 3840
aaaacctggt tctccattcc tgagaagaat cgacctttaa aggacagaat taatatagtt 3900
ctcagtagag aactcaaaga accaccacga ggagctcatt ttcttgccaa aagtttggat 3960
gatgccttaa gacttattga acaaccggaa ttggcaagta aagtagacat ggtttggata 4020
gtcggaggca gttctgttta ccaggaagcc atgaatcaac caggccacct cagactcttt 4080
gtgacaagga tcatgcagga atttgaaagt gacacgtttt tcccagaaat tgatttgggg 4140
aaatataaac ttctcccaga atacccaggc gtcctctctg aggtccagga ggaaaaaggc 4200
atcaagtata agtttgaagt ctacgagaag aaagactaac aggaagatgc tttcaagttc 4260

tctgctcccc tcctaaagct atgcattttt ataagaccat gggacttttg ctggctttag 4320
atcagcctcg actgtgcctt ctagttgcca gccatctgtt gtttgcccct cccccgtgcc 4380
ttccttgacc ctggaaggtg ccactcccac tgtcctttcc taataaaatg aggaaattgc 4440
atcgcattgt ctgagtaggt gtcattctat tctggggggt ggggtggggc aggacagcaa 4500
gggggaggat tgggaagaca atagcaggca tgctggggat gcggtgggct ctatggcttc 4560
tgaggcggaa agaaccagct ggggctcgaa gcggccgctc cggatcgagg ccgctactaa 4620
ctctctcctc cctccttttt cctgcaggac gaggcagcgc ggctatcgtg gctggccacg 4680
acgggcgttc cttgcgcagc tgtgctcgac gttgtcactg aagcgggaag ggactggctg 4740
ctattgggcg aagtgccggg gcaggatctc ctgtcatctc accttgctcc tgccgagaaa 4800
gtatccatca tggctgatgc aatgcggcgg ctgcatacgc ttgatccggc tacctgccca 4860
ttcgaccacc aagcgaaaca tcgcatcgag cgagcacgta ctcggatgga agccggtctt 4920
gtcgatcagg atgatctgga cgaagagcat caggggctcg cgccagccga actgttcgcc 4980
aggctcaagg cgcgcatgcc cgacggcgag gatctcgtcg tgacccatgg cgatgcctgc 5040
ttgccgaata tcatggtgga aaatggccgc ttttctggat tcatcgactg tggccggctg 5100
ggtgtggcgg accgctatca ggacatagcg ttggctaccc gtgatattgc tgaagagctt 5160
ggcggcgaat gggctgaccg cttcctcgtg ctttacggta tcgccgctcc cgattcgcag 5220
cgcatcgcct tctatcgcct tcttgacgag ttcttctgag cgggactctg gggttcgaaa 5280
tgaccgacca agcgacgccc aacctgccat cacgagattt cgattccacc gccgccttct 5340
atgaaaggtt gggcttcgga atcgttttcc gggacgccgg ctggatgatc ctccagcgcg 5400
gggatctcat gctggagttc ttcgcccacc ccaacttgtt tattgcagct tataatggtt 5460
caaataaagc aatagcatca caaatttcac aaataaagca tttttttcac tgcattctag 5520
ttgtggtttg tccaaactca tcaatgtatc ttatcatgtc tggatcgcgg ccggccgcca 5580
ccgcggtgga gctttaatta aggcgcgcca gctccagctt ttgttccctt tagtgagggt 5640
taatttcgag cttggcgtaa tcatggtcat agctgtttcc tgtgtgaaat tgttatccgc 5700
tcacaattcc acacaacata cgagccggaa gcataaagtg taaagcctgg ggtgcctaat 5760
gagtgagcta actcacatta attgcgttgc gctcactgcc cgctttccag tcgggaaacc 5820
tgtcgtgcca gctgcattaa tgaatcggcc aacgcgcggg gagaggcggt ttgcgtattg 5880
ggcgctcttc cgcttcctcg ctcactgact cgctgcgctc ggtcgttcgg ctgcggcgag 5940
cggtatcagc tcactcaaag gcggtaatac ggttatccac agaatcaggg gataacgcag 6000
gaaagaacat gtgagcaaaa ggccagcaaa aggccaggaa ccgtaaaaag gccgcgttgc 6060
tggcgttttt ccataggctc cgcccccctg acgagcatca caaaaatcga cgctcaagtc 6120
agaggtggcg aaacccgaca ggactataaa gataccaggc gtttccccct ggaagctccc 6180

tcgtgcgctc tcctgttccg accctgccgc ttaccggata cctgtccgcc tttctccctt 6240

cgggaagcgt ggcgctttct catagctcac gctgtaggta tctcagttcg gtgtaggtcg 6300

ttcgctccaa gctgggctgt gtgcacgaac cccccgttca gcccgaccgc tgcgccttat 6360

ccggtaacta tcgtcttgag tccaacccgg taagacacga cttatcgcca ctggcagcag 6420

ccactggtaa caggattagc agagcgaggt atgtaggcgg tgctacagag ttcttgaagt 6480

ggtggcctaa ctacggctac actagaagaa cagtatttgg tatctgcgct ctgctgaagc 6540

cagttacctt cggaaaaaga gttggtagct cttgatccgg caaacaaacc accgctggta 6600

gcggtggttt ttttgtttgc aagcagcaga ttacgcgcag aaaaaaagga tctcaagaag 6660

atcctttgat cttttctacg gggtctgacg ctcagtggaa cgaaaactca cgttaaggga 6720

ttttggtcat gagattatca aaaaggatct tcacctagat ccttttaaat taaaaatgaa 6780

gttttaaatc aatctaaagt atatatgagt aaacttggtc tgacagttac caatgcttaa 6840

tcagtgaggc acctatctca gcgatctgtc tatttcgttc atccatagtt gcctgactcc 6900

ccgtcgtgta gataactacg atacgggagg gcttaccatc tggccccagt gctgcaatga 6960

taccgcgaga cccacgctca ccggctccag atttatcagc aataaaccag ccagccggaa 7020

gggccgagcg cagaagtggt cctgcaactt tatccgcctc catccagtct attaattgtt 7080

gccgggaagc tagagtaagt agttcgccag ttaatagttt gcgcaacgtt gttgccattg 7140

ctacaggcat cgtggtgtca cgctcgtcgt ttggtatggc ttcattcagc tccggttccc 7200

aacgatcaag gcgagttaca tgatccccca tgttgtgcaa aaaagcggtt agctccttcg 7260

gtcctccgat cgttgtcaga agtaagttgg ccgcagtgtt atcactcatg gttatggcag 7320

cactgcataa ttctcttact gtcatgccat ccgtaagatg cttttctgtg actggtgagt 7380

actcaaccaa gtcattctga gaatagtgta tgcggcgacc gagttgctct tgcccggcgt 7440

caatacggga taataccgcg ccacatagca gaactttaaa agtgctcatc attggaaaac 7500

gttcttcggg gcgaaaactc tcaaggatct taccgctgtt gagatccagt tcgatgtaac 7560

ccactcgtgc acccaactga tcttcagcat cttttacttt caccagcgtt tctgggtgag 7620

caaaaacagg aaggcaaaat gccgcaaaaa agggaataag ggcgacacgg aaatgttgaa 7680

tactcatact cttccttttt caatattatt gaagcattta tcagggttat tgtctcatga 7740

gcggatacat atttgaatgt atttagaaaa ataaacaaat aggggttccg cgcacatttc 7800

cccgaaaagt gccac 7815

<210> 5
<211> 9527
<212> DNA
<213> Artificial sequence

<220>
<221> source

<223>  /note="Description of artificial sequence: synthetic nucleotide
      sequence of HuCC49 Gly/Ser construct contained in Polycis 2
      vector"

<400>  5
ctgacgcgcc ctgtagcggc gcattaagcg cggcgggtgt ggtggttacg cgcagcgtga      60

ccgctacact tgccagcgcc ctagcgcccg ctcctttcgc tttcttccct tcctttctcg     120

ccacgttcgc cggctttccc cgtcaagctc taaatcgggg gctcccttta gggttccgat     180

ttagtgcttt acggcacctc gaccccaaaa aacttgatta gggtgatggt tcacgtaggg     240

tcgcgacgta ccgggccccc cctcgattaa ttaatcgagc tactagcttt gcttctcaat     300

ttcttatttg cataatgaga aaaaaaggaa aattaatttt aacaccaatt cagtagttga     360

ttgagcaaat gcgttgccaa aaaggatgct ttagagacag tgttctctgc acagataagg     420

acaaacatta ttcagaggga gtacccagag ctgagactcc taagccagtg agtggcacag     480

catccaggga gaaatatgct tgtcatcacc gaagcctgat tccgtagagc cacaccctgg     540

taagggccaa tctgctcaca caggatagag agggcaggag ccagggcaga gcatataagg     600

tgaggtagga tcagttgctc ctcacatttg cttctgacat agttgtgcca gcatggagga     660

atcgatcctc catgcttgaa caagatggat tgcacgcagg ttctccggcc gcttgggtgg     720

agaggctatt cggctatgac tgggcacaac agacaatcgg ctgctctgat gccgccgtgt     780

tccggctgtc agcgcagggg cgcccggttc tttttgtcaa gaccgacctg tccggtgccc     840

tgaatgaact gcaggtaagt gcggccgctc taggcctcca aaaaagcctc ctcactactt     900

ctggaatagc tcagaggccg aggcggcctc ggcctctgca taaataaaaa aaattagtca     960

gccatgcatg gggcggagaa tgggcggaac tgggcggagt taggggcggg atgggcggag    1020

ttaggggcgg gactatggtt gctgactaat tgagatgcat gctttgcata cttctgcctg    1080

ctggggagcc tggggacttt ccacacctgg ttgctgacta attgagatgc atgctttgca    1140

tacttctgcc tgctggggag cctggggact ttccacaccc taactgacac acattccaca    1200

gaattaattc ccctagttat taatagtaat caattacggg gtcattagtt catagcccat    1260

atatggagtt ccgcgttaca taacttacgg taaatggccc gcctggctga ccgcccaacg    1320

acccccgccc attgacgtca ataatgacgt atgttcccat agtaacgcca atagggactt    1380

tccattgacg tcaatgggtg gagtatttac ggtaaactgc ccacttggca gtacatcaag    1440

tgtatcatat gccaagtacg ccccctattg acgtcaatga cggtaaatgg cccgcctggc    1500

attatgccca gtacatgacc ttatgggact ttcctacttg gcagtacatc tacgtattag    1560

tcatcgctat taccatggtg atgcggtttt ggcagtacat caatgggcgt ggatagcggt    1620

ttgactcacg gggatttcca agtctccacc ccattgacgt caatgggagt ttgttttggc    1680

accaaaatca acgggacttt ccaaaatgtc gtaacaactc cgccccattg acgcaaatgg    1740

gcggtaggcg tgtacggtgg gaggtctata taagcagagc tgggtacgtg aaccgtcaga    1800

tcgcctggag acgccatcac agatctgttt aaacgctagc ctctcaccat ggatagccag 1860
gcccaggtgc tcatgctcct gctgctgtgg gtgagcggca catgcggcga catcgtgatg 1920
agccagtctc cagactccct ggccgtgtcc ctgggcgaga gggtgactct gaattgcaag 1980
tccagccagt ccctgctcta tagcggaaat cagaagaact atctcgcctg gtatcagcag 2040
aaaccagggc agagccctaa actgctgatt tactgggcat ccgctaggga atccggcgtg 2100
cctgatcgct tcagcggcag cggatctggg acagacttca ctctgacaat cagcagcgtg 2160
caggcagaag acgtggcagt ctattattgt cagcagtatt atagctatcc cctcacattc 2220
ggcgctggca ccaagctgga actgaaacgt acggtggctg caccatctgt cttcatcttc 2280
ccgccatctg atgagcagtt gaaatctgga actgcctctg ttgtgtgcct gctgaataac 2340
ttctatccca gagaggccaa agtacagtgg aaggtggata acgccctcca atcgggtaac 2400
tcccaggaga gtgtcacaga gcaggacagc aaggacagca cctacagcct cagcagcacc 2460
ctgacgctga gcaaagcaga ctacgagaaa cacaaagtct acgcctgcga agtcacccat 2520
cagggcctga gctcgcccgt cacaaagagc ttcaacaggg gagagtgttg aattcctccc 2580
tccccccccc ctaacgttac tggccgaagc cgcttggaat aaggccggtg tgcgtttgtc 2640
tatatgttat tttccaccat attgccgtct tttggcaatg tgagggcccg gaaacctggc 2700
cctgtcttct tgacgagcat tcctaggggt ctttcccctc tcgccaaagg aatgcaaggt 2760
ctgttgaatg tcgtgaagga agcagttcct ctggaagctt cttgaagaca aacaacgtct 2820
gtagcgaccc tttgcaggca gcggaacccc ccacctggcg acaggtgcct ctgcggccaa 2880
aagccacgtg tataagatac acctgcaaag gcggcacaac cccagtgcca cgttgtgagt 2940
tggatagttg tggaaagagt caaatggctc tcctcaagcg tattcaacaa ggggctgaag 3000
gatgcccaga aggtacccca ttgtatggga tctgatctgg ggcctcggtg cacatgcttt 3060
acatgtgttt agtcgaggtt aaaaaacgtc taggcccccc gaaccacggg gacgtggttt 3120
tcctttgaaa aacacgatga taatatgggt tggagcctca tcttgctctt ccttgtcgct 3180
gttgctacgc gtgtcctgtc ccaggtccag ctggtgcagt ccggcgctga ggtggtgaaa 3240
cctggggctt ccgtgaagat ttcctgcaag gcaagcggct acaccttcac tgatcacgca 3300
atccactggg tgaaacagaa tcctggacag cgcctggagt ggattggata tttctctccc 3360
ggaaacgatg attttaagta caatgagagg ttcaagggca aggccacact gactgcagac 3420
acatctgcca gcactgccta cgtggagctc tccagcctga gatccgagga tactgcagtg 3480
tacttctgca caagatccct gaatatggcc tactggggac agggaaccct ggtcaccgtc 3540
tccagcgcta gcaccaaggg cccatcggtc ttccccctgg caccctcctc caagagcacc 3600
tctgggggca cagcggccct gggctgcctg gtcaaggact acttccccga accggtgacg 3660
gtgtcgtgga actcaggcgc cctgaccagc ggcgtgcaca ccttcccggc tgtcctacag 3720

tcctcaggac tctactccct cagcagcgtg gtgaccgtgc cctccagcag cttgggcacc 3780
cagacctaca tctgcaacgt gaatcacaag cccagcaaca ccaaggtgga caagaaagtt 3840
gagcccaaat cttgtgacaa aactcacaca tgcccaccgt gcccagcacc tgaactcctg 3900
gggggaccgt cagtcttcct cttcccccca aaacccaagg acaccctcat gatctcccgg 3960
acccctgagg tcacatgcgt ggtggtggac gtgagccacg aagaccctga ggtcaagttc 4020
aactggtacg tggacggcgt ggaggtgcat aatgccaaga caaagccgcg ggaggagcag 4080
tacaacagca cgtaccgtgt ggtcagcgtc ctcaccgtcc tgcaccagga ctggctgaat 4140
ggcaaggagt acaagtgcaa ggtctccaac aaagccctcc cagcccccat cgagaaaacc 4200
atctccaaag ccaaagggca gccccgagaa ccacaggtgt acaccctgcc cccatcccgg 4260
gatgagctga ccaagaacca ggtcagcctg acctgcctgg tcaaaggctt ctatcccagc 4320
gacatcgccg tggagtggga gagcaatggg cagccggaga acaactacaa gaccacgcct 4380
cccgtgctgg actccgacgg ctccttcttc ctctacagca agctcaccgt ggacaagagc 4440
aggtggcagc aggggaacgt cttctcatgc tccgtgatgc atgaggctct gcacaaccac 4500
tacacgcaga agagcctctc cctgtctccg ggtaaatgag gatccaattc gactgatgca 4560
acggttacca actacctaga ctggattcgt gacaacatgc ggccgtgata tctacgtatg 4620
atcagcctcg actgtgcctt ctagttgcca gccatctgtt gtttgcccct cccccgtgcc 4680
ttccttgacc ctggaaggtg ccactcccac tgtcctttcc taataaaatg aggaaattgc 4740
atcgcattgt ctgagtaggt gtcattctat tctggggggt ggggtggggc aggacagcaa 4800
gggggaggat tgggaagaca atagcaggca tgctggggat gcggtgggct ctatggcttc 4860
tgaggcggaa agaaccagct ggggctcgac agcgctgcga tcgcctcgac tagtagcttt 4920
gcttctcaat ttcttatttg cataatgaga aaaaaaggaa aattaatttt aacaccaatt 4980
cagtagttga ttgagcaaat gcgttgccaa aaaggatgct ttagagacag tgttctctgc 5040
acagataagg acaaacatta ttcagaggga gtacccagag ctgagactcc taagccagtg 5100
agtggcacag catccaggga gaaatatgct tgtcatcacc gaagcctgat tccgtagagc 5160
cacaccctgg taagggccaa tctgctcaca caggatagag agggcaggag ccagggcaga 5220
gcatataagg tgaggtagga tcagttgctc ctcacatttg cttctgacat agttgtgttg 5280
ggagcttgga tagcttgggg gggggacagc tcagggctgc gatttcgcgc caaacttgac 5340
ggcaatccta gcgtgaaggc tggtaggatt ttatccccgc tgccatcatg gttcgaccat 5400
tgaactgcat cgtcgccgtg tcccaaaata tggggattgg caagaacgga gacctaccct 5460
ggcctccgct caggaacgag ttcaagtact tccaaagaat gaccacaacc tcttcagtgg 5520
aaggtaaaca gaatctggtg attatgggta ggaaaacctg gttctccatt cctgagaaga 5580
atcgaccttt aaaggacaga attaatatag ttctcagtag agaactcaaa gaaccaccac 5640

gaggagctca ttttcttgcc aaaagtttgg atgatgcctt aagacttatt gaacaaccgg 5700
aattggcaag taaagtagac atggtttgga tagtcggagg cagttctgtt taccaggaag 5760
ccatgaatca accaggccac ctcagactct ttgtgacaag gatcatgcag gaatttgaaa 5820
gtgacacgtt tttcccagaa attgatttgg ggaaatataa acttctccca gaatacccag 5880
gcgtcctctc tgaggtccag gaggaaaaag gcatcaagta taagtttgaa gtctacgaga 5940
agaaagacta acaggaagat gctttcaagt tctctgctcc cctcctaaag ctatgcattt 6000
ttataagacc atgggacttt tgctggcttt agatcagcct cgactgtgcc ttctagttgc 6060
cagccatctg ttgtttgccc ctcccccgtg ccttccttga ccctggaagg tgccactccc 6120
actgtccttt cctaataaaa tgaggaaatt gcatcgcatt gtctgagtag gtgtcattct 6180
attctggggg gtggggtggg gcaggacagc aagggggagg attgggaaga caatagcagg 6240
catgctgggg atgcggtggg ctctatggct tctgaggcgg aaagaaccag ctggggctcg 6300
aagcggccgc tccggatcga ggccgctact aactctctcc tccctccttt ttcctgcagg 6360
acgaggcagc gcggctatcg tggctggcca cgacgggcgt tccttgcgca gctgtgctcg 6420
acgttgtcac tgaagcggga agggactggc tgctattggg cgaagtgccg gggcaggatc 6480
tcctgtcatc tcaccttgct cctgccgaga aagtatccat catggctgat gcaatgcggc 6540
ggctgcatac gcttgatccg gctacctgcc cattcgacca ccaagcgaaa catcgcatcg 6600
agcgagcacg tactcggatg gaagccggtc ttgtcgatca ggatgatctg gacgaagagc 6660
atcaggggct cgcgccagcc gaactgttcg ccaggctcaa ggcgcgcatg cccgacggcg 6720
aggatctcgt cgtgacccat ggcgatgcct gcttgccgaa tatcatggtg gaaaatggcc 6780
gcttttctgg attcatcgac tgtggccggc tgggtgtggc ggaccgctat caggacatag 6840
cgttggctac ccgtgatatt gctgaagagc ttggcggcga atgggctgac cgcttcctcg 6900
tgctttacgg tatcgccgct cccgattcgc agcgcatcgc cttctatcgc cttcttgacg 6960
agttcttctg agcgggactc tggggttcga aatgaccgac caagcgacgc ccaacctgcc 7020
atcacgagat ttcgattcca ccgccgcctt ctatgaaagg ttgggcttcg gaatcgtttt 7080
ccgggacgcc ggctggatga tcctccagcg cggggatctc atgctggagt tcttcgccca 7140
cccaacttgt ttattgcagc ttataatggt tacaaataaa gcaatagcat cacaaatttc 7200
acaaataaag catttttttc actgcattct agttgtggtt tgtccaaact catcaatgta 7260
tcttatcatg tctggatcgc ggccggccgc caccgcggtg gagctttaat taaggcgcgc 7320
cagctccagc ttttgttccc tttagtgagg gttaatttcg agcttggcgt aatcatggtc 7380
atagctgttt cctgtgtgaa attgttatcc gctcacaatt ccacacaaca tacgagccgg 7440
aagcataaag tgtaaagcct ggggtgccta atgagtgagc taactcacat taattgcgtt 7500
gcgctcactg cccgctttcc agtcgggaaa cctgtcgtgc cagctgcatt aatgaatcgg 7560

ccaacgcgcg gggagaggcg gtttgcgtat tgggcgctct tccgcttcct cgctcactga 7620
ctcgctgcgc tcggtcgttc ggctgcggcg agcggtatca gctcactcaa aggcggtaat 7680
acggttatcc acagaatcag gggataacgc aggaaagaac atgtgagcaa aaggccagca 7740
aaaggccagg aaccgtaaaa aggccgcgtt gctggcgttt ttccataggc tccgcccccc 7800
tgacgagcat cacaaaaatc gacgctcaag tcagaggtgg cgaaacccga caggactata 7860
aagataccag gcgtttcccc ctggaagctc cctcgtgcgc tctcctgttc cgaccctgcc 7920
gcttaccgga tacctgtccg cctttctccc ttcgggaagc gtggcgcttt ctcatagctc 7980
acgctgtagg tatctcagtt cggtgtaggt cgttcgctcc aagctgggct gtgtgcacga 8040
accccccgtt cagcccgacc gctgcgcctt atccggtaac tatcgtcttg agtccaaccc 8100
ggtaagacac gacttatcgc cactggcagc agccactggt aacaggatta gcagagcgag 8160
gtatgtaggc ggtgctacag agttcttgaa gtggtggcct aactacggct acactagaag 8220
aacagtattt ggtatctgcg ctctgctgaa gccagttacc ttcggaaaaa gagttggtag 8280
ctcttgatcc ggcaaacaaa ccaccgctgg tagcggtggt ttttttgttt gcaagcagca 8340
gattacgcgc agaaaaaaag gatctcaaga agatcctttg atcttttcta cggggtctga 8400
cgctcagtgg aacgaaaact cacgttaagg gattttggtc atgagattat caaaaaggat 8460
cttcacctag atccttttaa attaaaaatg aagttttaaa tcaatctaaa gtatatatga 8520
gtaaacttgg tctgacagtt accaatgctt aatcagtgag gcacctatct cagcgatctg 8580
tctatttcgt tcatccatag ttgcctgact ccccgtcgtg tagataacta cgatacggga 8640
gggcttacca tctggcccca gtgctgcaat gataccgcga gacccacgct caccggctcc 8700
agatttatca gcaataaacc agccagccgg aagggccgag cgcagaagtg gtcctgcaac 8760
tttatccgcc tccatccagt ctattaattg ttgccgggaa gctagagtaa gtagttcgcc 8820
agttaatagt ttgcgcaacg ttgttgccat tgctacaggc atcgtggtgt cacgctcgtc 8880
gtttggtatg gcttcattca gctccggttc ccaacgatca aggcgagtta catgatcccc 8940
catgttgtgc aaaaaagcgg ttagctcctt cggtcctccg atcgttgtca gaagtaagtt 9000
ggccgcagtg ttatcactca tggttatggc agcactgcat aattctctta ctgtcatgcc 9060
atccgtaaga tgcttttctg tgactggtga gtactcaacc aagtcattct gagaatagtg 9120
tatgcggcga ccgagttgct cttgcccggc gtcaatacgg gataataccg cgccacatag 9180
cagaacttta aaagtgctca tcattggaaa acgttcttcg gggcgaaaac tctcaaggat 9240
cttaccgctg ttgagatcca gttcgatgta acccactcgt gcacccaact gatcttcagc 9300
atcttttact ttcaccagcg tttctgggtg agcaaaaaca ggaaggcaaa atgccgcaaa 9360
aaagggaata agggcgacac ggaaatgttg aatactcata ctcttccttt ttcaatatta 9420
ttgaagcatt tatcagggtt attgtctcat gagcggatac atatttgaat gtatttagaa 9480

aaataaacaa ataggggttc cgcgcacatt tccccgaaaa gtgccac 9527